(19) Europäisches Patentamt / European Patent Office / Office européen des brevets

(11) **EP 2 459 231 B1**

(12) **EUROPÄISCHE PATENTSCHRIFT**

(45) Veröffentlichungstag und Bekanntmachung des Hinweises auf die Patenterteilung:
**08.06.2016 Patentblatt 2016/23**

(51) Int Cl.:
***A61K 48/00*** *(2006.01)*

(21) Anmeldenummer: **10742089.5**

(22) Anmeldetag: **30.07.2010**

(86) Internationale Anmeldenummer:
**PCT/EP2010/004681**

(87) Internationale Veröffentlichungsnummer:
**WO 2011/012316 (03.02.2011 Gazette 2011/05)**

(54) **RNA MIT EINER KOMBINATION AUS UNMODIFIZIERTEN UND MODIFIZIERTEN NUCLEOTIDEN ZUR PROTEINEXPRESSION**

RNA WITH A COMBINATION OF UNMODIFIED AND MODIFIED NUCLEOTIDES FOR PROTEIN EXPRESSION

ARN AYANT UNE COMBINAISON DE NUCLÉOTIDES NON MODIFIÉS ET MODIFIÉS POUR L'EXPRESSION PROTÉIQUE

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **31.07.2009 DE 102009035507**
**22.10.2009 DE 102009050308**

(43) Veröffentlichungstag der Anmeldung:
**06.06.2012 Patentblatt 2012/23**

(73) Patentinhaber: **ethris GmbH**
**82152 Martinsried (DE)**

(72) Erfinder:
• **RUDOLPH, Carsten**
**80801 München (DE)**
• **KORMANN, Michael**
**72076 Tübingen (DE)**

(74) Vertreter: **Vossius & Partner**
**Patentanwälte Rechtsanwälte mbB**
**Siebertstrasse 3**
**81675 München (DE)**

(56) Entgegenhaltungen:
**WO-A1-2009/127230 WO-A2-99/14346**
**WO-A2-2007/024708**

• **KARIKO KATALIN ET AL: "Suppression of RNA recognition by Toll-like receptors: The impact of nucleoside modification and the evolutionary origin of RNA", August 2005 (2005-08), IMMUNITY, VOL. 23, NR. 2, PAGE(S) 165-175, XP002634064, ISSN: 1074-7613 Seite 168, rechte Spalte, Absatz 3 - Seite 169, rechte Spalte, Absatz 1**
• **Mutschler E., Geisslinger G., Kroemer H.K., Schäfer-Korting M.: "Mutschler Arzneimittelwirkungen", 1 January 2001 (2001-01-01), Wissenschaftliche Verlagsgesellschaft mbh Stuttgart 2001 pages 621-622,**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft ein Polyribonucleotid, insbesondere Messenger-RNA, das/die eine Kombination aus unmodifizierten und modifizierten Nucleotiden enthält, zur Proteinexpression und die Verwendung derartiger RNAs zur Therapie von Erkrankungen und für diagnostische Verfahren, wobei in dem Polyribonucleotid 5 bis 50% der Uridinnucleotide und 5 bis 50% der Cytidinnucleotide modifizierte Uridinnucleotide bzw. modifizierte Cytidinnucleotide sind und wobei die modifizierten Uridinnucleotide 2-Thiouridin sind und die modifizierten Cytidinnucleotide 5-Methylcytidin sind.

Messenger-RNAs (mRNA) sind Polymere, die aus Nucleosid-Phosphat-Bausteinen hauptsächlich mit Adenosin, Cytidin, Uridin und Guanosin als Nucleosiden aufgebaut sind, die als Zwischenträger die genetische Information von der DNA im Zellkern ins Zytoplasma bringen, wo sie in Proteine übersetzt wird. Sie sind somit als Alternative zur Genexpression geeignet.

Die Erforschung der biochemischen Vorgänge in der Zelle und die Erforschung des menschlichen Genoms haben Zusammenhänge zwischen defizienten Genen und Krankheiten aufgedeckt. Es bestand daher schon lange der Wunsch, auf defizienten Genen beruhende Krankheiten durch Gentherapie zu heilen. Die Erwartungen waren hoch, doch Versuche hierzu scheiterten in der Regel. Ein erster Ansatz zur Gentherapie bestand darin, die intakte DNA eines defizienten oder defekten Gens in einem Vektor in den Zellkern zu bringen, um die Expression des intakten Gens und damit die Bereitstellung des fehlenden oder defekten Proteins zu erreichen. Diese Versuche waren in der Regel nicht erfolgreich und die wenigen erfolgreichen Versuche waren mit erheblichen Nebenwirkungen belastet, insbesondere einer erhöhten Tumorigenese.

Weiterhin gibt es Krankheiten, die auf einem Mangel an Proteinen oder einem Proteindefekt beruhen, ohne dass dies auf einen genetischen Defekt zurückzuführen ist. Auch in einem solchen Fall wird in Betracht gezogen, durch Verabreichung von DNA die entsprechenden Proteine in vivo zu erzeugen. Auch die Bereitstellung von Faktoren, die im Stoffwechsel eine Rolle spielen und aus pathologischen oder nichtpathologischen Gründen gestört oder gehemmt sind, könnten durch eine nebenwirkungsfreie oder -arme Nucleinsäure-Therapie behandelt werden.

Es wurde auch schon vorgeschlagen, mRNAs für die Therapie von Erberkrankungen zu nutzen, um Gendefekte, die zu Krankheiten führen, zu behandeln. Vorteil hierbei ist es, dass die mRNA nur in das Zytoplasma einer Zelle eingebracht werden muss, nicht jedoch in den Kern eingeschleust werden muss. Das Einschleusen in den Kern ist schwierig und ineffizient, außerdem besteht ein erhebliches Risiko, dass die chromosomale DNA verändert wird, wenn der Vektor oder Teile davon in das Genom eingebaut werden.

Zwar konnte gezeigt werden, dass in vitro transkribierte Messenger-RNA in Säugetiergewebe tatsächlich exprimiert werden kann, allerdings bauten sich bei dem Versuch, mRNA für die Therapie von Erkrankungen zu nutzen, weitere Hürden auf. Die mangelnde Stabilität der mRNA führte dazu, dass das gewünschte Protein nicht in ausreichender Menge im Säugetiergewebe zur Verfügung gestellt werden konnte. Ein weiterer wesentlicher Nachteil ergab sich dadurch, dass mRNA erhebliche immunologische Reaktionen auslöst. Es wird davon ausgegangen, dass diese starken Immunreaktionen durch Bindung an Toll-artige Rezeptoren wie TLR3, TLR7, TLR8 und Helicase-RIG-1 entstehen.

Um eine immunologische Reaktion zu verhindern, wurde in WO 2007/024708 vorgeschlagen, RNA einzusetzen, bei der eines der vier Ribonucleotide durch ein modifiziertes Nucleotid ersetzt ist. Insbesondere wurde untersucht, wie sich mRNA verhält, bei der Uridin insgesamt durch Pseudouridin ersetzt ist. Es wurde gefunden, dass ein derartiges RNA-Molekül signifikant weniger immunogen ist. Allerdings war die biologische Aktivität dieser Produkte für eine erfolgreiche Therapie noch nicht ausreichend. Außerdem hat sich herausgestellt, dass sich RNA-Sequenzen, bei denen zwei oder mehr Arten von Nucleotiden vollständig durch Modifikationen ersetzt sind, schwierig oder gar nicht herstellen lassen.

Um dem Körper notwendige oder hilfreiche Proteine bereitzustellen und/oder um eine auf mangelnden oder defizienten Proteinen beruhende Krankheit durch Nucleinsäuren behandeln zu können, ist es wünschenswert, eine Nucleinsäure zur Verfügung zu haben, die Zellen transfizieren kann, die lange genug in der Zelle stabil bleibt und eine ausreichende Menge an Protein liefert, so dass eine allzu häufige Dosierung vermieden wird. Gleichzeitig darf diese Nucleinsäure aber keine immunologischen Reaktionen in erheblichem Ausmaß hervorrufen.

Es war daher Aufgabe der vorliegenden Erfindung, ein Mittel bereitzustellen, das zur Therapie von durch defiziente oder defekte Gene hervorgerufenen Krankheiten bzw. durch mangelnde oder defekte Proteine hervorgerufenen Krankheiten geeignet ist, oder das in vivo notwendige oder hilfreiche Proteine erzeugen kann, das keine oder eine stark verringerte Immunantwort auslöst, in physiologischer Umgebung stabil ist, d.h. nicht sofort nach Verabreichung abgebaut wird und insgesamt als Mittel zur Therapie geeignet ist. Weiterhin war es Aufgabe der Erfindung ein Mittel bereitzustellen für die Therapie von Krankheiten, die sich durch in-vivo Bildung von Proteinen positiv beeinflussen lassen.

Diese Aufgabe wird gelöst mit einem Polyribonucleotid, wie es in Anspruch 1 definiert ist. Insbesondere geeignet ist mRNA, die ein Protein oder Proteinfragment codiert, dessen Defekt oder Mangel für den Körper nachteilig ist bzw. dessen Expression für den Körper von Vorteil ist. Wenn im Folgenden der Begriff "Polyribonucleotid" oder "mRNA" verwendet wird, so ist, wenn der Zusammenhang nicht dagegen spricht, immer davon auszugehen, dass es sich hierbei um ein Polyribonucleotid oder eine mRNA handelt, die ein Protein oder Proteinfragment codiert, das mit einem Leiden oder Mangel, wie oben beschrieben, in Verbindung steht oder ein Protein oder Proteinfragment codiert, das für den Organismus hilfreich oder unterstützend ist.

Es wurde überraschenderweise gefunden, dass die obengenannten Probleme mit Ribonucleinsäure bzw. Polyribonucleotiden (im folgenden allgemein auch als RNA bezeichnet), insbesondere mit Messenger-RNA (mRNA) gelöst werden können, wenn eine RNA eingesetzt wird, die sowohl unmodifizierte als auch modifizierte Nucleotide enthält, wobei es wesentlich ist, dass jeweils ein vorbestimmter Anteil der Uridin- und der Cytidinnucleotide in modifizierter Form vorliegt. Gemäß der vorliegenden Erfindung sind in einem erfindungsgemäßen Polyribonucleotid 5 bis 50% der Uridinnucleotide modifiziert, und zwar 2-Thiouridin, und 5 bis 50% der Cytidinnucleotide modifiziert, und zwar 5-Methylcytidin.

Weiterhin wurde überraschenderweise festgestellt, dass RNA, bei der zwei Arten von Nucleotiden partiell durch jeweils modifizierte Nucleotide ersetzt sind wie in Anspruch 1 angegeben, eine hohe Translations- und Transfektionseffizienz zeigen, d.h. die RNA transfiziert mehr Zellen und erzeugt pro Zelle mehr von dem codierten Protein, als es mit bekannter RNA möglich war. Darüber hinaus ist die erfindungsgemäß modifizierte RNA länger aktiv als die aus dem Stand der Technik bekannte RNA oder unmodifizierte RNA.

Die mit der erfindungsgemäßen RNA erzielten Vorteile werden weder mit unmodifizierter noch mit vollständig modifizierter RNA erhalten. Es hat sich herausgestellt, dass sowohl eine verringerte Immunogenität als auch eine erhöhte Stabilität erzielt werden kann, wenn der Anteil an modifizierten Uridin- und Cytidinnucleotiden in der mRNA gezielt eingestellt wird und mindestens jeweils 5% aber nicht mehr als 50% beträgt. Wird eine mRNA ohne Modifizierungen verwendet, so ist diese höchst immunogen, während dann, wenn alle Uridin- und Cytidinnucleotide in modifizierter Form vorliegen, die biologische Aktivität zu gering ist, um für therapeutische Zwecke eingesetzt werden zu können. RNA, in der der Anteil an modifizierten Nucleotiden sehr hoch ist, kann garnicht oder unter sehr schwierigen Bedingungen hergestellt werden. So wurde festgestellt, dass eine Nucleotidmischung, die nur Pseudouridin statt Uridin und nur modifiziertes Cytosin und/oder modifiziertes Adenosin enthält, keine RNA-Sequenz liefern kann. Überraschenderweise lassen sich aber RNA-Sequenzen, die in der erfindungsgemäßen Art und Weise modifiziert sind, ohne weiteres mit vernünftiger Effizienz herstellen.

Außerdem wurde gefunden, dass die Art der Modifizierung kritisch ist. Die erfindungsgemäß modifizierten mRNAs zeigen eine geringe Immunogenität und haben eine lange Lebensdauer.

Es wurde gefunden, dass die Stabilität der erfindungsgemäßen RNA gegenüber bisher verwendeten Nucleinsäuren stark erhöht ist. So wurde festgestellt, dass die erfindungsgemäße mRNA 10 Tage nach der Transfektion in einer 10-fach höheren Menge nachweisbar ist als unmodifizierte mRNA. Neben hohen Transfektionsraten ermöglicht vor allem die erhöhte Lebensdauer den Einsatz der erfindungsgemäßen mRNA für therapeutische Zwecke, da die hohe Stabilität und damit lange Lebensdauer es ermöglicht, eine Dosierung in längeren Zeitabständen vorzunehmen, die somit auch für den Patienten annehmbar sind.

Erfindungsgemäß wird damit ein besonders vorteilhaftes Mittel für therapeutische Zwecke zur Verfügung gestellt. Die erfindungsgemäße RNA erfüllt die Voraussetzungen, die an ein in der Therapie zu verwendendes Produkt gestellt werden: Als RNA muss sie zur Entfaltung der Wirkung nur in das Zytoplasma und nicht in den Zellkern eingebracht werden, die Gefahr einer Integration in das Genom besteht nicht, die erfindungsgemäße Art der Modifizierung verhindert weitgehend eine Immunreaktion und die Modifikation schützt darüber hinaus die RNA vor einem schnellen Abbau. Damit gelingt es mit der erfindungsgemäßen RNA physiologische Funktionen in Geweben zu generieren oder zu regenerieren, z.B. Funktionen, die durch ein defizientes oder defektes Gen ausgefallen waren, in vivo wiederherzustellen und damit durch defiziente oder defekte Gene hervorgerufene Krankheiten zu behandeln. Weiterhin wurde überraschenderweise gefunden, dass erfindungsgemäße Polyribonucleotide Krankheiten positiv beeinflussen können, indem in vivo Proteine gebildet werden, die auf den Krankheitsverlauf direkt oder indirekt Einfluss nehmen können. Erfindungsgemäß können daher auch Polyribonucleotide bereitgestellt werden, die solche Faktoren codieren, die für den Organismus generell oder in einer bestimmten Situation nützlich und unterstützend sind, z.B. Wachstumsfaktoren, Angiogenesefaktoren, Stimulatoren, Induktoren, Enzyme oder andere biologisch aktive Moleküle.

Die Erfindung wird in der folgenden Beschreibung und den beigefügten Figuren näher erläutert.

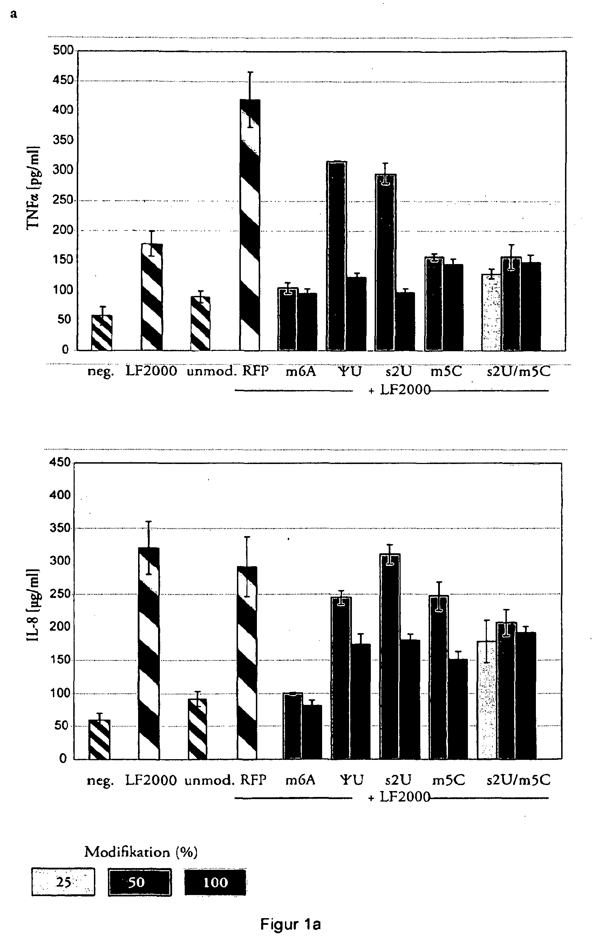

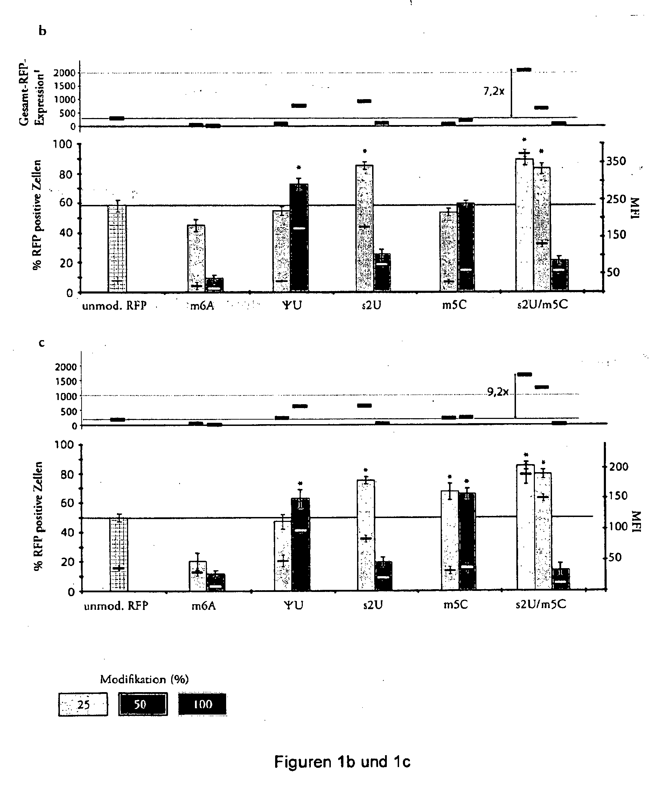

Fig. 1 zeigt den Einfluss unterschiedlicher Nucleotidmodifikationen auf Immunogenität und Stabilität verschiedener mRNAs. Fig. 1A ist ein Diagramm, in dem der TNF-$\alpha$-Pegel nach Verabreichung verschiedener RNAs mit unterschiedlich modifizierten Nucleotiden aufgetragen ist. Unmodifizierte und zu 25% einfach modifizierte RNA führt zu einem hohen Pegel an Entzündungsmarkern und zeigt die hohe Immunogenität dieser RNA, während für erfindungsgemäß zweifach modifizierte RNA die Entzündungsmarker in tolerierbarer Menge vorliegen. Die Figuren 1B und 1C zeigen die biologische Aktivität (Transfektionseffizienz und Expression) von in verschiedener Weise modifizierter mRNA in menschlichen Zellen und Mauszellen als Prozentanteil der für rotfluoreszierendes Protein (RFP) positiven Zellen und die RFP-Menge pro Zelle. Die Diagramme zeigen, dass die von unmodifizierter, einfach modifizierter und vollständig modifizierter RNA kodierten Proteine nur in geringerem Prozentanteil nachgewiesen werden können, während die erfindungsgemäß teilweise zweifach modifizierte RNA aufgrund ihrer höheren Stabilität signifikant höhere Mengen an Protein liefert.

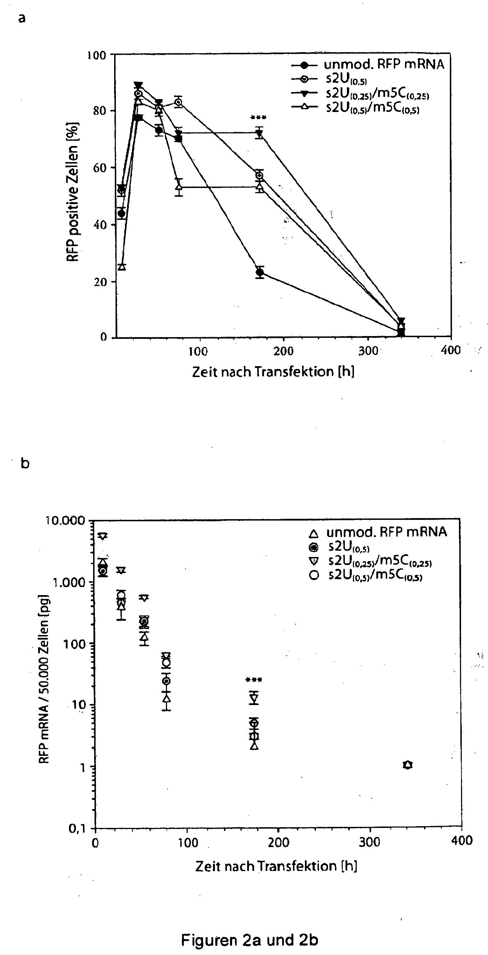

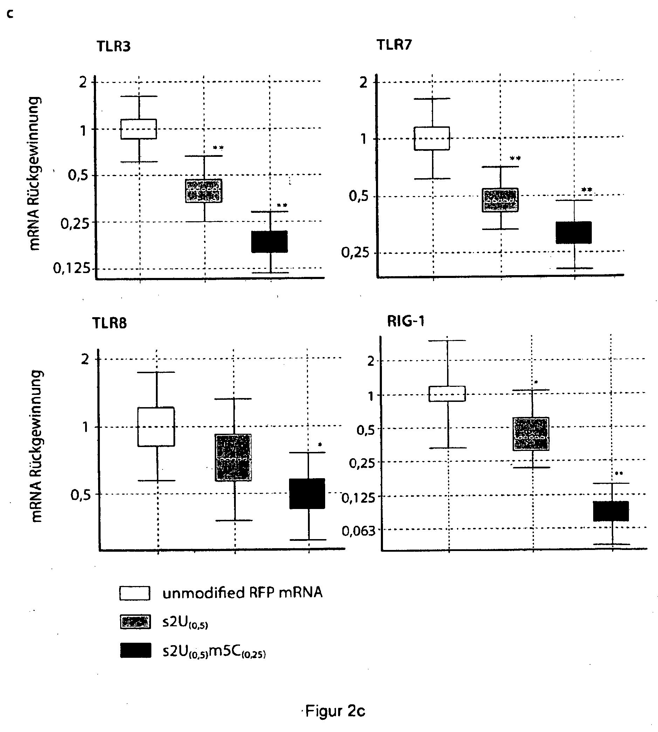

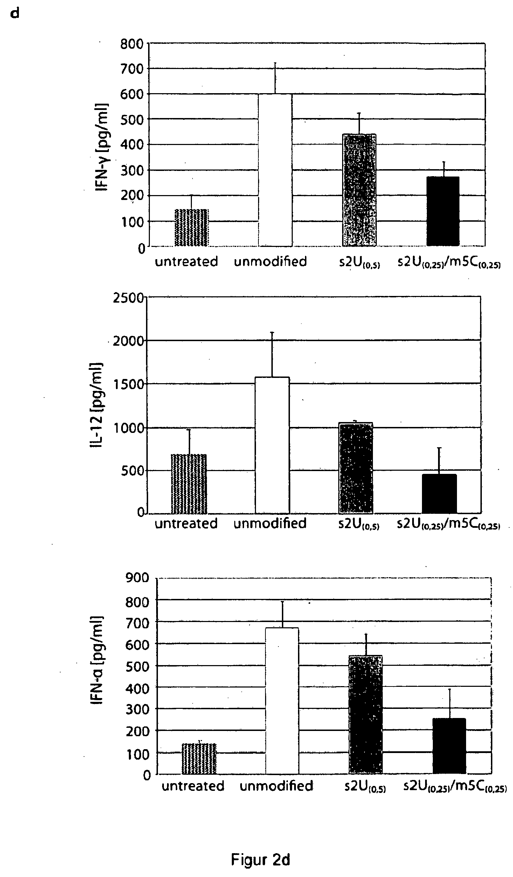

Fig. 2 zeigt für mehrfach modifizierte mRNA höhere Stabilität und längere Expressionsdauer. Fig. 2A und B zeigen jeweils Diagramme, in denen die Expressionsdauer verschiedener modifizierter und unmodifizierter mRNAs aufgetragen ist. Fig. 2C zeigt Daten für die RNA-Immunpräzipitation für unmodifizierte RNA, einfach modifizierte RNA und mehrfach modifizierte RNA. Fig. 2D zeigt Diagramme, in denen die Immunogenität verschiedener mRNAs nach intravenöser in vivo Verabreichung aufgetragen ist. Die Daten zeigen, dass eine erfindungsgemäß zweifach modifizierte RNA eine Kombination aus hoher Stabilität und geringer Immunogenität aufweist.

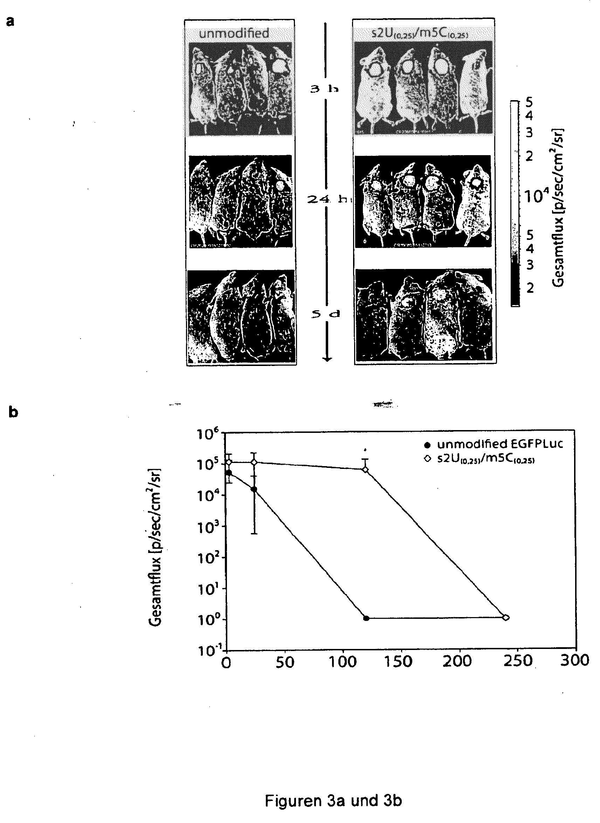

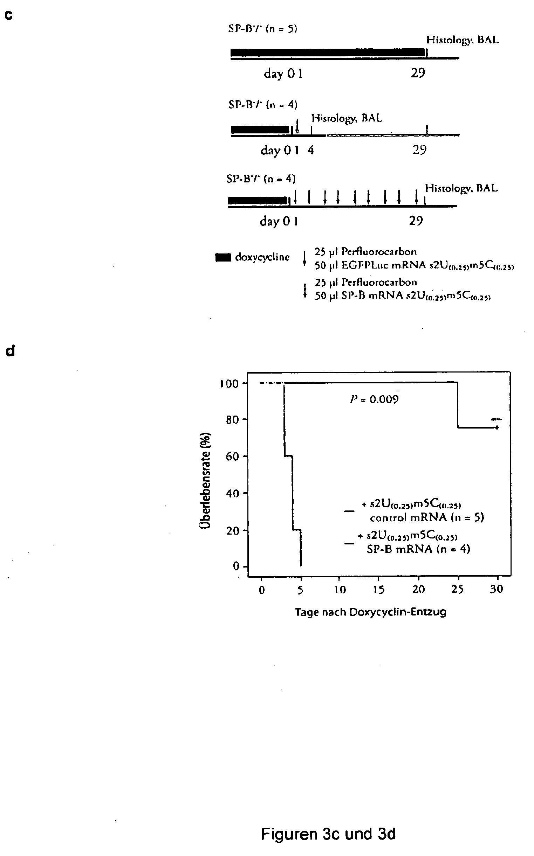

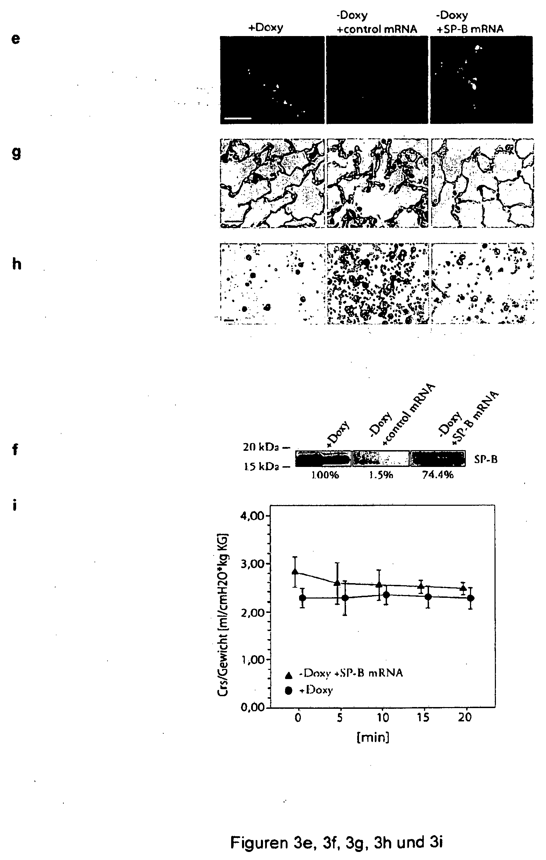

Fig. 3 zeigt verschiedene Testergebnisse, die nach intratrachealer Aerosolapplikation von modifizierter SP-B-mRNA in SP-B konditional-defiziente Mäuse erhalten wurden. Fig. 3A zeigt Biolumineszenzaufnahmen der Lunge von mit unmodifizierter und mehrfach modifizierter RNA behandelten Mäusen. Es ist klar zu sehen, dass nur von erfindungsgemäß modifizierter RNA auch noch nach 5 Tagen eine ausreichende Menge Protein exprimiert wird, während bei unmodifizierter RNA schon nach 3 Stunden die Expression gering ist. Fig. 3B zeigt ein Diagramm, in dem der Flux gegen die Zeit nach der Transfektion aufgetragen ist. Deutlich ist zu erkennen, dass die erfindungsgemäße Modifikation die Expressionsdauer verlängert. Fig. 3C zeigt das Dosierschema für SP-B-mRNA. Fig. 3D zeigt ein Diagramm, das die Überlebensrate für Mäuse, die mit modifizierter mRNA behandelt wurden im Vergleich zu Mäusen, die mit Kontroll-mRNA behandelt wurden, darstellt, wobei die Überlebensrate bei mit erfindungsgemäßer RNA behandelten Mäusen deutlich länger ist. Fig. 3E zeigt ein Immunostaining, wobei zu sehen ist, dass mit erfindungsgemäßer RNA, die SP-B codiert, das SP-B in SP-B defizienten Mäusen rekonstituiert werden konnte. Fig. 3F zeigt als Ergebnis einer semi-quantitativen Western-Blot-Analyse die Verteilung von Proteinen im zellfreien BALF-Überstand. Die Figuren 3G und H zeigen Aufnahmen von Lungenhistologiepräparaten und Bronchoalveolar-Lavage-Präparaten von gemäß 3C behandelten Mäusen. Während Lungen- und Lavagepräparate von Mäusen, die Kontroll-RNA erhalten hatten, die für SP-B-Defizienz üblichen Lungenschäden zeigten, waren die Präparate von mit erfindungsgemäßer RNA behandelten Mäusen unauffällig. Fig. 3I zeigt ein Diagramm zur Lungenverträglichkeit über die Zeit. Die Lungenfunktion blieb über längere Zeit erhalten bei Behandlung mit erfindungsgemäßer RNA, während bei mit Kontroll-RNA behandelten Tieren Lungenschäden gefunden wurden.

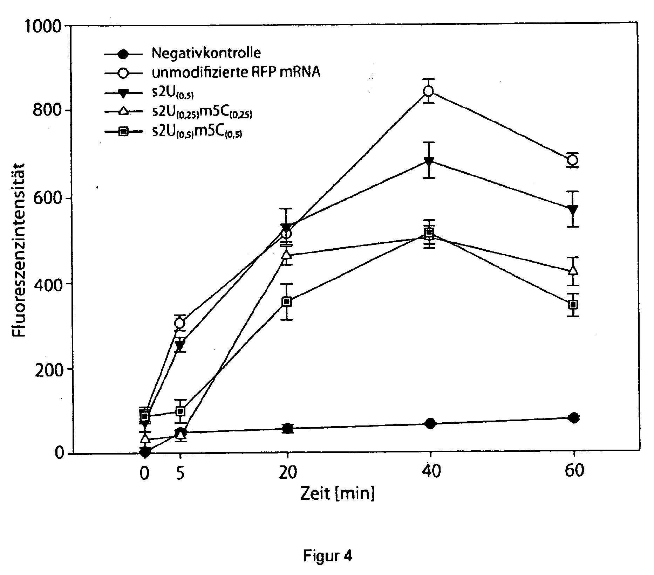

Fig. 4 zeigt ein Diagramm, in dem die Fluoreszenzintensität des gebildeten RFP über die Zeit aufgetragen wurde für unmodifizierte und unterschiedlich modifizierte mRNAs. Die modifizierte mRNA wird im Gegensatz zur unmodifizierten mRNA später und weniger stark translatiert.

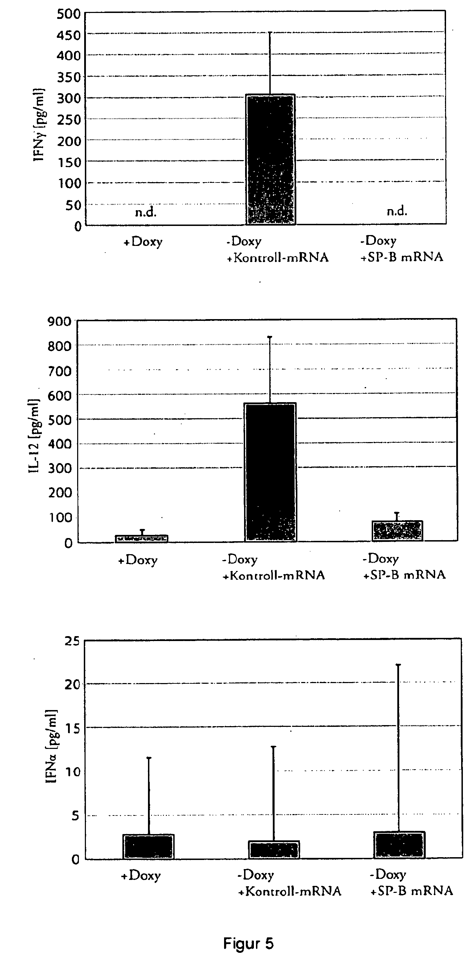

Fig. 5 zeigt drei Diagramme, in dem Entzündungsmarker für mit unterschiedlichen mRNAs behandelte Mäuse aufgetragen sind. Es ist deutlich zu erkennen, dass erfindungsgemäß modifizierte RNA keine entzündlichen Reaktionen hervorruft, während unmodifizierte RNA zu einer starken Immunreaktion führt.

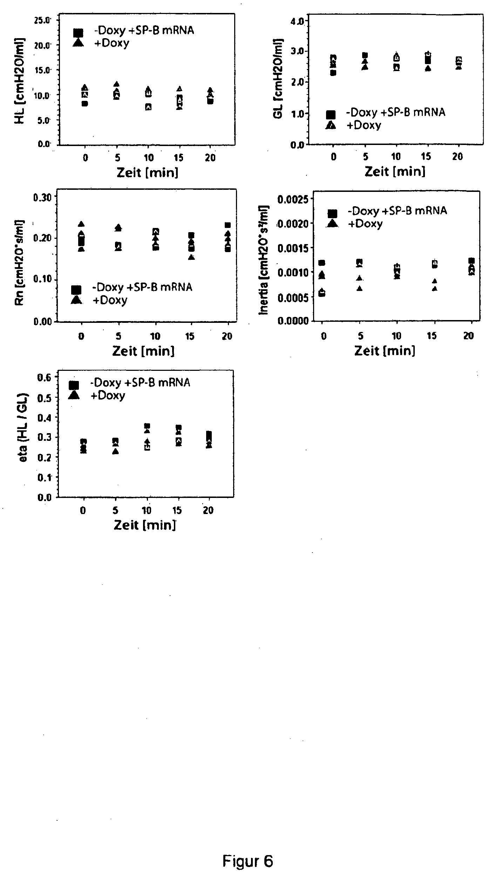

Fig. 6 zeigt Diagramme, in denen unterschiedliche lungentypische Parameter für mit unterschiedlichen erfindungsgemäßen mRNAs behandelte Mäuse aufgetragen sind. Die Parameter sind Gewebeelastizität (HL), Gewebedämpfung (GL), Gewebeträgheit, Atemwegswiderstand (Rn) und Lungengewebekomposition Eta (GL/HL). Für die efindungsgemäßen RNAs war keiner der Parameter gegenüber der positiven Kontrollgruppe verschlechtert.

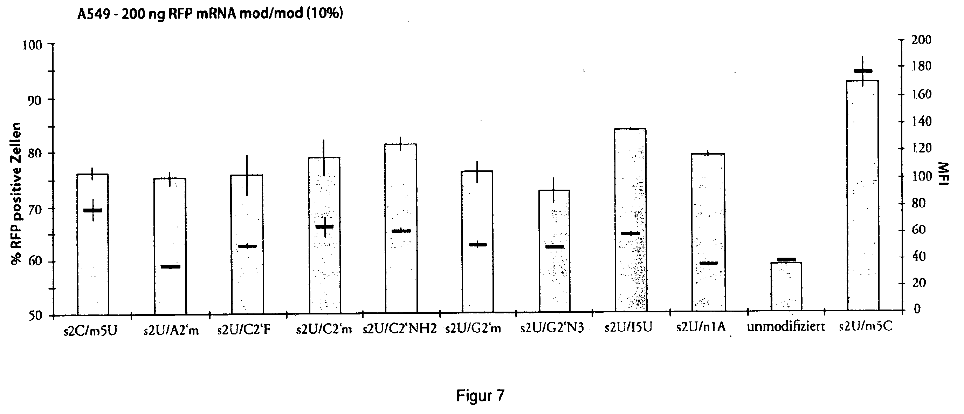

Fig. 7 zeigt die Expressionsfähigkeit unterschiedlich modifizierter mRNA in einem Diagramm, in dem der Prozentanteil an RFP positiven Zellen für mRNA mit unterschiedlichem Anteil an modifizierten Nucleotiden aufgetragen ist. Der Vergleich zeigt, dass nur erfindungsgemäß modifizierte mRNA zu langanhaltender Expression führt, während nicht erfindungsgemäß modifizierte mRNA sowohl in menschlichen Zellen als auch in Mauszellen in geringerem Ausmaß exprimiert.

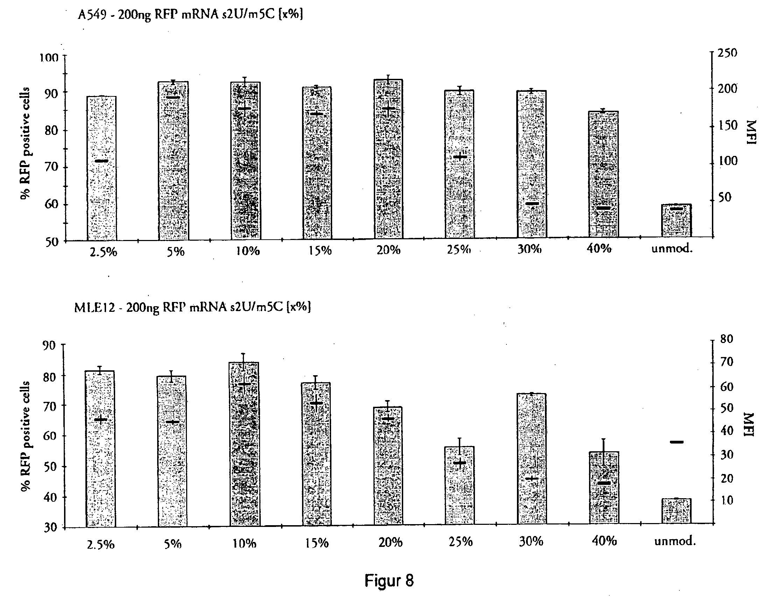

Fig. 8 zeigt die Expressionsfähigkeit unterschiedlich modifizierter mRNA in einem Diagramm, in dem der Prozentanteil an RFP positiven Zellen für mRNA mit unterschiedlich modifizierten Nucleotiden aufgetragen ist. Der Vergleich zeigt, dass nur erfindungsgemäß modifizierte mRNA zu langanhaltender Expression führt, während nicht erfindungsgemäß modifizierte mRNA sowohl in menschlichen Zellen als auch in Mauszellen in geringerem Ausmaß exprimiert.

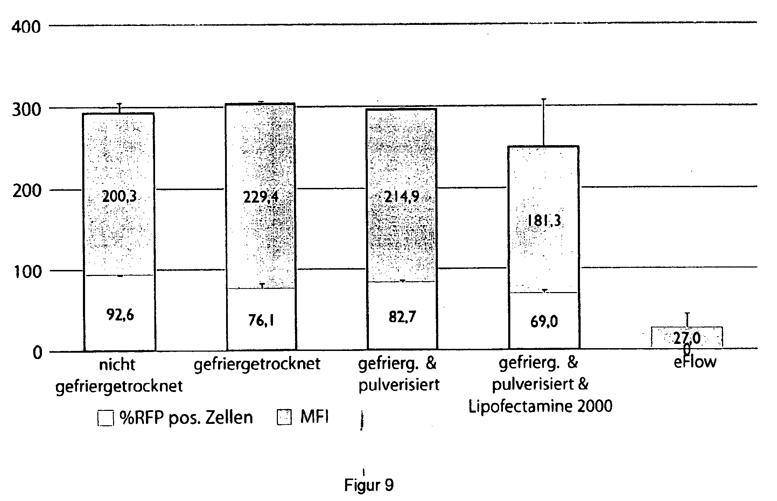

Fig. 9 zeigt die Stabilität von gefriergetrockneter erfindungsgemäßer RNA.

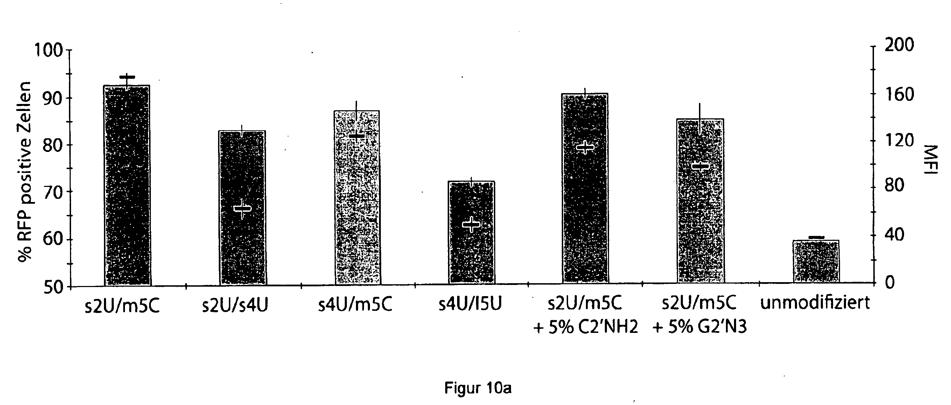

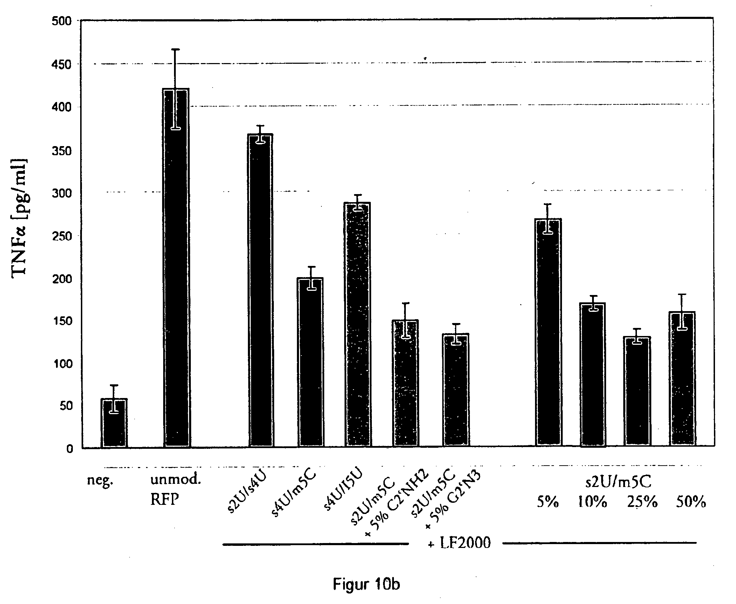

Fig. 10A zeigt ein Diagramm, in dem die Transfektionseffizienz für verschieden modifizierte Nucleotide aufgetragen ist. Es ist deutlich zu erkennen, dass die höchste Transfektionseffizienz mit RNA erzielt wird, bei der 10% der Uridinnucleotide und 10% der Cytidinnucleotide und gegebenenfalls noch 5% weitere Nucleotide modifiziert sind. Fig. 10B zeigt ein Diagramm, in dem die TNF-$\alpha$-Bildung als Marker für die immunologische Reaktion für RNA mit unterschiedlich modifizierten Nucleotiden aufgetragen ist. Es sind die Ergebnisse eines ELISA von human PBMCs, die jeweils mit 5 μg mRNA transfiziert wurden. Die Modifikationsrate war, wenn nicht anders angegeben, jeweils 10%. Es ist deutlich erkennbar, dass RNA, bei der zwischen 5 und 50% der Uridinnucleotide und Cytidinnucleotide modifiziert sind, eine deutlich verringerte Immunogenizität im Vergleich zu unmodifizierter RNA hat.

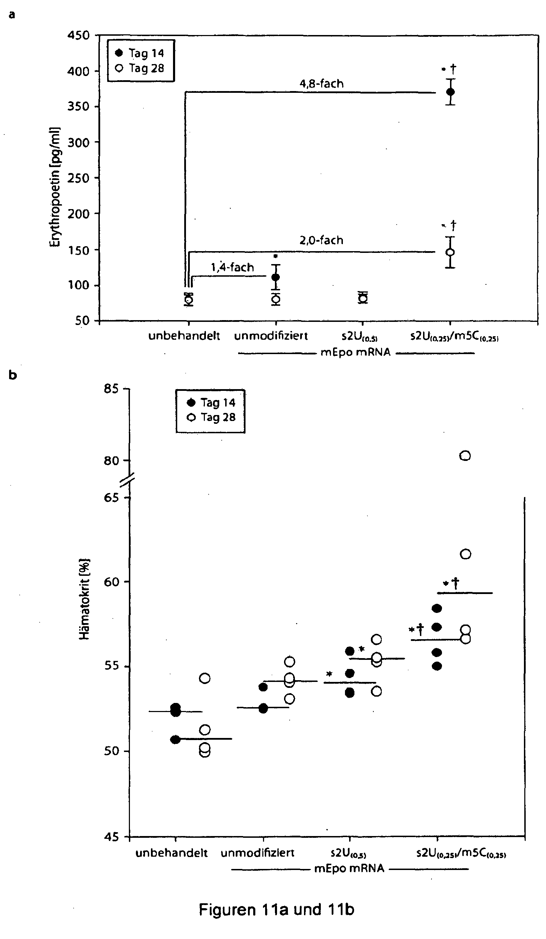

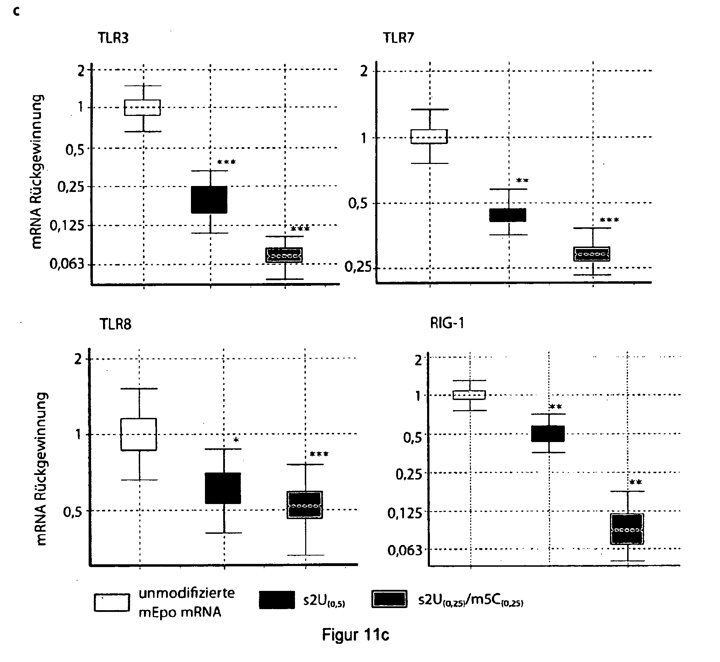

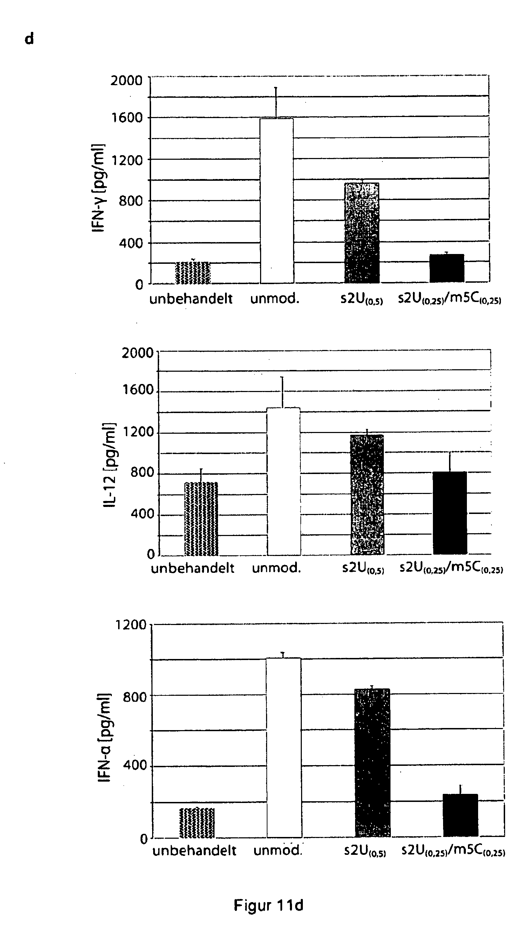

Fig. 11 zeigt die Ergebnisse verschiedener Tests, mit denen die Stabilität und Immunogenität von erfindungsgemäß modifizierter mRNA, die EPO codiert, gemessen wurde. Diagramm 11 (a) zeigt den Anteil an Erythropoetin, der 14 Tage nach Verabreichung von EPO codierender mRNA, die in unterschiedlicher Weise modifiziert ist, nachweisbar ist. Deutlich erkennbar ist, dass nach 14 Tagen der Anteil an EPO bei Mäusen, denen erfindungsgemäß modifizierte mRNA injiziert wurde, 4,8 Mal höher ist als bei unbehandelten Mäusen, aber auch 4,8 Mal höher als bei mit unmodifizierter RNA behandelten Mäusen und immer noch 2,5-fach höher ist als bei mit einfach modifizierter RNA behandelten Mäusen.

Diagramm 11 (b) zeigt Hämatokritwerte 14 Tage bzw. 28 Tage nach Verabreichung von EPO codierender mRNA mit unterschiedlichen Modifikationen. Das Diagramm zeigt deutlich, dass mit erfindungsgemäß modifizierter mRNA behandelte Mäuse einen erheblich höheren Hämatokritwert haben.

In den Diagrammen von Fig. 11(c) ist die Bildung der für eine immunologische Reaktion typischen Faktoren aufgetragen. Es zeigt sich, dass alle vier Entzündungsmarker bei der Verabreichung von unmodifizierter mRNA erhöht sind, während bei erfindungsgemäß modifizierter RNA eine immunologische Reaktion kaum nachweisbar ist.

Die Diagramme von Fig. 11(d) zeigen die entsprechenden Werte für IFN-$\alpha$ und IL-12, die ebenfalls Entzündungsmarker sind. Auch hier zeigt sich, dass erfindungsgemäß modifizierte mRNA praktisch keine immunologische Reaktion hervorruft im Gegensatz zu unmodifizierter mRNA.

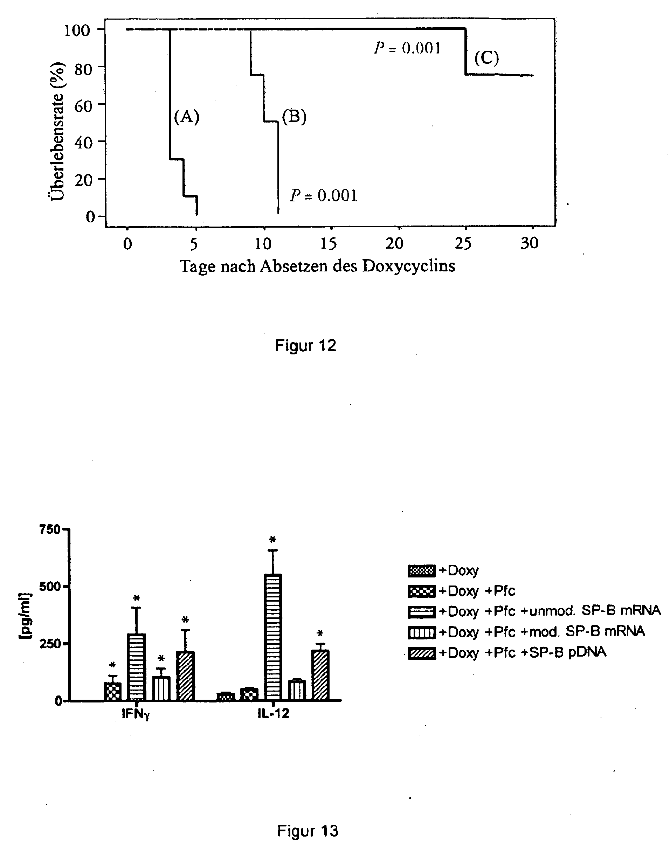

Fig. 12 zeigt ein Diagramm, in dem die Überlebensrate von drei Gruppen von Mäusen aufgetragen ist, die erfindungsgemäß modifizierte SP-B-mRNA zweimal in einer Woche (B) bzw. 28 Tage lang zweimal pro Woche (C), oder in der Vergleichsgruppe modifizierte EGFPLuc-mRNA (A) erhielten. Es zeigt sich, dass die Mäuse nur solange überleben, wie sie SP-B-mRNA erhalten (B, C). Ohne Zuführung von SP-B-mRNA sterben die Mäuse (A).

Fig. 13 zeigt Cytokinspiegel in der broncheoalveolären Lavage von Mäusen 8 Stunden nach Applikation von unmodifizierter SP-B-mRNA, erfindungsgemäß modifizierter SP-B-mRNA bzw. von SP-B-Plasmid-DNA. Die Ergebnisse zeigen, dass im Gegensatz zur intratrachealen Applikation von unmodifizierter mRNA oder Plasmid-DNA, die jeweils zu einem starken Anstieg der Entzündungsmarker IFN$\gamma$ und IL-12 führen, bei Verabreichung von erfindungsgemäß modifizierter SP-B-mRNA die Entzündungsmarker im Vergleich zur unbehandelten Gruppe bzw. zur mit Perfluorcarbon behandelten Gruppe praktisch nicht erhöht sind.

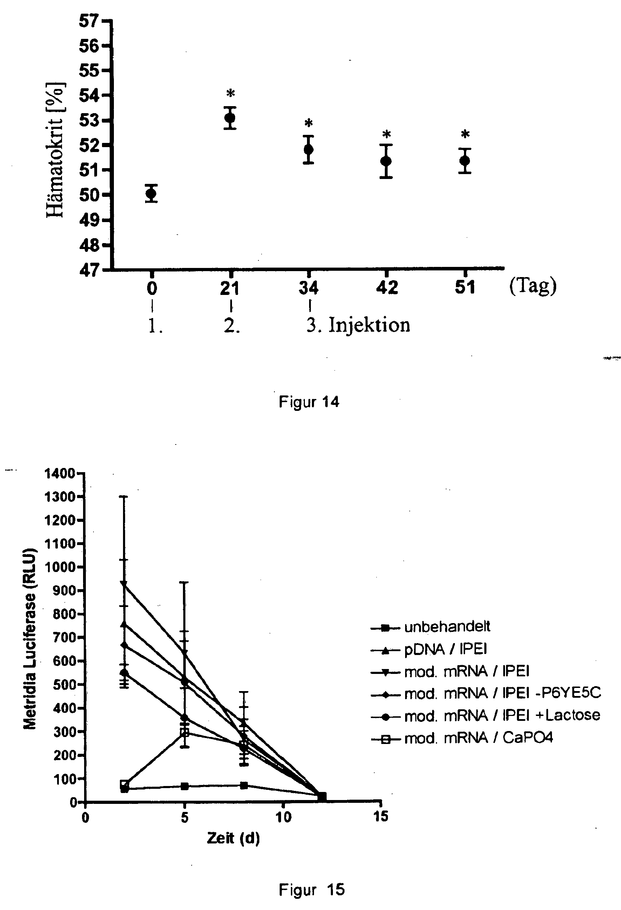

Fig. 14 zeigt Hämatokritwerte, wie sie nach wiederholter Applikation von erfindungsgemäß modifizierter mEPO-mRNA erhalten werden. Die Ergebnisse zeigen, dass die wiederholte Applikation von erfindungsgemäß modifizierter mEPO-mRNA gut vertragen wird und zu langanhaltender Erhöhung des Hämatokrits führt.

Fig. 15 zeigt die Luciferaseexpression von Zellen, die mit Titanimplantaten inkubiert wurden, die mit unterschiedlichen Formen von erfindungsgemäß modifizierter RNA enthaltenden Beschichtungen versehen waren. Es zeigte sich, dass erfindungsgemäß modifizierte RNA, die in einer Beschichtung aus verzögert freisetzendem Polymer enthalten war, die auf Titanplatten aufgebracht wurde, und daraus nach und nach abgegeben wurde, ihre Aktivität nicht verlor.

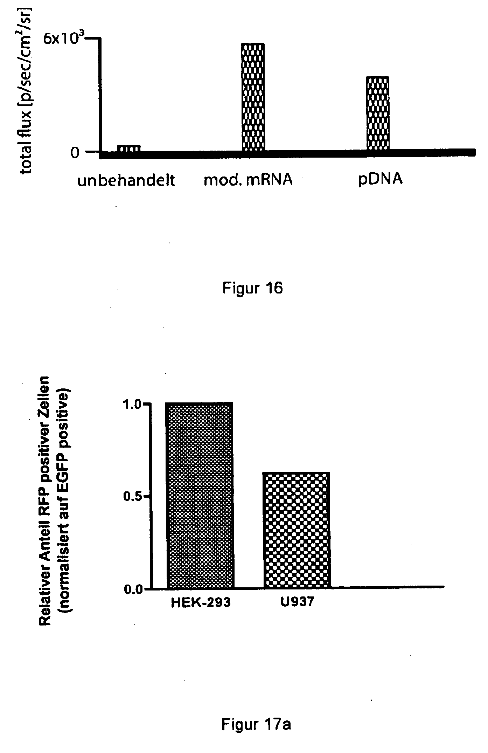

Fig. 16 zeigt die Luciferaseexpression für auf Titanimplantate aufgebrachte Beschichtungen, die modifizierte mRNA enthielten. Es zeigte sich, dass die Proteinexpression für erfindungsgemäß modifizierte mRNA weitaus höher war als für unbehandelte RNA. aber auch höher war als für Plasmid-DNA.

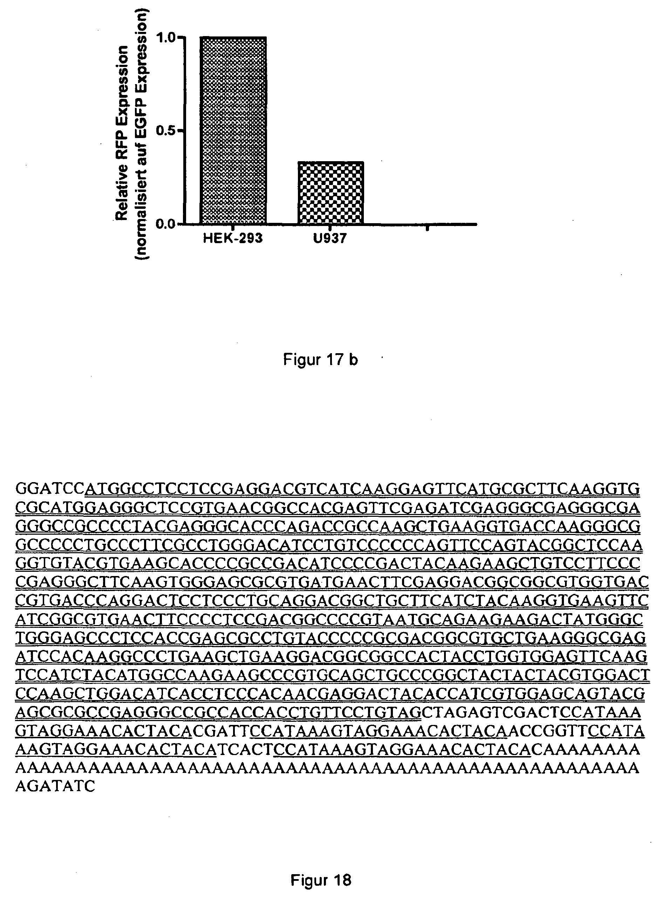

Figuren 17A und 17B zeigen den relativen Anteil von RFP-positiven Zellen bzw. der relativen RFP-Expression von mRNA, die Mikro-RNA-Bindungsstellen für Mikro-RNA 142-3p aufweist. Es zeigte sich, dass der Anteil an RFP-positiven Zellen für Mikro-RNA-Bindungsstellen aufweisende RNA geringer war und die Expression des codierten Proteins erheblich geringer war in den Zellen, die die entsprechende Mikro-RNA 142-3p enthielten.

Figur 18 zeigt die Sequenz einer durch Einbau von Mikro-RNA-Bindungsstellen modifizierten RNA, die RFP codiert. Grau unterlegt dargestellt ist die RFP Sequenz. Unterstrichen ist die vierfache Tandem-Wiederholung der microRNA Bindungsstelle für die microRNA 142-3p (hellgrau unterlegt) mit den Abstandssequenzen (nicht unterlegt).

Erfindungsgemäß werden ein Polyribonucleotidmolekül mit partiell mehrfach modifizierten Nucleotiden wie in Anspruch 1 angegeben, eine entsprechende partiell mehrfach modifizierte mRNA, eine entsprechende IVT-mRNA, sowie die Verwendung der RNA-Moleküle zur Herstellung eines Arzneimittels zur Behandlung von auf defizienten oder defekten Genen beruhenden Krankheiten oder zur Behandlung von Krankheiten, die durch Bereitstellung von Proteinen in vivo, wie Faktoren, Stimulatoren, Induktoren oder Enzyme gelindert oder geheilt werden können, bereitgestellt. Beschrieben wird auch, dass die erfindungsgemäße mRNA kombiniert werden kann mit Targetbindungsstellen, Zielführungssequenzen und/oder mit Mikro-RNA-Bindungsstellen, um eine Aktivität der gewünschten mRNA nur in den geeigneten Zellen zuzulassen. Die erfindungsgemäße RNA kann ferner mit microRNA's oder shRNAs downstream des 3'PolyA-Schwanzes kombiniert werden. Die Wirkungsdauer der erfindungsgemäßen RNA kann durch weitere gezielte Modifikationen eingestellt oder verlängert werden.

Ein Gegenstand der Erfindung ist somit eine wie in Anspruch 1 definierte RNA mit erhöhter Stabilität und verringerter Immunogenität. Die erfindungsgemäße RNA kann in an sich bekannter Weise erzeugt werden. In der Regel wird sie durch Transkription einer DNA, die das intakte oder gewünschte Protein codiert, das ein Leiden beeinflussen kann oder dessen Mangel oder defiziente Form eine Krankheit verursacht, erzeugt.

Unter RNA soll im Rahmen der vorliegenden Erfindung jedes Polyribonucleotidmolekül verstanden werden, das, wenn es in die Zelle gelangt, zur Expression eines Proteins oder Fragments davon, geeignet ist bzw. zu einem Protein oder Fragment davon translatierbar ist. Der Begriff "Protein" umfasst dabei jede Art von Aminosäuresequenz, d.h. Ketten von zwei oder mehr Aminosäuren, die jeweils über Peptidverbindungen verknüpft sind und schließt Peptide sowie Fusionsproteine mit ein.

Die erfindungsgemäße RNA enthält eine Ribonucleotidsequenz, die ein Protein oder Fragment davon codiert, dessen Funktion in der Zelle oder in der Umgebung der Zelle gebraucht wird oder nützlich ist, z.B ein Protein, dessen Fehlen oder defekte Form Auslöser für eine Krankheit oder ein Leiden ist, dessen Bereitstellung eine Krankheit oder ein Leiden lindern oder verhindern kann, oder ein Protein, das einen Prozess, der für den Körper nützlich ist, in einer Zelle oder in ihrer Umgebung fördern kann. In der Regel enthält die erfindungsgemäße RNA die Sequenz für das komplette Protein oder eine funktionelle Variante davon. Weiterhin kann die Ribonucleotidsequenz ein Protein, das als Faktor, Induktor, Regulator, Stimulator oder Enzym wirkt, oder ein funktionelles Fragment davon codieren, wobei dieses Protein ein solches ist, dessen Funktion notwendig ist, um eine Störung, insbesondere Stoffwechselstörung zu beheben oder um Prozesse, wie die Bildung neuer Gefäße, Gewebe etc. in vivo zu initiieren. Unter funktioneller Variante wird hierbei ein Fragment verstanden, das in der Zelle die Funktion des Proteins, dessen Funktion in einer Zelle gebraucht wird oder dessen Fehlen oder Defekt krankheitsverursachend ist, übernehmen kann. Darüber hinaus kann die erfindungsgemäße RNA auch weitere funktionelle Bereiche und/oder 3'- und 5'-Nichtcodierungsbereiche aufweisen. Die 3'- und/oder 5'-nichtcodierenden Bereiche können die natürlicherweise das codierte Protein flankierenden Bereiche sein oder aber artifizielle Sequenzen, die zur Stabilisierung der RNA beitragen. Der Fachmann kann die jeweils geeigneten Sequenzen hierfür durch Routineversuche auffinden.

So kann die RNA eine m7GpppG-Cap, eine Internal Ribosome Entry Site (IRES) enthalten und/oder einen PolyA-Tail am 3'-Ende, insbesondere um die Translation zu verbessern. Die RNA kann weitere die Translation fördernde Bereiche aufweisen. Wesentlich für die erfindungsgemäße RNA ist ihr Anteil an modifizierten Nucleotiden wie in Anspruch 1 definiert.

Eine erfindungsgemäße RNA mit erhöhter Stabilität und verringerter Immunogenität wird erhalten, indem zu ihrer Herstellung eine Nucleotidmischung verwendet wird, in der der Anteil der modifizierten Cytidinnucleotide (5-Methylcytidin) und der modifizierten Uridinnucleotide (2-Thiouridin) eingestellt wird. Bevorzugt wird die erfindungsgemäße RNA hergestellt mit einer Nucleotidmischung, die sowohl unmodifizierte als auch modifizierte Nucleotide enthält, wobei 5 bis 50% der Cytidinnucleotide und 5 bis 50% der Uridinnucleotide modifiziert sind, wobei die modifizierten Cytidinnucleotide 5-Methylcytidin und die modifizierten Uridinnucleotide 2-Thiouridin sind. Die adenosin- und guanosinhaltigen Nucleotide können unmodifiziert sein. Es kann ebenfalls eine Nucleotidmischung verwendet werden, in der einige der ATPs und/oder GTPs ebenfalls modifiziert sind, wobei deren Anteil 20% nicht übersteigen sollte, und wobei bevorzugt deren Anteil, falls vorhanden, in einem Bereich von 0,5 bis 10% liegen sollte.

Somit wird eine mRNA zur Verfügung gestellt, die 5 bis 50% modifizierte Cytidinnucleotide und 5 bis 50% Uridinnucleotide sowie 50 bis 95% unmodifizierte Cytidinnucleotide, 50 bis 95% unmodifizierte Uridinnucleotide aufweist, wobei die modifizierten Cytidinnucleotide 5-Methylcytidin und die modifizierten Uridinnucleotide 2-Thiouridin sind und wobei die Adenosin- und Guanosinnucleotide unmodifiziert oder teilweise modifiziert sein können, wobei sie bevorzugt in unmodifizierter Form vorliegen.

Bevorzugt sind 10 bis 35% der Cytidin- und Uridinnucleotide modifiziert und besonders bevorzugt liegt der Anteil der modifizierten Cytidinnucleotide in einem Bereich von 7,5 bis 25% und der Anteil der modifizierten Uridinnucleotide in einem Bereich von 7,5 bis 25%. Es wurde gefunden, dass tatsächlich ein relativ geringer Anteil, z.B. nur jeweils 10% an modifizierten Cytidin- und Uridinnucleotiden, die gewünschten Eigenschaften erzielen kann, unter der Voraussetzung, dass es sich um die erfindungsgemäßen Modifizierungen handelt.

Die Art der Modifikation der Nucleoside hat Einfluss auf die Stabilität und damit die Lebensdauer und biologische Aktivität der mRNA. Geeignete Modifikationen sind in der folgenden Tabelle aufgeführt:

| Bezeichnung | Basenmodifikation (5-Position) | Zuckermodifikation (2'-Position) | natürlich in mRNA |
|---|---|---|---|
| Uridin | | | |
| 5-Methyluridin-5'-Triphosphat (m5U) | $CH_3$ | - | nein |
| 5-Iodouridin-5'-Triphosphat (I5U) | J | - | nein |
| 5-Bromouridin-5'-Triphosphat (Br5U) | Br | - | nein |
| 2-Thiouridin-5'-Triphosphat (S4U) | S (in 2 Position) | - | nein |
| 4-Thiouridin-5'-Triphosphat (S2U) | S (in 4 Position) | - | nein |
| 2'-Methyl-2'-deoxyuridin-5'-Triphosphat (U2'm) | - | $CH_3$ | ja |
| 2'-Amino-2'-deoxyuridin-5'-Triphosphat (U2'NH2) | - | $NH_2$ | nein |
| 2'-Azido-2'-deoxyuridin-5'-Triphosphat (U2'N3) | - | $N_3$ | nein |
| 2'-Fluoro-2'-deoxyuridin-5'-Triphosphat (U2'F) | - | F | nein |
| Cytidin | | | |
| 5-Methylcytidin-5'-Triphosphat (m5C) | $CH_3$ | - | ja |
| - 5-Iodocytidin-5'-Triphosphat (I5C) | J | - | nein |
| 5-Bromocytidin-5'-Triphosphat (BrSC) | Br | - | nein |
| 2-Thiocytidin-5'-Triphosphat (S2C) | S (in 2 Position) | - | nein |
| 2'-Methyl-2'-deoxycytidin-5'-Triphosphat (C2'm) | - | $CH_3$ | ja |
| 2'-Amino-2'-deoxycytidin-5'-Triphosphat (C2'NH2) | - | $NH_2$ | nein |
| 2'-Azido-2'-deoxycytidin-5'-Triphosphat (C2'N3) | - | $N_3$ | nein |
| 2'-Fluoro-2'-deoxycytidin-5'-Triphosphat (C2'F) | - | F | nein |
| Adenosin | | | |
| N6-Methyladenosin-5'-Triphosphat (m6A) | $CH_3$ (in 6-Position) | - | ja |
| N1-Methyladenosin-5'-Triphosphat (m1A) | $CH_3$ (in 1-Position) | - | nein |
| 2'-O-Methyladenosin-5'-Triphosphat (A2'm) | - | $CH_3$ | ja |
| 2'-Amino-2'-deoxyadenosin-5'-Triphosphat (A2'NH2) | - | $NH_2$ | nein |
| 2'-Azido-2'-deoxyadenosin-5'-Triphosphat (A2'N3) | - | $N_3$ | nein |
| 2'-Fluoro-2'-deoxyadenosin-5'-Triphosphat (A2'F) | - | F | nein |
| Guanosin | | | |
| N1-Methylguanosin-5'-Triphosphat (m1G) | $CH_3$ (in 1-Position) | - | nein |
| 2'-O-Methylguanosin-5'-Triphosphat (G2'm) | - | $CH_3$ | ja |
| 2'-Amino-2'-deoxyguanosin-5'-Triphosphat (G2'NH2) | - | $NH_2$ | nein |
| 2'-Azido-2'-deoxyguanosin-5'-Triphosphat (G2'N3) | - | $N_3$ | nein |
| 2'-Fluoro-2'-deoxyguanosin-5'-Triphosphat (G2'F) | - | F | nein |

Es wurde gefunden, dass besonders gute Ergebnisse dann erzielt werden, wenn die erfindungsgemäße RNA 2'-Thiouridin als modifiziertes uridinhaltiges Nucleotid und 5'-Methylcytidin als modifiziertes Cytidinnucleotid enthält, wobei jeweils 5 bis 50% der Uridin- bzw. Cytidinnucleotide entsprechend modifiziert sind. In einer besonders bevorzugten Ausführungsform sind diese beiden Nucleotide jeweils in einem Anteil von 10 bis 30% vorhanden. In anderer Art modifizierte Nucleotide können ggf. hinzukommen, solange der Gesamtanteil an modifizierten Nucleotiden 50% der jeweiligen Nucleotidart nicht übersteigt.

Bevorzugt ist ein Polyribonucleotid, bei dem 5 bis 30% und insbesondere 7,5 bis 25% der Uridinnucleotide 2'-Thiouridinnucleotide sind, und 5 bis 30% und insbesondere 7,5 bis 25% der Cytidinnucleotide 5'-Methylcytidinnucleotide sind, wobei die Adenosin- und Guanosinnucleotide unmodifizierte oder teilweise modifizierte Nucleotide sein können. In einer bevorzugten Ausführungsform weist diese erfindungsgemäße mRNA zusätzlich eine 7'-Methylguanosinkappe und/oder ein Poly(A)-Ende auf. Somit wird in einer bevorzugten Ausführungsform die mRNA in ihrer reifen Form, d.h. mit einer GppG-Kappe, einer IRES und/oder einen PolyA-Schwanz hergestellt.

Die für eine spezifische RNA optimalen Anteile an 2-Thiouridin bzw. 5-Methylcytidin können mit Routineversuchen ermittelt werden.

Als optimal wird in diesem Zusammenhang eine mRNA bezeichnet, deren Immunogenität so gering ist, dass sie den behandelten Organismus nicht belastet und die eine vorbestimmte Stabilität und damit vorbestimmte Expressionsdauer aufweist. Verfahren zur Überprüfung und Bestimmung dieser Eigenschaften sind dem Fachmann bekannt und werden unten und in den Beispielen beschrieben.

Die erfindungsgemäße RNA kann in an sich bekannter Weise hergestellt werden. Geeignet ist z.B. ein Verfahren, bei dem die erfindungsgemäße mRNA durch in-vitro-Transkription aus einer Mischung von ATP, CTP, GTP und UTP hergestellt wird, wobei 5 bis 50%, bevorzugt 5 bis 30% und insbesondere 7,5 bis 25% der Cytidinnucleotide 5-Methylcytidin sind und 5 bis 50%, bevorzugt 5 bis 30% und insbesondere 7,5 bis 25% der Uridinnucleotide 2-Thiouridin sind und der Rest unmodifiziert ist. Guanosin- und Adenosin-Nucleoside, insbesondere Adenosin, können gegebenenfalls ebenfalls modifiziert sein. Wesentlich für die Erfindung ist jedoch die Modifikation von UTP und CTP als 2-Thiouridin bzw. 5-Methylcytidin im angegebenen Bereich. Wenn der Anteil an modifiziertem UTP und/oder CTP geringer oder höher ist, werden die vorteilhaften Eigenschaften nicht mehr erzielt. So wurde gefunden, dass außerhalb der beanspruchten Bereiche die mRNA nicht mehr so stabil ist. Bei einem geringeren Anteil an Modifikation sind außerdem immunologische Reaktionen zu befürchten. Um das geeignete Verhältnis von unmodifizierten und modifizierten Nucleotiden einzustellen, wird geeigneterweise die RNA erzeugt unter Verwendung einer Nucleotidmischung deren Nucleosidanteile entsprechend dem gewünschten Verhältnis teilweise modifiziert und teilweise unmodifiziert sind, wobei erfindungsgemäß mindestens 5% der Uridinnucleoside und mindestens 5% der Cytidinnucleoside modifiziert sind, insgesamt aber nicht mehr als jeweils 50 % Uridinnucleoside bzw. Cytidinnucleoside modifiziert sind. Weitere Nucleoside, d.h. Adenosine und Guanosine, können modifiziert sein, allerdings sollte auch für diese Nucleoside eine obere Grenze von 50% Modifikation, bevorzugt 20%, nicht überschritten werden. Bevorzugt sind nur die entsprechenden Anteile der Uridinnucleoside und Cytidinnucleoside modifiziert.

Die Länge der erfindungsgemäß eingesetzten mRNA hängt ab von dem Genprodukt bzw. Protein oder Proteinfragment, das bereitgestellt bzw. ergänzt werden soll. Die mRNA kann daher sehr kurz sein, z.B. nur 20 oder 30 Nucleotide aufweisen, oder aber entsprechend der Länge des Gens mehrere Tausend Nucleotide aufweisen. Der Fachmann kann die jeweils geeignete Sequenz in üblicher Weise auswählen. Wesentlich ist, dass die Funktion des eine Krankheit verursachenden Proteins, des eine Krankheit hindernden oder verhindernden Proteins bzw. des eine nützliche Eigenschaft beisteuemden Proteins, für das die mRNA eingesetzt werden soll, bereitgestellt werden kann.

Zur Herstellung der erfindungsgemäßen RNA wird 2'-Thiouridin als modifiziertes uridinhaltiges Nucleotid verwendet. Weiterhin wird 5'-Methylcytidin als modifiziertes Cytidinnucleotid verwendet. Somit kann zur Herstellung der erfindungsgemäßen RNA eine Nucleotidmischung eingesetzt werden, die neben ATP und GTP jeweils 95 bis 50% unmodifiziertes CTP und 95 bis 50% unmodifiziertes UTP sowie 5 bis 50% 2'-Thiouridinnucleotide und 5 bis 50% Methylcytidinnucleotide enthält. Besonders bevorzugt ist ein Polyribonucleotid, bei dem 5 bis 30 und insbesondere 7,5 bis 25% der Uridinnucleotide 2'-Thiouridinnucleotide sind, 5 bis 30% und insbesondere 7,5 bis 25% der Cytidinnucleotide 5'-Methylcytidinnucleotide sind, wobei die Adenosin- und Guanosinnucleotide unmodifizierte Nucleotide sind. Eine derartige Kombination führt zur Herstellung einer teilweise modifizierten RNA, die sich durch besonders hohe Stabilität auszeichnet. Es konnte gezeigt werden, dass RNA, die hergestellt wurde mit einer Nucleotidmischung, die als CTP bzw. UTP jeweils 5 bis 50% 2-Thiouridin- bzw. 5-Methylcytidin-Alucleotide enthielt, besonders stabil ist, d.h. eine im Vergleich zu unmodifizierter oder in bekannter Weise modifizierter RNA bis zu 10-fach erhöhte Lebensfähigkeit aufwies.

Weiterhin ist es bevorzugt, dass das aus unmodifizierten und modifizierten Nucleotiden aufgebaute Polyribonucleotidmolekül eine 7'-Methylguanosinkappe und/oder ein Poly(A)-Ende aufweist. Darüber hinaus kann die RNA noch zusätzliche Sequenzen aufweisen, z.B. nicht translatierte Bereiche und funktionelle Nucleinsäuren, wie sie dem Fachmann wohlbekannt sind.

Die erfindungsgemäße RNA kann, z. B., als in-vitro transkribierte RNA (IVT-RNA) zur Verfügung gestellt werden. Die zur Durchführung der in-vitro-Transkription notwendigen

Materialien sind dem Fachmann bekannt und im Handel erhältlich, insbesondere Puffer, Enzyme und Nucleotidmischungen. Die Art der zur Herstellung der erfindungsgemäßen RNA eingesetzten DNA ist ebenfalls nicht kritisch, in der Regel ist es klonierte DNA.

Wie oben ausgeführt, wird eine RNA, insbesondere mRNA zur Verfügung gestellt, die modifizierte Uridinnucleoside und modifizierte Cytidinnucleoside in einem vorbestimmten Anteil wie in Anspruch 1 angegeben aufweist. Der für eine spezifische mRNA optimale Anteil an modifizierten Uridinnucleosiden bzw. Cytidinnucleosiden kann mit Routineversuchen ermittelt werden, die dem Fachmann wohlbekannt sind.

Die erfindungsgemäße RNA wird bevorzugt zur Therapie von Erkrankungen oder zur Bereitstellung von für den Körper nützlichen Proteinen eingesetzt. Wenn die erfindungsgemäße RNA zur Therapie von Erkrankungen eingesetzt wird, weist sie bevorzugt das in-vitro-Transkript für ein Protein oder Proteinfragment auf, dessen Defekt oder Mangel zu einem krankhaften Zustand führt bzw. dessen Bereitstellung zur Linderung eines Leidens führt. Zur Herstellung der erfindungsgemäßen RNA wird bevorzugt eine DNA eingesetzt, die ein Protein oder Proteinfragment codiert, dessen Defekt oder Mangel zu einer Krankheit oder einem Leiden in Beziehung steht. In einer Ausführungsform wird zur Herstellung der erfindungsgemäßen RNA die DNA eines Gens eingesetzt, dessen Defekt oder Mangel zu einer Krankheit oder einem Leiden führt. In einer anderen Ausführungsform wird zur Herstellung der erfindungsgemäßen RNA eine DNA eingesetzt, die ein Protein codiert, dessen, gegebenenfalls zeitweises, Vorhandensein für einen Organismus nützlich oder heilsam ist. Als Krankheit oder Leiden wird dabei jeder Zustand angesehen, bei dem subjektiv und/oder objektiv körperliche und/oder geistig-seelische Störungen bzw. Veränderungen vorliegen, oder bei dem der regelwidrige Verlauf leiblicher, seelischer oder geistiger Lebensvorgänge Krankenpflege notwendig macht und ggf. Arbeitsunfähigkeit zur Folge haben kann.

Unter einem Protein oder Proteinfragment, dessen Vorhandensein ein Leiden lindern kann oder das für den Organismus nützlich oder fördernd sein kann, werden dabei solche Proteine oder Proteinfragmente verstanden, die, ohne dass ein Gendefekt vorliegt, dem Organismus vollständig oder zeitweise zur Verfügung gestellt werden sollen, da sie entweder aufgrund irgendwelcher Störungen oder aufgrund natürlicher Gegebenheiten fehlen oder weil sie dem Organismus unter bestimmten Umständen nützen können, z.B. bei der Behandlung von Defekten oder im Rahmen von Implantationen. Hierzu zählen auch veränderte Formen von Proteinen oder Proteinfragmenten, d.h. Formen von Proteinen, die sich im Verlauf des Stoffwechsels verändern, z.B. gereifte Formen eines Proteins etc. Bereitgestellt werden können auch Proteine, die für Wachstumsprozesse und Angiogenese eine Rolle spielen, die z.B. bei einer kontrollierten Regenerierung notwendig sind und dann gezielt durch Einführung der erfindungsgemäßen mRNA entstehen können. Dies kann z.B. nützlich sein bei Wachstumsprozessen oder zur Behandlung von Knochendefekten, Gewebedefekten und im Rahmen von Implantationen und Transplantationen.

Es wurde gefunden, dass die erfindungsgemäß modifizierte mRNA vorteilhaft verwendet werden kann, um das Einwachsen von implantierten Prothesen zu fördern. Die erfindungsgemäße mRNA kann, wenn sie auf der Oberfläche von einzusetzenden Prothesen, wie Zahnimplantaten, Hüftendoprothesen, Knieendoprothesen oder Wirbelfusionskörpern zur Verfügung steht, Faktoren freisetzen, die das Einwachsen, die Neubildung von Gefäßen und andere Funktionen, die für die neu eingesetzten Prothesen nützlich sind, fördern können. So ist es beispielsweise bekannt, biologisch wirksame Substanzen, wie Wachstumsfaktoren wie BMP-2 oder Angiogenesefaktoren im Rahmen einer Implantation von Prothesen oder danach zu verabreichen. Da biologische Substanzen sehr häufig äußerst kurze Halbwertszeiten haben, war es bisher notwendig, sehr hoch zu dosieren, was den Patienten mit starken Nebenwirkungen belastet. Erfindungsgemäß wird dieser Nachteil vermieden, da unter Verwendung der erfindungsgemäßen RNA die gewünschten und/oder benötigten Proteine gezielt und angemessen dosiert eingesetzt werden können. Dies vermindert oder erspart sogar dem Patienten die Nebenwirkungen. In dieser Ausführungsform kann die erfindungsgemäße RNA, die gewünschte und/oder benötigte Substanzen, wie Wachstumsfaktoren, Angiogenesefaktoren etc. codiert, in einer die RNA dosiert abgebenden Beschichtung auf das Implantat aufgebracht und daraus dann dosiert nach und nach abgegeben werden, so dass kontinuierlich oder intermittierend die Zellen in der Umgebung des Implantats die gewünschten Faktoren erzeugen und ggf. freisetzen können. Träger, in der Regel biokompatible, synthetische, natürliche oder gemischt natürlich-synthetische Polymere, deren Abgabeeigenschaften gezielt eingestellt werden können, sind wohlbekannt und brauchen daher hier nicht näher erläutert werden. Zum Einsatz kommen z.B. Polylactid- oder Polylactid/Glycolidpolymere. Auf diese Weise ist es möglich, die gewünschten Faktoren kontinuierlich, intermittierend, über längere oder kürzere Zeit und an gewünschter Stelle gezielt abzugeben.

Unter einem defizienten oder defekten Gen oder unter Defizienz oder Mangel werden im Rahmen der vorliegenden Erfindung solche Gene verstanden, die nicht, nicht korrekt oder nicht in ausreichendem Umfang exprimiert werden und dadurch Krankheiten oder Leiden verursachen, z.B. indem sie Stoffwechselstörungen verursachen.

Die erfindungsgemäße RNA kann geeigneterweise eingesetzt werden in jedem Fall, in dem ein Protein, das natürlicherweise im Organismus vorhanden wäre, aufgrund von Gendefekten oder Krankheiten jedoch nicht, in defizienter Form oder in zu geringer Menge vorliegt, dem Organismus zur Verfügung gestellt werden soll. Proteine bzw. die sie codierenden Gene, deren Defizienz oder Defekt mit einer Krankheit in Verbindung gebracht werden, sind bekannt. Im folgenden werden unterschiedliche Proteine und Gene aufgeführt, bei deren Mangel die erfindungsgemäße RNA eingesetzt werden kann.

## Tabelle 2

| Krankheiten, für die die Applikation von erfindungsgemäßer mRNA indiziert sein kann: | | |
|---|---|---|
| **Organ** | **Defekt** | |
| Lunge | Surfactant Protein B Defizienz | |
| Lunge | ABCA3 Defizienz | |
| Lunge | Zystische Fibrose | |
| Lunge | Alpha-1-Antitrypsin-Defizienz | |
| Plasmaproteine | Gerinnungsdefekte wie Hämophilie A u. B | |
| Plasmaproteine | Komplementdefekte wie Protein C-Defizienz | |
| Plasmaproteine | Thrombotische Thrombozytopenische Purpura (TPP, ADAMTS 13-Defizienz) | |
| Plasmaproteine | Angeborene Hämochromatosen (z.B. Hepcidin Mangel) | |
| | | |
| Schwere kombinierte Immundefekte (SCID) (T-, B- und NK-Zellen) | | |
| X-chromosomal vererbte kombinierte Immundefekte (X-SCID) | | |
| ADA-SCID (SCID infolge Mangels an Adenosindeaminase) | | |
| SCID mit RAG1-Mutation | | |
| SCID mit RAG2-Mutation | | |
| SCID mit JAK3-Mutation | | |
| SCID mit IL7R-Mutation | | |
| SCID mit CD45-Mutation | | |
| SCID mit CD3δ-Mutation | | |
| SCID mit CD3ε-Mutation | | |
| SCID mit Purinnukleosid-phosphorylase-Mangel (PNP-Mangel) | | |
| | | |
| Septische Granulomatosen (Graulozyten) | | |
| **Krankheit** | **Defekt bzw. Mutation** | |
| X-chromosomal-rezessive CGD | Mutation des gp91-phox-Gens | |
| CGD Cytochrom b positiv Typ1 | Mutation des p47-phox Gens | |
| CGD Cytochrom b positiv Typ 2 | Mutation des p67-phox Gens | |
| CGD Cytochrom b negativ | Mutation des p22-phox Gens | |
| | | |
| sonstige Speicherkrankeiten | | |
| Mutation im Glucocerebrosidase Gen | Morbus Gaucher | |
| Mutation im GALC-Gen | Morbus Krabbe | |
| lysosomal Speicherkrankheiten | Mukopolysaccharidosen | |

| Glykogenspeicherkrankheiten | | |
|---|---|---|
| **Typ** | **Defekt** | **Eigenname** |
| I (a-d) | Ia: Glucose-6-Phosphatase Ib,Ic,Id: Glucose-6-phosphat-Translokase | Morbus von Gierke |
| II | Lysosomale α-Glucosidase | Morbus Pompe |
| III | Glykogen-Debranching-Enzym | Morbus Cori |
| IV | 1,4-α-Glucan-verzweigendes Enzym | Morbus Andersen |
| V | Glykogenphosphorylase des Muskels | Morbus McArdle |

| | | | |
|---|---|---|---|
| VI | Glykogenphosphorylase/Phosphorylase-Kinase System (Leber und Muskel) | | Morbus Hers |
| VII | Phosphofruktokinase (Muskel) | | Morbus Tarui |
| VIII | Phosphorylase der Leber | | |
| IX (a-c) | Phosphorylase der Leber | | |
| X | cAMP-akt. Phosphorylase | | |
| XI | GLUT-2-Defekt | | Fanconi-Bickel-Syndrom |
| 0 | UDP-Glykogen-Synthase | | |
| sonstige Speicherkrankeiten | | | |
| | Mutation im Glucocerebrosidase Gen | | Morbus Gaucher |
| | Mutation im GALC-Gen | | Morbus Krabbe |
| | lysosomal Speicherkrankheiten | | Mukopolysaccharidosen |

Weitere auf defekten Genen beruhende Krankheiten sind im folgenden angegeben:

| Typ | Variante | | Klinische Merkmale | Defektes Enzym |
|---|---|---|---|---|
| I-H | Hurler-Pfaundler-Syndrom | | Dysmorphie (Gargoylismus), kognitive Retardierung, Skelettfehlbildung (Dysostose), Hornhauttrübung, Minderwuchs, Hernien, | $\alpha$-L-Iduronidase |
| I-S | Scheie-Krankheit | | geistig nicht eingeschränkt, Skelettfehibildung (Dysostose), Hornhauttrübung, Herzklappenfehler | $\alpha$-L-Iduronidase |
| I-H/S | Hurler/Scheie-Variante | | geistig zwischen I-H und I-S | $\alpha$-L-Iduronidase |
| II | Hunter-Syndrom | | mäßige kognitive Retardierung, Skelettfehibildung (Dysostose), erhebliche somatische Veränderungen, frühe Gehörlosigkeit | Iduronatsulfatsilfatase |
| III | Sanfilippo-Syndrom | Typ A | kognitive Retardierung, Dysmorphie, Hornhauttrübung kann fehlen, häufig Schwerhörigkeit, rasches Voranschreiten | Heparansulfatsulfamidase |
| | | Typ B | | $\alpha$-N-Acetylqlukoseamidase |
| | | Typ C | | Acetyl-CoA: $\alpha$-Glukosaminid-N-Acetyltransferase |
| | | Typ D | | N-Acetylglukosamin-6-sulfatsulfatase |
| IV | Morquio-Syndrom | Typ A | übliche kognitive Entwicklung, Skelettfehibildung (Dysostose) sehr ausgeprägt, Hornhauttrübunq fehlt | N-Acetylglukosamin-6-sulfatsulfatase |
| | | Typ B | milde Verlaufsform von Typ A | $\beta$-Galactosidase |
| V | jetzt: Typ I-S, s.o. | | | |
| VI | Maroteaux-Lasny-Syndrom | | übliche kognitive Entwicklung, schwere Skelettfehlblldung (Dysostose), Hornhauttrübung, Minderwuchs | N-Acetylgalaktosamin-4-sulfatsulfatase |
| VII | Sly-Syndrom | | mäßige Dysmorphie und Skelettfehlbildungen, Hornhauttrübung, übliche bis eingeschränkte Intelligenz | $\beta$-Glucuronidase |

Die obige Tabelle zeigt somit Beispiele für Gene, deren Defekt zu einer Krankheit führt, die durch Transkriptersatztherapie mit der erfindungsgemäßen RNA behandelt werden kann. Insbesondere können hier Erbkrankheiten genannt werden, die z.B. die Lunge betreffen, wie SPB-Defizienz, ABCA3-Defizienz, cystische Fibrose und α1-Antitrypsindefizienz; die Plasmaproteine betreffen und Gerinnungsdefekte und Komplementdefekte erzeugen; Immundefekte, wie z.B. SCID; septische Granulomatose und Speicherkrankheiten. Bei all diesen Krankheiten ist ein Protein, z.B. ein Enzym defekt, das durch Behandlung mit der erfindungsgemäßen RNA, die das von dem defekten Gen codierte Protein oder ein funktionelles Fragment davon zur Verfügung stellt, behandelt werden kann.

Beispiele für Proteine, die von der erfindungsgemäßen RNA codiert werden können, sind daher Erythropoietin (EPO), Wachstumshormon (Somatotropin, hGH), Cystic Fibrosis Transmembrane Conductance Regulator (CFTR), Wachstumsfaktoren wie GM-CSF, G-CSF, MPS, Protein C, Hepcidin, ABCA3 und Surfactant Protein B. Weitere Beispiele für Krankheiten, die mit der erfindungsgemäßen RNA behandelt werden können, sind Hämophilie A/B, Morbus Fabry, CGD, ADAMTS13, Morbus Hurler, über das X-Chromosom vermittelte A-γ-Globulinämie, mit Adenosindeaminase in Beziehung stehende Immundefizienz und das Respiratory Distress Syndrom bei Neugeborenen, das mit SP-B in Beziehung steht. Besonders bevorzugt beinhaltet die erfindungsgemäße mRNA die Sequenz für Surfactant Protein-B (SP-B) oder für Erythropoietin. Weitere Beispiele für Proteine, die von erfindungsgemäß modifizierter RNA codiert werden können, sind Wachstumsfaktoren, wie BMP-2 oder Angiogenesefaktoren.

Ein weiterer Einsatzbereich der erfindungsgemäßen RNA ergibt sich für solche Krankheiten oder Leiden, bei denen im Organismus Proteine nicht mehr oder nicht gebildet werden, z.B. aufgrund von Organversagen. Derzeit wird bei solchen Krankheiten ein rekombinantes Protein zur Substitution verabreicht. Erfindungsgemäß wird hierfür nun RNA bereitgestellt, so dass die Substitution des fehlenden Proteins auf der Ebene des Transkripts erfolgen kann. Dies hat mehrere Vorteile. Falls das Protein Glycosylierungen aufweist, so wird durch die Substitution auf Transkriptebene erreicht, dass die für den Menschen typische Glycosylierung im Organismus erfolgt. Bei rekombinantem, d.h. normalerweise in Mikroorganismen erzeugten Proteinen ist die Glycosylierung in der Regel anders als im zu substituierenden Organismus. Dies kann zu Nebenwirkungen führen. Generell ist davon auszugehen, dass das von der erfindungsgemäßen RNA exprimierte Protein mit dem körpereigenen in Bezug auf Struktur und Glycosylierung identisch ist, was bei rekombinanten Proteinen in der Regel nicht der Fall ist.

Beispiele für Proteine, deren Substitution oder Einsatz wünschenswert sein kann, sind funktionelle Proteine wie Erythropoietin, Wachstumsfaktoren, wie Somatotropin (hGH), G-CSF, GM-CSF und Thrombopoietin.

Ein weiteres Gebiet, auf dem die erfindungsgemäße RNA eingesetzt werden kann, ist das Gebiet der regenerativen Medizin. Durch Krankheitsprozesse oder durch Alterung kommt es zu degenerativen Erkrankungen, die durch Zuführung von aufgrund der Krankheits- bzw. Alterungsprozesse zu wenig oder nicht produzierten Proteinen behandelt und gelindert oder sogar geheilt werden können. Durch Zuführung der entsprechenden diese Proteine codierenden RNA kann der Degenerationsprozess aufgehalten oder sogar eine Regeneration eingeleitet werden. Beispiele hierfür sind Wachstumsfaktoren zur Geweberegeneration, die z.B. bei Wachstumsstörungen, bei degenerativen Erkrankungen, wie Osteoporose, Arthrose oder gestörter Wundheilung eingesetzt werden können. Die erfindungsgemäße RNA bietet hier nicht nur den Vorteil, dass das fehlende Protein gezielt und in richtiger Dosierung zur Verfügung gestellt werden kann, sondern es ist darüber hinaus möglich, das Protein in einem Zeitfenster bereitzustellen. So kann beispielsweise bei gestörter Wundheilung der entsprechende Heilfaktor oder Wachstumsfaktor für eine begrenzte Zeit durch dosierte Verabreichung der RNA bereitgestellt werden. Über später zu erläuternde Mechanismen kann darüber hinaus dafür gesorgt werden, dass die RNA gezielt an den Ort ihrer gewünschten Wirkung gebracht wird.

Beispiele für Faktoren, die mit der erfindungsgemäßen RNA exprimiert werden können, um regenerativ zu wirken, sind Fibroblast Growth Factor (FGF), z.B. FGF-1-23; Transforming Growth Factor (TGF), z.B. TGF-α, TGF-β, BMPs (knochenmorphogenetisches Protein), z.B. BMP1 bis 7, 8a, b, 10, 15; Platelet Derived Growth Factor (PDGF), z.B. PDGF-A, PDGF-B, PDGF-C und PDGF-D; epidermaler Wachstumsfaktor (EGF), Granulocyte-Macrophage Colony Stimulating Factor (GM-CSF); Vascular Endothelial Growth Factor (VEGF-A bis F und PIGF); insulinähnliche Wachstumsfaktoren, z.B. IgF1 und IgF2; Hepatocyte Growth Factor (HGF); Interleukine, z.B. Interleukin-1B, IL-8, IL-1 bis 31; Nervenwachstumsfaktor (NGF) und weitere Faktoren, die die Bildung von Erythrozyten, Neutrophilen, Gefäßen usw. stimulieren.

Auch auf dem Gebiet der Krebserkrankungen kann die erfindungsgemäße RNA gezielt eingesetzt werden. Durch die Expression maßgeschneiderter T-Zellrezeptoren in T-Lymphozyten, die spezifische tumorassoziierte Tumorantigene erkennen, können diese noch effektiver werden. Es wurde bereits gezeigt, dass prinzipiell mRNA auf diesem Gebiet erfolgreich verwendet werden kann. Ihr Einsatz wurde jedoch bisher durch die bereits oben beschriebenen immunogenen Effekte verhindert. Mit der erfindungsgemäß bereitgestellten wenig immunogenen und hochstabilen RNA ist es nun möglich, entsprechend T-Zellrezeptoren zu exprimieren.

Erfindungsgemäße RNA kann auch dazu verwendet werden, Transkriptionsfaktoren zu exprimieren, die dafür sorgen, dass somatische Zellen in embryonale Stammzellen reprogrammiert werden. Beispiele hierfür sind O-cp3/4, Sox2, KLF4 und c-MYC. Erfindungsgemäße stabile RNA, insbesondere mRNA, die diese Transkriptionsfaktoren codiert, kann daher zur Erzeugung von Stammzellen führen, ohne die Nebenwirkungen zu erzeugen, die bei dem bisher in Betracht gezogenen Gentransfer über virale oder nicht virale Vektoren eintreten können.

Ein Vorteil bei Anwendung der erfindungsgemäßen RNA ist es, dass im Gegensatz zur Verwendung von DNA-Vektoren die Dauer der Behandlung einstellbar ist. Im Fall der Induktion von Stammzellen ist es in der Regel wünschenswert, dass die Transkriptionsfaktoren nur vorübergehend aktiv sind, um somatische Zellen in Stammzellen umzuprogrammieren. Durch dosierte Gabe der entsprechenden, die Transkriptionsfaktoren codierenden RNA ist die Aktivität zeitlich steuerbar. Im Gegensatz dazu ist bei den bisher bekannten Verfahren die Gefahr der Integration der applizierten Gene vorhanden, die zu Komplikationen, z.B. Tumorigenese, führt und außerdem verhindert, dass die Zeitdauer gesteuert werden kann.

Auch im Bereich der Impfstoffe bietet die erfindungsgemäße RNA neue Möglichkeiten. Die klassische Impfstoffentwicklung beruht auf getöteten oder abgeschwächten Erregern. In jüngerer Zeit wurde auch DNA in Betracht gezogen, die ein Protein des Erregers codiert. Die Herstellung dieser Impfstoffe ist aufwändig und dauert sehr lange. Häufig treten Nebenwirkungen auf, die dazu führen, dass auf Impfungen verzichtet wird. Mit der erfindungsgemäßen mRNA ist es möglich, einen Impfstoff bereitzustellen, der die mit Erregern oder DNA verbundenen Probleme nicht aufweist. Außerdem kann ein derartiger Impfstoff sehr schnell bereitgestellt werden, sobald die Antigensequenzen eines Erregers vorliegen. Dies ist besonders vorteilhaft bei drohenden Pandemien. In einer Ausführungsform der vorliegenden Erfindung wird daher eine RNA bereitgestellt, die einen antigenen Teil eines Krankheitserregers codiert, z.B. ein Oberflächenantigen. Es ist auch möglich, eine mRNA bereitzustellen, die eine Aminosäuresequenz codiert, die eine Kombination aus mehreren Epitopen, gegebenenfalls verbunden durch Spacerabschnitte, aufweist. Eine Kombination mit immunmodulierenden Substanzen ist auch möglich, entweder indem die RNA ein Fusionsprotein codiert oder als Kombination von Nucleinsäuren.

Weiterhin kann die erfindungsgemäße RNA auch solche Proteine codieren, die als Faktoren, Stimulatoren, Induktoren etc. Einfluss auf den Krankheitsverlauf haben. Beispiele sind Erkrankungen, die nicht unmittelbar auf einen Gendefekt zurückzuführen sind, aber bei denen mit Hilfe der mRNA-Expression das Krankheitsgeschehen positiv beeinflusst werden kann. Beispiele sind: Erythropoietin zur Stimulation der Bildung von Erythrozyten, G-CSF oder GM-CSF zur Bildung von Neutrophilen, Wachstumsfaktoren zur Bildung neuer Gefäße, zur Knochen- und Wundheilung als Faktoren für das "Tissue Engineering", Behandlung von Tumoren durch Induktion der Apoptose oder durch Bildung von proteinösen Zellgiften z.B. Diphterietoxin A, durch Induktion von pluripotenten Stammzellen (iPS) etc.

Es wurde festgestellt, dass nur ein erfindungsgemäßes Polyribonucleotid, das einen vorbestimmten Anteil an modifizierten und nichtmodifizierten Nucleotiden aufweist, eine geringe Immunogenität bei gleichzeitig hoher Stabilität aufweist. Um für ein bestimmtes Polyribonucleotid die optimale Kombination an modifizierten und unmodifizierten Nucleotiden bestimmen zu können, können Immunogenität und Stabilität in an sich bekannter Weise bestimmt werden. Zur Bestimmung der Immunogenität einer RNA können verschiedene dem Fachmann wohlbekannte Verfahren verwendet werden. Ein gut geeignetes Verfahren ist die Bestimmung von Entzündungsmarkern in Zellen als Reaktion auf die Verabreichung von RNA. Ein derartiges Verfahren ist in den Beispielen beschrieben. Gemessen werden üblicherweise Cytokine, die mit einer Entzündung in Verbindung stehen, wie z.B. TNF-$\alpha$, IFN-$\alpha$, IFN-$\beta$, IL-8, IL-6, IL-12, oder weitere dem Fachmann bekannte Cytokine. Auch die Expression von DC-Aktivierungsmarkern kann zur Abschätzung der Immunogenität verwendet werden. Ein weiterer Hinweis auf eine immunologische Reaktion ist der Nachweis der Bindung an die Toll-artigen Rezeptoren TLR-3, TLR-7 und TLR-8 sowie an Helicase RIG-1.

Die Immunogenität wird in der Regel in Relation zu einer Kontrolle bestimmt. In einem üblichen Verfahren wird Zellen entweder die erfindungsgemäße RNA oder eine unmodifizierte oder in anderer Weise modifizierte RNA verabreicht und die Sekretion von Entzündungsmarkern in einem bestimmten Zeitabstand als Reaktion auf die Verabreichung der RNA gemessen. Als Standard, mit dem verglichen wird, kann entweder unmodifizierte RNA dienen, wobei dann die Immunantwort geringer sein sollte, oder RNA, von der bekannt ist, dass sie keine oder eine geringe Immunantwort hervorruft, wobei dann die Immunantwort der erfindungsgemäßen RNA im selben Bereich liegen sollte und nicht erhöht sein sollte. Mit der erfindungsgemäßen RNA ist es möglich, die Immunantwort gegenüber unmodifizierter RNA um mindestens 30%, in der Regel mindestens 50% oder sogar 75% zu senken oder sogar ganz zu verhindern.

Die Immunogenität kann bestimmt werden über die Messung der oben genannten Faktoren, insbesondere über die Messung der TNF-$\alpha$- und IL-8-Pegel sowie die Bindefähigkeit an TLR-3-, TLR-7-, TLR-8- und Helicase-RIG1. Um somit festzustellen, ob eine mRNA die gewünschte geringe Immunogenität aufweist, kann die Menge an einem oder mehreren der oben genannten Faktoren nach Verabreichung des jeweiligen Polyribonucleotids gemessen werden. So kann z.B. Mäusen über die Schwanzvene oder i.p. eine Menge an zu untersuchender mRNA verabreicht werden und dann eine oder mehrere der oben genannten Faktoren im Blut nach einem vorbestimmten Zeitraum, z.B. nach 7 oder 14 Tagen bestimmt werden. Die Menge an Faktor wird dann in Beziehung gesetzt zu der Menge an Faktor, die im Blut von unbehandelten Tieren vorliegt. Als sehr wertvoll für die Bestimmung der Immunogenität hat sich erwiesen, die Bindefähigkeit an TLR-3, TLR-7, TLR-8 und/oder Helicase RIG-1 zu bestimmen. Sehr gute Hinweise liefern auch die TNF-$\alpha$-Pegel und IL-8-Pegel. Mit der erfindungsgemäßen mRNA ist es möglich, die Bindefähigkeit an TLR-3, TLR-7, TLR-8 und RIG-1 um mindestens 50% im Vergleich zu unmodifizierter RNA zu senken. In der Regel gelingt es, die Bindung an die genannten Faktoren um mindestens 75% oder sogar um 80% zu senken. In bevorzugten Ausführungsformen liegt die Bindefähigkeit an TLR-3, TLR-7, TLR-8 und RIG-1 im selben Bereich für die erfindungsgemäße mRNA und für Tiere, denen keine mRNA verabreicht wurde. Mit anderen Worten ruft die erfindungsgemäße mRNA praktisch keine entzündlichen bzw. immunologischen Reaktionen hervor.

In jedem Fall weist die erfindungsgemäße RNA eine so geringe Immunogenität auf, dass das Allgemeinbefinden des Patienten nicht beeinflusst wird. Ein geringer Anstieg der oben genannten Faktoren kann daher so lange toleriert, so lange das Allgemeinbefinden sich dadurch nicht verschlechtert. Weitere Eigenschaften der erfindungsgemäßen mRNA sind ihre Effizienz und Stabilität. Hierfür sind Transkriptionseffizienz, Transfektionseffizienz, Translationseffizienz und Dauer der Proteinexpression wichtig und können mit an sich bekannten Verfahren bestimmt werden.

Die Transkriptionseffizienz gibt an, wie effizient RNA aus DNA erzeugt werden kann. Hier kann es bei Verwendung eines hohen Anteils an modifizierten Nucleotiden zu Problemen kommen. Die erfindungsgemäß modifizierte RNA kann mit hoher Transkriptionseffizienz hergestellt werden.

Um eine stabile und ausreichende Expression der von der RNA codierten Proteine zu erhalten, ist es wichtig, dass ausreichend RNA die gewünschten Zellen erreicht. Dies kann bestimmt werden, indem nach Verabreichung von markierter RNA der Anteil an RNA, der die Zellen erreicht hat, durch Messung der Markierung bestimmt wird. Zur Bestimmung der Markierung kann Durchflusscytometrie verwendet werden. Wenn mit einem fluoreszierenden Molekül markiert wurde, so kann die Transfektionseffizienz z.B. berechnet werden als Prozentanteil der Zellpopulation, bei dem die Fluoreszenzintensität gegenüber Kontrollzellen, die nur mit PBS behandelt wurden, höher ist. Es wurde gefunden, dass die erfindungsgemäß modifizierte RNA gut herzustellen ist, im Gegensatz zu RNA, bei der zwei oder mehr Nucleotidarten zu 100% durch modifizierte Nucleotide ersetzt sind, und dass die Transfektionseffizienz für erfindungsgemäße RNA, bei der nur ein Teil der Nucleotide modifiziert ist, weit höher ist als bei RNA, bei der eine Art von Nucleotiden zu jeweils 100% modifiziert ist.

Die Translationseffizienz bezeichnet die Effizienz, mit der die RNA in das Protein translatiert wird. Je höher die Translationseffizienz ist, desto geringer kann die Dosis an RNA sein, die dann zur Behandlung eingesetzt werden muss. Die Translationseffizienz kann bestimmt werden, indem der Anteil an Translation für erfindungsgemäß modifizierte RNA verglichen wird mit der Translationsquote von unmodifizierter RNA. Die Translationseffizienz ist in der Regel bei der erfindungsgemäßen RNA etwas geringer als bei unmodifizierter RNA. Dies wird allerdings durch die weitaus höhere Stabilität, die sich in der Dauer der Proteinexpression ausdrückt, mehr als ausgeglichen.

Die erfindungsgemäße RNA sorgt insbesondere für eine hohe Stabilität, die zu lang andauernder Proteinexpression führt. Insbesondere wenn die erfindungsgemäß modifizierte RNA zur Behandlung von auf Gendefekten beruhenden Krankheiten vorgesehen ist, ist sie umso wertvoller, je länger sie in der Zelle erhalten bleibt. Je schneller die RNA abgebaut wird, desto schneller endet die Proteinexpression und desto häufiger muss die RNA verabreicht werden. Umgekehrt kann die Dosierungsfrequenz bei einer stabilen RNA, die lange in der Zelle bleibt, stark vermindert werden. Es wurde gefunden, dass erfindungsgemäß modifizierte RNA bis zu 4 Wochen stabil exprimiert wird.

Für andere Ausführungsformen, d.h. wenn RNA nur für eine zeitweise Expression vorgesehen ist, kann die Dauer der Proteinexpression eingestellt werden durch Beeinflussung der Stabilität.

Eine weitere wertvolle Eigenschaft der erfindungsgemäßen RNA ist es daher, dass die Wirkungsdauer gezielt über die Stabilität eingestellt werden kann, so dass die Dauer der Proteinexpression so zugeschnitten werden kann, dass sie in einem gewünschten Zeitfenster stattfindet. Andererseits kann eine sehr lang wirksame RNA da eingesetzt werden, wo dies erforderlich ist. Die erfindungsgemäß modifizierte RNA, deren Expression bis zu 4 Wochen andauern kann, ist daher ideal geeignet zur Behandlung chronischer Erkrankungen, da hier nur alle 4 Wochen dosiert werden muss. Auch für Ausführungsformen, bei denen die RNA Faktoren codiert, die über längere Zeit dem Organismus zugeführt werden sollen, um Krankheiten zu lindern oder zu verhindern, ist die hohe Stabilität und lang andauernde Proteinexpression vorteilhaft, z.B. für die Verwendung von Erythropoietin codierender RNA. Besonders vorteilhaft kann die erfindungsgemäße RNA auch für die Behandlung von Hämophilie eingesetzt werden. Hier ist es bisher notwendig, den fehlenden Faktor wöchentlich zu verabreichen. Mit Bereitstellung der erfindungsgemäßen RNA kann die Frequenz der Verabreichung gesenkt werden, so dass den Faktor codierende RNA nur noch alle 2 oder sogar alle 4 Wochen verabreicht werden muss.

Die Stabilität der erfindungsgemäßen mRNA kann mit an sich bekannten Verfahren bestimmt werden. Geeignet sind insbesondere Verfahren zur Bestimmung der Lebensfähigkeit von Zellen, die erfindungsgemäß modifizierte RNA enthalten im Vergleich zu Zellen, die unmodifizierte oder vollmodifizierte RNA enthalten, z.B. im Vergleich zu unmodifizierter oder in bekannter Weise modifizierter RNA. Es kann auch die Produktion des codierten Proteins über die Zeit verfolgt werden. Unter Stabilität einer RNA wird hier verstanden, dass die RNA, wenn sie in die Zelle gebracht worden ist, das gewünschte Protein exprimieren kann bzw. in das Protein oder ein funktionelles Fragment davon translatierbar ist, über längere Zeit expressionsfähig bleibt, nicht gleich abgebaut wird und nicht inaktiviert wird.

Ein Verfahren, um die Stabilität und die Überlebensdauer von RNA in einer Zelle zu testen, besteht daher darin, zu bestimmen, wie lange ein von der RNA codiertes Protein in der Zelle nachweisbar ist oder seine Funktion ausübt. Methoden hierzu sind in den Beispielen beschrieben. So kann beispielsweise eine mRNA mit einer ein Reportermolekül codierenden Sequenz in die Zelle eingebracht werden, ggf. zusammen mit einer ein gewünschtes Protein codierenden RNA und nach vorbestimmten Zeiträumen dann die Gegenwart von Reportermolekül und ggf. Protein bestimmt werden. Geeignete Reportermoleküle sind im Stand der Technik wohlbekannt und die üblicherweise verwendeten können auch hier eingesetzt werden. In einer bevorzugten Ausführungsform wird RFP, rot fluoreszierendes Protein, als Reportermolekül verwendet.

Wie oben ausgeführt kann die erfindungsgemäße RNA zur Therapie verwendet werden, so dass in der Zelle, in die die RNA gebracht wird, ein Protein gebildet werden kann, das natürlicherweise nicht oder nicht in gewünschtem Ausmaß exprimiert wird. Die erfindungsgemäße RNA kann dabei sowohl dann eingesetzt werden, wenn das Protein aufgrund einer Defizienz eines Gens nicht gebildet wird, als auch in den Fällen, in denen ein Protein aufgrund einer Erkrankung nicht gebildet wird oder in solchen Fällen, in denen die Zuführung des Proteins für den Organismus vorteilhaft ist. Die RNA kann auch dazu verwendet werden, ein in nicht ausreichendem Umfang exprimiertes Protein zu ergänzen. Die jeweils angewendete Dosis richtet sich nach der Funktion, die die RNA erfüllen soll. Wie oben ausgeführt, kann die Wirkungsdauer der erfindungsgemäßen RNA gezielt eingestellt werden. Die Dauer der Behandlung richtet sich nach der jeweiligen Indikation. Wenn die RNA zur chronischen Therapie einer auf einem defizienten Gen beruhenden Krankheit verwendet wird, wird die Wirkungsdauer so lange wie möglich sein, während sie bei anderen Indikationen gezielt auf ein Zeitfenster eingestellt werden kann.

Gemäß einer besonders bevorzugten Ausführungsform wird als RNA eine IVT-mRNA verwendet, die das Surfactant-Protein-B codiert. Wenn dieses Protein bei Säugetieren defizient ist, kommt es zur Ausbildung des Atemnotsyndroms der Früh- und Neugeborenen, auch als Respiratory-Distress-Syndrom bezeichnet. Dieses Syndrom führt aufgrund einer Lungenkrankheit bei Neugeborenen häufig zum Tod. Die Verwendung einer mehrfach modifizierten in vitro transkribierten SP-B codierenden mRNA, bei der 5 bis 50% der Uridinnucleoside und 5 bis 50% der Cytidinnucleoside wie in Anspruch 1 angegeben modifiziert sind, führt dazu, dass das Protein gebildet wird und die Krankheit gemildert oder geheilt wird.

Gemäß einer weiteren bevorzugten Ausführungsform wird als RNA eine IVT-mRNA verwendet, die Erythropoietin codiert. Erythropoietin ist ein für den Organismus sehr wichtiges Protein, das z.B. bei Nierenerkrankungen nicht mehr in ausreichender Menge zur Verfügung steht und deshalb zugeführt werden muss. Derzeit wird hierzu rekombinantes Erythropoietin verwendet, das in Mikroorganismen oder tierischen Zellen erzeugt wurde und daher eine nicht natürlicherweise vorkommende Glycosylierung aufweist. Bei Verwendung des rekominanten EPOs kam es in seltenen Fällen zu schwerwiegenden Nebenwirkungen, z.B. einer Erythrozytenaplasie.

Die erfindungsgemäß bereitgestellte IVT-mRNA enthält eine Ribonucleinsäure, die Erythropoietin codiert, wobei 5 bis 50% der Uridinnucleotide und 5 bis 50% der Cytidinnucleotide wie in Anspruch 1 angegeben modifiziert sind. In einer besonders bevorzugten Ausführungsform wird eine EPO codierende mRNA bereitgestellt, bei der 15 bis 25% der Uridinnucleotide und 15 bis 25% der Cytidinnucleotide wie in Anspruch 1 angegeben modifiziert sind. Es wurde gefunden, dass diese mRNA eine gegenüber unmodifizierter RNA stark verminderte immunogenität aufweist. Gleichzeitig zeigt sie eine Transfektionseffizienz von über 90% und eine solche Stabilität, dass der Hämatokritwert nach 14 Tagen immer noch erhöht ist. Da das von der erfindungsgemäßen RNA im Organismus gebildete EPO die richtige Glykosylierung aufweist, sind Nebenwirkungen nicht zu befürchten. Durch gezielte intermittierende Verabreichung der erfindungsgemäß modifizierten EPO codierenden RNA konnte der Hämatokritwert über längere Zeit auf dem gewünschten Pegel gehalten werden.

Erfindungsgemäß wird eine nicht immunogene stabile RNA bereitgestellt, die in vivo in Säugetieren anwendbar ist und die das notwendige Protein in einer Form bereitstellt, die dem natürlicherweise vorhandenen körpereigenen Protein sehr ähnlich ist, wenn nicht identisch ist und insbesondere die körpereigene Glycosylierung aufweist.

Die erfindungsgemäße mRNA kann direkt so, wie sie ist, eingesetzt werden. Es besteht aber auch die Möglichkeit, die mRNA weiterhin zu modifizieren, um zusätzliche nützliche Eigenschaften einzuführen. Die mRNA kann einerseits modifiziert werden, indem an den codierenden Strang weitere codierende oder nicht codierende Sequenzen angefügt werden. Sie kann andererseits auch modifiziert werden, indem an in den modifizierten Nucleotiden vorgesehene funktionelle Gruppen weitere Moleküle gebunden werden.

Die erfindungsgemäße mRNA kann mit zielführenden Liganden kombiniert werden, die an für die Zielzellen spezifische Oberflächenrezeptoren binden, so dass eine Rezeptor-vermittelte Transfektion der Zielzelle möglich ist. Hierzu können einerseits Vehikel, die für das Einbringen von mRNA in Zellen geeignet sind, oder aber die mRNA selbst mit einem Liganden modifiziert werden. Beispiele für geeignete Vehikel für das Einbringen von mRNA in Zellen sind kationische Agenzien. Hierzu zählen kationische Lipide, kationische Polymere oder auch Nanopartikel, Nanokapseln, magnetische Nanopartikel und Nanoemulsionen. Geeignete Vehikel sind dem Fachmann bekannt und in der Fachliteratur beschrieben. Auch geeignete Liganden sind dem Fachmann wohlbekannt und in der Literatur beschrieben und erhältlich. Als Liganden können z.B. verwendet werden: Transferrin, Lactoferrin, Clenbuterol, Zucker, Uronsäuren, Antikörper, Aptamere etc.

Die mRNA kann aber auch selbst mit einem Liganden modifiziert werden. Hierzu sind bevorzugt mRNAs mit modifizierten Nukleosiden geeignet, die in der 2'Position der Ribose eine primäre Aminogruppe oder eine Azidogruppe tragen. Beispiele sind in der Tabelle oben zu finden. Derartige Modifikationen sind besonders bevorzugt, da sie zur biologischen Aktivität beitragen. Über diese Modifikationen lässt sich durch Amidbildung oder "Klick"-Chemie der Ligand einfach einbauen, z.B. über Bioconjugate Techniques.

Möglich ist auch, eine RNA-Sequenz, die an Proteine, z.B. Rezeptoren, binden kann (Aptamere), am 5' Ende der mRNA einzufügen. Dieses

Vorgehen hat den Vorteil, dass der Ligand bereits auf DNA-Ebene direkt in die Matrize eingefügt werden und kloniert werden kann und durch die IVT in die mRNA eingefügt wird. Es ist daher keine anschließende Modifikation der mRNA mit dem Liganden mehr notwendig.

Es wird auch beschrieben, die mRNA durch zusätzliche Modifikationen mit inerten Polymeren z.B. Polyethylenglycol (PEG) zu modifizieren. Verfahren hierzu sind dem Fachmann wohlbekannt, es können Verfahren, wie sie für Liganden bekannt sind, angewendet werden. So kann beispielsweise bei einem kleinen Teil der für die erfindungsgemäße mRNA verwendeten modifizierten Nucleotiden eine Bindungsstelle für Polyethylenglycol vorgesehen werden, an der das PEG nach Transkription gebunden wird. Das Polyethylenglycol dient zur extrazellulären Stabilisierung der mRNA, d.h. es schützt das Polyribonucleotidmolekül, solange bis es in der Zelle angekommen ist. Beim Eintritt in die Zelle wird das PEG abgespalten. Die Bindung zwischen PEG und RNA wird daher bevorzugt so ausgebildet, dass die Abspaltung bei Eintritt in die Zelle erleichtert wird. Hierzu kann z.B. eine funktionelle Gruppe vorgesehen werden, die pH-abhängig gespalten wird. Auch andere die RNA stabilisierende Moleküle können über entsprechende aktive Stellen an den modifizierten Nucleotiden vorgesehen werden. Auf diese Weise kann die mRNA durch sterische Stabilisierung vor enzymatischem Abbau geschützt werden und eine Interaktion mit Bestandteilen von Biofluiden verhindert werden. Die so modifizierte mRNA kann als "Stealth"-mRNA bezeichnet werden.

Ein bevorzugtes Verfahren zum Schutz und zur Stabilisierung von RNA wird in EP 11 98 489 beschrieben.

Erfindungsgemäße RNA wird bevorzugt mit den in EP 11 98 489 beschriebenen Methoden geschützt. Es wurde gefunden, dass einerseits die erfindungsgemäß modifizierte RNA auch vorteilhaft mit diesem Verfahren stabilisiert und geschützt werden kann, und andererseits, dass die Aktivität von entsprechend behandelter erfindungsgemäßer RNA nicht oder nicht wesentlich eingeschränkt wird.

Bevorzugt wird daher erfindungsgemäß modifizierte RNA gemäß EP 11 98 489 behandelt.

Ein Beispiel für die zellspezifische Regulation ist der Einbau von Mikro-RNA-Bindungsstellen für Mikro-RNA 142-3p, die in hämatopoetischen Zellen exprimiert wird, nicht aber in Zellen anderen Ursprungs. Dadurch wird die Expression so gesteuert, dass die mRNA-Translation in hämatopoetischen Zellen stark verringert ist gegenüber anderen Zellen. Entsprechend kann die Expression in anderen Zellarten gezielt gesteuert werden durch Einbau der jeweils geeigneten Mikro-RNA-Bindungsstellen, die dem Fachmann bekannt sind.

Es ist ebenfalls möglich, die erfindungsgemäße mRNA mit einem Target bzw. einer Bindungsstelle für mindestens eine Mikro-RNA, die nur in gesunden Zellen, nicht aber den von der Krankheit betroffenen Zellen vorhanden ist, zu kombinieren.

Auf diese Weise wird erreicht, dass das von der mRNA codierte Protein nur in den Zellen erzeugt wird, die das Protein benötigen. Die Auswahl der geeigneten Targets erfolgt mit Routineverfahren, die dem Fachmann wohlbekannt sind. Ein gängiges Verfahren, dass auf DNA Ebene durchgeführt wird, ist die Klonierung einer microRNA Bindungsstelle in die 3'UTR (Gu et al, Nat Struct Mol Biol. 2009 Feb;16(2):144-50., Brown et al, Nat Biotechnol. 2007 Dec;25(12):1457-67., Brown et al, Nat Med. 2006 May;12(5):585-91., WO 2007000668). Vorteilhaflerweise wird eine mit einer Bindungsstelle für microRNA versehene RNA dann verwendet, wenn die RNA ein Zellgift codiert. In diesem Fall ist es besonders wünschenswert, das für Zellen giftige Protein nur dorthin zu tragen, wo es seine Wirkung entfalten soll. Für diese Ausführungsform kann es auch von Vorteil sein, die Wirkungsdauer der RNA einzustellen, indem gezielt die RNA so modifiziert wird, dass ihre Stabilität in einem vorbestimmten Zeitfenster liegt.

Weiterhin kann die erfindungsgemäße RNA mit microRNAs oder shRNAs downstream des 3'PolyA-Schwanzes kombiniert werden. Dies hat den Vorteil, dass der mRNA-microRNA/shRNA-Hybrid intrazellulär von Dicer gespalten werden kann und damit zwei Wirkmoleküle, die in unterschiedlichen Kaskaden der Krankheitsentstehung eingreifen, freigesetzt werden können. Ein derartiges Hybrid kann bereitgestellt werden zur Behandlung von Krankheiten wie Krebs oder Asthma. Die erfindungsgemäße RNA ist folglich geeignet, gleichzeitig eine defizienten mRNA zu komplementieren und in eine defekte microRNA Kaskade einzugreifen.

Erfindungsgemäß wird somit eine RNA mit vorteilhaften Eigenschaften zur Verfügung gestellt, die mit einem Screeningverfahren getestet werden kann, bei dem eine für ein Reporterprotein, z.B. das rot fluoreszierende Protein (RFP), codierende Sequenz verwendet wird. Wenn die Toxizität und Stabilität von Sequenzen eines Reportergens mit unmodifizierten, einfach oder mehrfach modifizierten Nukleotiden mit unterschiedlichen Modifikationen auf ihre Immunogenität und Transfektionseffizienz untersucht werden, zeigt sich, dass nur die erfindungsgemäße, d.h. mehrfach modifizierte mRNA, bei der jeweils mindestens 5% der Uridinnucleoside bzw. Cytidinnucleoside durch modifizierte Nucleoside ersetzt sind, zu einer stark verminderten Immunogenität gegenüber menschlichen primären Monozyten im Blut führt, und gleichzeitig hohe Transfektionsraten von mehr als 80% liefern kann. Dies kann beispielsweise in Alveolarepithelzellen Typ II beim Menschen oder in der Maus getestet werden. Außerdem ist die Dauer der RNA-Expression für erfindungsgemäß modifizierte RNAs signifikant länger als bei bekannter RNA. Es wurde gefunden, dass hauptsächlich aufgrund der höheren Stabilität und geringeren Immunogenität der erfindungsgemäß mehrfach modifizierten mRNA die Expression länger andauert, als bei bekannten Präparaten. In quantitativer Auswertung zeigte ein erfindungsgemäß modifiziertes Derivat 10 Tage nach der Transfektion eine 10-fach höhere Menge an Expressionsprodukt als nicht oder nur einfach modifizierte RNA.

Beschrieben wird auch ein Verfahren zum Screenen von Nucleotidsequenzen, um die Immunogenität und Expressionsqualität zu testen, bei dem die mRNA-Sequenz mit mindestens einem Rezeptor ausgewählt aus TLR3, TLR3, TLR8 und Helicase-RIG-1 in Verbindung gebracht wird und die Bindefähigkeit im Vergleich zu einer Kontrollsequenz gemessen wird. Als Kontrollsequenz wird eine Sequenz verwendet, deren Bindefähigkeit bekannt ist. Je schwächer die Bindung an mindestens einen dieser Rezeptoren ist, desto vielversprechender ist die Sequenz.

Die Eigenschaften von erfindungsgemäßer mRNA, insbesondere IVT-mRNA können mit einem Screeningverfahren an einer ein Reporterprotein exprimierenden RNA getestet werden. Als Reporterprotein wird das rot fluoreszierende Protein (RFP) bevorzugt. Dieses Protein codierende Sequenzen, die Nukleotide mit unterschiedlichen Modifikationen aufweisen, können auf ihre Immunogenität und Transfektionseffizienz untersucht werden. So können für Tests verschiedene Modifikationen von mRNA verwendet werden, z.B. Uridinnucleoside teilweise durch 2-Thlouridinnucleoside (im folgenden auch als s2U bezeichnet) ersetzt und Cytidinnucleoside teilweise durch 5-Methylcytidinnucleoside (im folgenden auch als m5C bezeichnet) ersetzt werden.

Die Figuren 1A, 1B, 1C und 2A und 2B zeigen die Ergebnisse, die bei Durchführung eines solchen Screeningverfahrens erhalten werden. Nähere Einzelheiten sind in den Beispielen zu finden. Die in den Figuren dargestellten Ergebnisse beruhen auf Versuchen, die für RFP-RNA durchgeführt wurden und zeigen, dass nur mehrfach modifizierte mRNA, bei der jeweils mindestens 5% der Uridinnucleoside und jeweils mindestens 5% der Cytidinnucleoside modifiziert sind, zu einer stark verminderten Immunogenität gegenüber menschlichen primären Monozyten im Blut, sowohl ex vivo als auch in vivo führen, und gleichzeitig hohe Transfektionsraten von mehr als 80% sowohl in Alveolarepithelzellen Typ II beim Menschen als auch bei der Maus liefern kann. Außerdem ist die Dauer der Expression für erfindungsgemäß modifizierte mRNAs signifikant länger als für nicht modifizierte mRNA.

Ferner beschrieben wird ein Verfahren, um zu

testen, ob eine in betracht gezogene RNA zur Therapie geeignet ist, unter Verwendung eines mRNA-Immunpräzipitations-(RIP)-Tests. Ein geeigneter RIP-Test wird in den Beispielen näher beschrieben. Studien haben gezeigt, dass Zellen des Immunsystems durch unmodifizierte Reporter-mRNA über eine RNA-Bindung an Tolllike-Rezeptor (TLR) 3, TLR7, TLR8 und Helicase-RIG-1 aktiviert werden. Wenn die Ergebnisse zeigen, dass die Bindung einer getesteten mRNA an TLR3, TLR7, TLR8 und/oder RIG-1 stark vermindert ist im Vergleich zu unmodifizierter mRNA, ist dies ein Hinweis auf verminderte Immunogenität. Es konnte gezeigt werden, dass in dieser Hinsicht erfindungsgemäß verwendete Mehrfachmodifikationen signifikant wirksamer als einzelne s2U-Modifikationen sind. In den Beispielen wurde der Einfluss von RNA auf die Pegel an IFN-$\gamma$, IL-12 und IFN-$\alpha$ untersucht, nachdem die RNA Mäusen intravenös injiziert worden war. Es zeigte sich dass mehrfach modifiziertes s2U$_{(0,25)}$m5C$_{(0,25)}$RFPmRNA eine Immunantwort verhinderte. Zusammen zeigen die in den Beispielen erhaltenen Ergebnisse, dass mehrfach modifizierte mRNA signifikant die TLR- und RIG-1-Bindung vermindert und damit die Immunantwort senkt bei gleichzeitig erhöhter und verlängerter Expression. Eine mehrfach modifizierte RNA, insbesondere IVT-mRNA ist daher ein geeigneter Kandidat für die in-vivo-Behandlung einer auf einem defizienten Gen beruhenden Krankheit. Ein besonders vielversprechender Kandidat wird im Folgenden kurz erläutert und in den Beispielen näher beschrieben.

Um zu testen, ob es möglich ist, erfindungsgemäß modifizierte RNA zur Behandlung in der Lunge einzusetzen, wurde mehrfach modifizierte mRNA, die für ein Fusionsprotein aus Enhanced Green Fluorescent Protein und Luciferase (EGFPLuc) codierte, direkt in die Lunge einer Maus eingeführt und getestet, ob Luciferase exprimiert wurde im Vergleich zu unmodifizierter EGFPLuc-RNA. Die Luciferaseexpression erreichte nach drei Stunden in der Lunge ein Maximum, obwohl der gesamte Lumineszenzflux nach 24 Stunden schnell abnahm auf sehr geringe Anteile 5 Tage nach der Behandlung. Im Gegensatz dazu wurden hohe Expressionswerte beobachtet bei Mäusen, die mit mehrfach modifizierter EGFPLuc-mRNA behandelt worden waren, bis 5 Tage nach der Behandlung.

Insbesondere wird durch die vorliegende Erfindung eine RNA bereitgestellt, deren therapeutisches Potenzial die Behandlung der auf SP-B Defizienz zurückzuführenden Krankheit zulässt, nämlich s2U$_{(0.25)}$m5C$_{(0,25)}$SP-B-mRNA. SP-B ist ein relativ kleines amphipathisches Peptid, das von einem einzelnen Gen codiert wird und durch proteolytische Prozessierung einen Vorläufer mit 381 Aminosäuren in Alveolarepithelzellen vom Typ II, die die Alveolen auskleiden, erzeugt. Es verbessert die Verteilung, Adsorption und Stabilität der Surfactantlipide, die zur Reduktion der Oberflächenspannung in den Alveolen erforderlich sind. Bei Mangel an SP-B kommt es zu Symptomen wie verdickten Alveolarwänden, zellulärer Infiltration und interstiellen Ödemen. Diese Lungenschädigung wird von Kongestion, d.h. einer erhöhten Anzahl von Erythrozyten, und einer erhöhten Anzahl von Makrophagen, Neutrophilen und entsprechenden Anteilen an entzündlichen Zytokinen in der Bronchoalveolarwaschflüssigkeit begleitet. Die erbliche Defizienz bei Menschen und Untersuchungen an transgenen Mäusen haben erwiesen, dass SP-B eine wesentliche Rolle beim Überleben nach der Geburt spielt. Erbliche SP-B-Defizienz, die durch Mutationen im SP-B-Gen entsteht, ist wesentlich für den Ersatz des Surfactants und führt zu einem tödlichen Versagen des Respirationstraktes bei Neugeborenen während der ersten Lebensmonate. Eine Lungentransplantation ist daher die einzige derzeit verfügbare therapeutische Intervention. Daher ist eine mRNA-Therapie für SP-B-Defizienz, die mit der erfindungsgemäßen RNA ermöglicht wird, eine wichtige alternative Behandlung.

Die erfindungsgemäße RNA kann zur Behandlung dieser Krankheit eingesetzt werden, bevorzugt mit Perfluorcarbon als Träger.

Daher wird auch ein pharmazeutisches Präparat beschrieben, umfassend Perfluorcarbon und $s2U_{(0,25)}m5C_{(0,25)}$SP-B-mRNA. Diese Kombination lässt es zu, SP-B in der Lunge von Patienten mit SP-B-Defizienz zu rekonstituieren, sodass die Überlebenschancen erhöht werden. Hierzu ist aufgrund der hohen Stabilität der erfindungsgemäßen RNA eine Dosierung in regelmäßigen Abständen, z.B. 1 bis 3 mal wöchentlich ausreichend. Bevorzugt wird hierzu die SP-B-mRNA als Aerosol intratracheal durch Sprühen mit Hochdruck verabreicht. Es wurde gefunden, dass die erfindungsgemäße mRNA die oben beschriebenen Symptome und damit die Lungenfunktion verbessern kann, was durch Überprüfung der Lungenparameter gezeigt werden kann, wie im Detail in den Beispielen ausgeführt.

Die erfindungsgemäße mRNA kann wirksam in therapeutischen Verfahren eingesetzt werden und macht eine Behandlung von auf mangelnden oder defekten Proteinen beruhenden Krankheiten möglich. Eine systemische Verabreichung der mehrfach modifizierten mRNA ist möglich. Es kann Fälle geben, in denen die mRNA-Translation in Zellen, die nicht von dem Gendefekt betroffen sind, unerwünscht ist, z.B. weil unerwünschte Nebenwirkungen entstehen. Um die mRNA gezielt nur in den Zellen translatieren zu lassen, die das codierte Protein benötigen, z.B. in Zellen, in denen ein Gendefekt besteht, kann entweder der entsprechende Vektor um Sequenzen ergänzt werden, die ein Adressieren des betroffenen Gewebes ermöglichen, z..B. über Liganden. In einer weiteren Ausführungsform können dem Vektor, der die mRNA enthält, Sequenzen, an die endogene micro-RNAs binden, die in den Zielzellen nicht exprimiert werden, zugefügt werden, so dass die mRNA in allen Zellen, die die entsprechenden endogenen micro-RNAs enthalten, abgebaut werden, während sie in den Zielzellen erhalten bleiben. Dadurch können Nebenwirkungen minimiert werden.

Die erfindungsgemäße RNA kann in an sich bekannter Weise an Patienten, die das von RNA codierte Protein oder Proteinfragment benötigen, z.B. weil sie eine auf einem defizienten Gen beruhende Krankheit haben, verabreicht werden. Dazu wird die RNA als pharmazeutisches Präparat mit üblichen pharmazeutisch annehmbaren Hilfsstoffen formuliert. Die Form des Präparats hängt ab von dem Ort und der Art der Verabreichung. Da sich die erfindungsgemäße RNA durch besonders hohe Stabilität auszeichnet, kann sie in vielfältiger Weise formuliert werden, je nachdem wo und in welcher Form sie angewendet werden soll. Es wurde gefunden, dass die erfindungsgemäße RNA so stabil ist, das sie gefriergetrocknet werden kann, in dieser Form verarbeitet, z.B. zerkleinert bzw. gemahlen, und aufbewahrt werden kann, und dann bei Bedarf rekonstituiert werden kann und ihre biologische Aktivität behält.

Wenn die RNA systemisch verabreicht wird, wird sie üblicherweise als injizierbare Flüssigkeit mit üblichen Hilfsstoffen, wie die Tonizität einstellenden Mitteln und Stabilisierungsmitteln formuliert, bevorzugt als Einheitsdosierungsform. Als Stabilisierungsmittel werden die üblicherweise bekannten verwendet, wie z.B. Lipide, Polymere und Nanosysteme oder Liposomen. In einer bevorzugten Ausführungsform wird eine zur parenteralen Verabreichung geeignete Zusammensetzung bereitgestellt, die erfindungsgemäß modifizierte RNA enthält, die EPO codiert.

In einer bevorzugten Ausführungsform, insbesondere wenn die RNA SP-B-Protein codiert, wird die erfindungsgemäße RNA in einer zur Aufnahme über die Lunge, z.B. durch Inhalation, geeigneten Form bereitgestellt. Geeignete Rezepturen hierfür sind dem Fachmann bekannt. Das Präparat ist In diesem Fall in einer Form, die über übliche Vernebler oder Inhalatoren in den Atemtrakt eingebracht werden kann, z.B. als zu vernebeinde Flüssigkeit oder als Pulver. Vorrichtungen zur Verabreichung als Flüssigkeit sind bekannt, geeignet sind Ultraschallvernebler oder Vernebler mit einer perforierten schwingenden Membran, die mit geringen Scherkräften im Vergleich zu Düsenjetverneblern arbeiten. Ebenfalls geeignet sind Pulveraerosole. Sowohl mit kationischen Lipiden komplexierte mRNA als auch nackte mRNA ist nach der Gefriertrocknung mit dem Zucker Sucrose als Pulver verfügbar, dass anschließend auf eine lungengängige Größe zerkleinert werden kann und weiterhin biologische Aktivität zeigt.

Beschrieben wird insbesondere eine zur pulmonalen Verabreichung vorgesehene pharmazeutische Zusammensetzung kombiniert mit Perfluorcarbon, das vorher oder gleichzeitig mit der pharmazeutischen Zusammensetzung verabreicht wird, um die Transfektionseffizienz zu erhöhen.

In einer weiteren bevorzugten Ausführungsform wird erfindungsgemäß modifizierte RNA in einem verzögert freisetzenden Polymer als Träger zur Beschichtung von Implantaten bereitgestellt. Hierfür kann sowohl die erfindungsgemäß modifizierte RNA an sich als auch eine mit Hüllpolymer und/oder Polymerkomplex geschützte RNA verwendet werden.

Ein weiterer Gegenstand der Erfindung sind Implantate, auf deren Oberfläche sich ein Überzug befindet aus einem verzögert freisetzenden Polymer, das RNA enthält, die zum Einwachsen des Implantats nützliche Faktoren codiert. Erfindungsgemäß in Betracht kommen dabei sowohl Beschichtungen, die mRNA enthalten, die nur einen Faktor codiert als auch solche Beschichtungen, die mRNA enthalten, die mehrere Faktoren, z.B. verschiedene Wachstumsfaktoren oder Wachstumsfaktoren und Angiogenesefaktoren oder weitere zum Einwachsen förderliche Faktoren codieren. Die unterschiedlichen Faktoren können auch in solcher Form bereitgestellt werden, dass sie zeitlich gestaffelt abgegeben werden.

Weiterhin ist unter dem Ausdruck "RNA, die einen oder mehrere Wachstumsfaktoren und einen oder mehrere Angiogenesefaktoren codiert" sowohl eine RNA-Sequenz zu verstehen, die mehr als ein Protein codiert, einzeln oder als Fusionsprotein, als auch eine Mischung von unterschiedlichen RNA-Sequenzen, die unterschiedliche Proteine codieren, wobei jede RNA-Sequenz jeweils ein Protein codiert.

Die Erfindung wird durch die folgenden Beispiele weiter erläutert.

Beispiel 1

Um die therapeutische Anwendbarkeit einer IVT-mRNA beurteilen zu können, wurde ausgewertet, ob nicht immunogene IVT-mRNA erhalten werden könnte für eine in-vivo-Anwendung. In einer ersten Stufe wurde daher in vitro transkribierte mRNA für das rot fluoreszierende Protein (RFP) mit modifizierten Nucleosiden in Bezug auf Immunogenizität und Transfektionseffizienz untersucht. Die Ergebnisse zeigen, dass mehrfach modifizierte mRNA, bei der 25% des Uridins durch 2-Thiouridin (s2U) und 25% des Cytidins durch 5-Methylcytidin (m5C) ersetzt sind ($s2U_{(0,25)}m5C_{(0,25)}$) IVT-mRNA liefert, die eine stark reduzierte Immunogenität gegenüber menschlichen primären einkernigen Blutzellen hat, wie in Fig. 1A gezeigt, und eine hohe Transfektionsrate von mehr als 80% bei Epithelzellen des Alveolattyps II sowohl beim Menschen (Fig. 1B) als auch bei der Maus (Fig. 1C). Weiterhin war die Dauer der mRNA-Expression signifikant verlängert (Fig. 2A). Die Ergebnisse zeigen, dass diese verlängerte Expression hauptsächlich auf der höheren Stabilität der erfindungsgemäß mehrfach modifizierten mRNA beruht. Eine absolute quantitative Auswertung zeigte eine ungefähr 10-fach größere Menge an $s2U_{(0,25)}m5C_{(0,25)}$RFP-mRNA 7 Tage nach der Transfektion (Fig. 2B). Die Translationseffizienz war etwas vermindert für die modifizierte RFP-mRNA und konnte daher nicht zu einer höheren und längeren Aktivität beitragen (Fig. 4).

In der nächsten Stufe wurde der Mechanismus untersucht, auf dem die verminderte Immunantwort beruht, wobei ein modifizierter RNA-Immunfällungstest (RIP-Assay) verwendet wurde. Studien haben gezeigt, dass Zellen des Immunsystems durch unmodifizierte Reporter-mRNA (1) aktiviert werden durch RNA-Bindung an Toll-like Receptor (TLR) 3 (2), TLR7 (3), TLR8 (4) und Helicase RIG-1 (5). Die Ergebnisse zeigen, dass die Bindung der erfindungsgemäßen mehrfach modifizierten RFP-mRNA an TLR3, TLR7, TLR8 und RIG-1 im Vergleich zu unmodifizierter RFP-mRNA stark reduziert war. In dieser Hinsicht waren die Mehrfachmodifikationen wesentlich wirksamer als eine einzelne s2U-Modifikation (Fig. 2C). Wie aus den Bindungsstudien zu erwarten war, erhöhte unmodifizierte RFP-mRNA IFN-γ, IL-12 und IFN-α in erheblichem Ausmaß, wenn es Mäusen intravenös injiziert wurde, während mehrfach modifizierte $s2U_{(0,25)}m5C_{(0,25)}$RFP-mRNA eine Immunantwort verhinderte (Fig. 2D). Diese Ergebnisse zeigen insgesamt, dass die erfindungsgemäß mehrfach modifizierte mRNA die TLR- und RIG-1-Bindung und damit die Immunantwort stark verminderte und gleichzeitig die Expression erhöhte und verlängerte, was eine derartige mRNA zu einem vielversprechenden Kandidaten für in-vivo-Tests macht.

Es wurde daher getestet, ob eine $s2U_{(0,25)}m5C_{(0,25)}$mRNA, die ein Fusionsprotein aus verstärkt grün fluoreszierendem Protein und Luciferase (EGFPLuc) codierte, die direkt in die Lungen der Maus eingeführt wurde, die Luciferaseexpression in vivo verstärken und verlängern konnte im Vergleich zu unmodifizierter EGFPLuc-mRNA. Zu diesem Zweck wurde eine an sich bekannte Hochdrucksprühvorrichtung für intratracheale Verabreichung verwendet, wie sie z.B. in (6) beschrieben wird, wobei vorher Perfluorcarbon (Fluorinert FC-77) verabreicht wurde (7), um die Transfektionseffizienz zu erhöhen. Die Luciferaseexpression erreichte nach 3 Stunden in den Lungen in vivo ein Maximum, obwohl die Gesamtlumineszenz nach 24 Stunden schnell abnahm bis auf einen niedrigen Pegel 5 Tage nach der Behandlung (Fig. 3A und B). Im Gegensatz dazu wurden hohe Expressionswerte beobachtet bei Mäusen, die mit $s2U_{(0,25)}m5C_{(0,25)}$EGFPLuc-mRNA behandelt wurden, bis zum 5. Tag nach der Behandlung (Fig. 3A und B).

Dies zeigt, dass das therapeutische Potenzial der erfindungsgemäßen mehrfach modifizierten mRNA zur Therapie sehr vielversprechend ist. Es wurde daher eine erfindungsgemäß mehrfach modifizierte $s2U_{(0,25)}m5C_{(0,25)}$SP-B-mRNA zur Behandlung von SP-B-defizienten Mäusen getestet. SP-B ist ein relativ kleines amphipathisches Peptid, das von einem einzelnen Gen codiert wird und durch proteolytische Prozessierung zu einem Vorläufer mit 381 Aminosäuren in Epithelzellen des Alveolartyps II umgewandelt wird, das die Alveolen auskleidet (8, 9). Es verbessert die Ausbreitung, Adsorption und Stabilität der oberflächenaktiven Lipide, die zur Reduktion der Oberflächenspannung im Alveolus erforderlich sind. Wenn das Gen für dieses Protein defizient ist, kommt es nach der Geburt zu Störungen im Respirationstrakt, die schnell zum Tod führen können. Es wurde beobachtet, dass ein erblicher Mangel bei Menschen und bei transgenen Mäusen eine wichtige Rolle für das postmortale Überleben spielt (10). Eine erbliche SP-B-Defizienz, die durch Mutationen im SP-B-Gen entsteht, verhindert die Bildung der oberflächenaktiven Lipide, was zu einem respiratorischen Versagen während der ersten Monate nach der Geburt führt (*11*). Eine Lungentransplantation ist die einzige therapeutische Intervention, die derzeit möglich ist (*12*). Daher wäre eine mRNA-Therapie für die SP-B-Defizienz eine alternative Behandlung, um die Lebensfähigkeit bei diesem Mangel zu gewährleisten.

Es wurde daher ein Knockout-Mausmodell für SP-B-Defizienz ausgewählt, um eine Gentherapie mit erfindungsgemäßer mehrfach modifizierter mRNA von SP-B zu testen. Dazu wurde ein Mausmodell ausgewählt, bei dem die Maus-SP-B-cDNA unter der Kontrolle von exogenem Doxycyclin in SP-B$^{-/-}$-Knockout-Mäusen exprimiert wurde. Entzug von Doxycyclin bei ausgewachsenen SP-B$^{-/-}$-Mäusen führte zu einem verminderten Gehalt an SP-B in der Lunge, was zu einem respiratorischen Versagen führte, wenn die SP-B-Konzentration unter 25% des normalen Pegels fiel. Konditionierte transgene Mäuse, die Doxycyclin erhielten, überlebten normal *(13, 14)*. Die angewendete therapeutische Strategie beinhaltete Folgendes: (i) Vorbehandlung der Mäuse mit Perfluorcarbon vor der Zuführung von SP-B-mRNA, um die Expression zu erhöhen, (ii) wiederholte Anwendung von SP-B-mRNA zweimal wöchentlich jeden dritten oder vierten Tag vier Wochen lang (Fig. 3C). Um einen Versuch zum Beweis dieses Prinzips durchzuführen, wurde s2U$_{(0,25)}$m5C$_{(0,25)}$SP-B-mRNA über einen Hochdruckvernebler intratracheal als Aerosol an konditionale SP-B$^{-/-}$-Mäuse verabreicht. Diese Behandlung rettete die Mäuse vor einem respiratorischen Versagen und verlängerte ihre durchschnittliche Lebensspanne auf 28,8 $\pm$ 1,1 Tage (Fig. 3D), bis zum definierten Endpunkt der Studie. Im Gegensatz dazu zeigten nach Entzug des Doxycyclins unbehandelte SP-B$^{-/-}$-Mäuse Symptome eines akuten respiratorischen Problems innerhalb von 3 bis 4 Tagen. Dies wurde auch nach Verabreichung von Perfluorcarbon allein oder Perfluorcarbon mit s2U$_{(0,25)}$m5C$_{(0,25)}$EGFPLuc-mRNA als Kontrolle beobachtet, wobei die Mäuse dann innerhalb von 3,8 $\pm$ 0,4 Tagen starben (Fig. 3D, und nicht gezeigte Daten). Weiterhin wurde eine erfolgreiche Rekonstitution von SP-B in den Lungen der mit s2U$_{(0,25)}$m5C$_{(0,25)}$SP-B-mRNA behandelten Mäuse durch Immunfärbung (Fig. 3E) und halbquantitative Western-Blot-Analyse (Fig. 3F) für SP-B bestätigt. Die Lungenhistologie war bei Mäusen normal, die 4 Wochen lang mit s2U$_{(0,25)}$m5C$_{(0,25)}$SP-B-mRNA behandelt worden waren, während die Lungen der Mäuse, die s2U$_{(0,25)}$m5C$_{(0,25)}$EGFPLuc-Kontroll-mRNA erhalten hatten, verdickte Alveolenwände, zelluläre Infiltrationen und interstitielle Ödeme nach 4 Tagen zeigten (Fig. 3G). Diese Lungenschädigung wurde von einer Kongestion begleitet (erhöhte Anzahl an Erythrozyten) und einer erhöhten Anzahl an Makrophagen, Neutrophilen und einem erhöhten Pegel an inflammatorischen Cytokinen (Fig. 3H und Fig. S4) in der bronchoalveolaren Waschflüssigkeit (BALF), während dies bei den mit SP-B-mRNA behandelten Mäusen weitgehend verhindert wurde. Es wurde gezeigt, dass der Entzug von Doxycyclin die Lungenfunktion ohne Behandlung verschlechterte (*14, 15*). Es wurde beobachtet, dass eine längere Behandlung von SP-B$^{-/-}$-Mäusen mit s2U$_{(0,25)}$m5C$_{(0,25)}$SP-B-mRNA die normale Lungenfunktion aufrechterhielt, ähnlich wie bei den SP-B$^{-/-}$-Mäusen, die Doxycyclin erhielten (Fig. 3I und Fig. S5).

Zusammenfassend zeigen diese Ergebnisse, dass alle funktionellen und pathologischen Parameter der SP-B-Defizienz in der Lunge sich wesentlich verbesserten und vergleichbar waren mit konditionalen SP-B$^{-/-}$-Mäusen, die Doxycyclin erhielten.

Die Ergebnisse zeigen die therapeutische Wirksamkeit der mehrfach modifizierten mRNA in einem Mausmodell für eine tödliche Lungenkrankheit. Die weitere Anwendung der mRNA-Therapie kann jedoch noch verbessert werden wie folgt: (i) eine unerwünschte mRNA-Translation in Zellen von nicht betroffenem Gewebe könnte zu unerwünschten Wirkungen außerhalb des Zielgebiets führen, (ii) wenn die mehrfach modifizierte mRNA auch in nicht betroffenes Gewebe gelangt, muss eine ausreichende Menge an mRNA bereitgestellt werden und (iii) eine wiederholte Dosierung ist für eine kurzzeitige mRNA-Aktivität notwendig. Um dies zu verbessern, kann die Mikro-RNA-Biologie herangezogen werden, um unerwünschte mRNA-Translation in nicht von der Krankheit betroffenen Zellen zu verhindern. Indem Zielsequenzen von endogenen Mikro-RNAs, die in der Zielzelle nicht exprimiert werden, eingebaut werden, kann der mRNA-Abbau in nicht von der Krankheit betroffenen Zellen gezielt bewirkt werden, wobei aber die mRNA in den Zielzellen aufrechterhalten wird, wodurch Nebenwirkungen minimiert werden (*16, 17*).

In einem weiteren Ansatz können Abgabesysteme, die zielführende Liganden, die an Zelloberflächen spezifische Rezeptoren binden, kombiniert werden, damit eine Rezeptor-vermittelte Transfektion der Zielzelle ermöglicht wird. Da heutzutage mRNA in großen Mengen hergestellt werden kann (18) und effiziente Herstellungsprozesse für die Herstellung auch von mehrfach modifizierter mRNA in größerem Maßstab möglich ist, ist der klinische Einsatz der erfindungsgemäßen mRNA möglich und lässt für jede Krankheit spezifisch zugeschnittene mRNA-Systeme zu entwickeln (19,20), wobei die Dosierungsfrequenz und die kurzzeitige Aktivität auf einem Minimum gehalten werden können, was mit den derzeit bekannten Therapien nicht möglich ist. Auf diese Art und Weise wird erfindungsgemäß eine wirksame molekulare Therapie für die Behandlung von auf einer Gendefizienz beruhenden Krankheit bereitgestellt.

Beispiel 2

Um zu zeigen, dass bei SP-B-defizienten Mäusen eine Besserung des Zustandes bzw. eine Erhöhung der Lebenserwartung nur durch Einsatz der erfindungsgemäß modifizierten mRNA, die SP-B codiert, erreicht wird, wurde ein weiterer Versuch durchgeführt. Es wurden das Mausmodell und Bedingungen, wie in Beispiel 1 beschrieben, verwendet.

Es wurden drei Gruppen von Mäusen gebildet. Eine Gruppe von SP-B-defizienten Mäusen erhielt zweimal in einer Woche erfindungsgemäß modifizierte mRNA (B), eine zweite Gruppe erhielt 28 Tage lang zweimal pro Woche erfindungsgemäß modifizierte mRNA (C), und zum Vergleich erhielt eine dritte Gruppe von Mäusen modifizierte EGFP-Luc-mRNA (A).

Es zeigte sich, dass die Mäuse, die keine erfindungsgemäß modifizierte SPB-mRNA erhielten, nach kurzer Zeit starben. Die Mäuse, die die erfindungsgemäße RNA erhielten, überlebten nur solange, wie sie die erfindunggemäße SP-B-RNA bekamen. Dies beweist, dass die erfindungsgemäße RNA biologisch aktiv ist und notwendiges Protein ersetzen kann.

Im Detail wurde der Versuch wie folgt durchgeführt. SP-B KO-Mäuse, wie in Beispiel 1 beschrieben, erhielten entweder modifizierte EGFP-Luc-mRNA (A) (n = 10) oder modifizierte SP-B-mRNA zweimal in einer Woche (B) (n = 4) oder modifizierte SP-B-mRNA 28 Tage lang zweimal pro Woche (C) (n = 4). Es wurden Kaplan-Meier-Überlebenskurven erstellt und ein Wilcoxon-Gehan-Test durchgeführt. Es wurde gefunden, dass die intratracheale Applikation der doppelt modifizierten SP-B-mRNA zweimal innerhalb einer Woche in die Lunge transgener SP-B-Mäuse (B), in denen das SP-B-Gen durch die Zugabe von Doxycyclin im Trinkwasser kontrolliert ist, die durchschnittliche Überlebenszeit der Mäuse nach Entzug des Doxycyclins aus dem Trinkwasser vor dem Beginn der Behandlung auf 10,2 ± 0,5 Tage (B) verlängert im Vergleich zu 3,4 ± 0,2 Tage nach Applikation einer EGFP-Luc-Kontroll-mRNA.

Die Ergebnisse sind in dem Diagramm von Fig. 12 dargestellt. Es zeigt sich, dass die intratracheale Applikation der erfindungsgemäßen doppelt modifizierten SP-B-mRNA tatsächlich lebensrettend ist. Ohne Zugabe der erfindungsgemäßen mRNA sterben die Mäuse nach kurzer Zeit ab. Dieser Versuch zeigt auch, dass einerseits SP-B-mRNA das lebensnotwendige SP-B in vivo erzeugt und andererseits, dass die SP-B-mRNA kontinuierlich appliziert werden muss, um die Versuchstiere vor dem Tod zu bewahren.

Beispiel 3

In einem weiteren Versuch, in dem die in Beispiel 1 beschriebenen Mäuse verwendet wurden, die alle Doxycylin erhielten, wurde untersucht, ob die erfindungsgemäße RNA in einer frühen Phase nach Verabreichung Entzündungsreaktionen hervorruft. Hierzu wurden 5 Gruppen gebildet und Cytokinspiegel, IFNγ und IL-12, 8 Stunden nach Applikation unterschiedlicher Präparate in der bronchoalveolären Lavage von Mäusen gemessen. Die sechs Gruppen, erhielten die folgenden Präparate: a) Vergleich, unbehandelt, d.h. weder Perfluorcarbon noch RNA; b) Vergleich, Perfluorcarbon; c) Vergleich, Perfluorcarbon und unmodifizierte SP-B-mRNA; d) Erfindung, Perfluorcarbon und modifizierte s2U(0,25)m5C(0,25)SP-B-mRNA; e) Vergleich, Perfluorcarbon und SP-B-Plasmid-DNA; (n = 4). Es wurden jeweils 20 μg (50 μl) eines Präparats verabreicht. Die Ergebnisse sind in Fig. 13 gezeigt. In Figur 13 ist der Mittelwert ± Standardfehler gezeigt. Folgende Abkürzungen wurden in Figur 13 verwendet: Doxy - Doxycyclin, Pfc - Perfluorcarbon, pDNA - Plasmid-DNA (*P < 0,05 verglichen mit der unbehandelten Gruppe).

Die Ergebnisse zeigen, dass bei intratrachealer Applikation von unmodifizierter mRNA oder Plasmid-DNA der Entzündungsmarker IL-12 in der bronchoalveolären Lavage stark erhöht ist, während die Applikation von doppelt modifizierter mRNA im Vergleich zu unbehandelten Mäusen zu keinem Anstieg von IL-12 führt. Die Applikation von doppelt modifizierter mRNA erhöht zwar den Spiegel des Entzündungsmarkers IFNγ leicht, allerdings nur soweit, wie es auch nach Applikation von Perfluorcarbon beobachtet wird. Im Gegensatz dazu führt die Applikation von unmodifizierter mRNA oder die Applikation von Plasmid-DNA auch zu einem deutlichen Anstieg des INFγ-Spiegels. Bei Verwendung der erfindungsgemäß modifizierten mRNA ist somit eine entzündliche Reaktion nicht zu befürchten, während die Verabreichung von unmodifizierter mRNA oder auch Plasmid-DNA sehr schnell Entzündungsreaktionen hervorruft.

Beispiel 4

Um die Einsatzmöglichkeiten der erfindungsgemäß modifizierten mRNA zu zeigen, wurden verschiedene Arten von Modifikationen und deren Einfluss auf die Transfektions- und Translationseffizienz sowie auf die Immunogenität untersucht. Es wurden A459-Zellen mit jeweils 200 ng mRNA transfiziert und dann untersucht, wie viele der Zellen transfiziert worden waren und in wie vielen Zellen das fluoreszierende Protein translatiert worden war. Diese Auswertung erfolgte über die mittlere Fluoreszenzintensität (MFI). Die Ergebnisse sind in Fig. 10A gezeigt. Getestet wurde erfindungsgemäß modifizierte mRNA und im Vergleich dazu eine nicht erfindungsgemäß modifizierte mRNA, bei der zwei unterschiedliche Modifikationen von Uridinnucleotiden verwendet wurden sowie nicht modifizierte mRNA. Die erfindungsgemäß modifizierten mRNA-Moleküle waren:

s2U/m5C bzw. s4U/m5C, wobei die modifizierten Nucleotide jeweils einen Anteil von 10% hatten sowie RNA-Moleküle, die zusätzlich zu jeweils 10%/10% s2U/m5C bzw. s2U/5mC, weitere 5% modifizierte Nucleotide enthielt, nämlich einmal $C_2$'$NH_2$ und einmal 5% G'$N_3$. Die Ergebnisse zeigen, dass die erfindungsgemäß modifizierte mRNA eine sehr hohe Transfektionseffizienz zeigt, während unmodifizierte mRNA und nicht erfindungsgemäß modifizierte mRNA jeweils eine weit geringere Transfektions- und Translationseffizienz zeigt.

Für die vorher beschriebene modifizierte mRNA wurde außerdem die Immunogenität getestet, indem der TNF-α-Pegel an menschlichen PBMCs. nach Verabreichung von jeweils 5 μg mRNA untersucht wurde. Die Ergebnisse sind in Fig. 10B dargestellt. Wie sich eindeutig zeigt, ist der TNF-α-Pegel stark erhöht bei Verabreichung von unmodifizierter mRNA oder bei mRNA, bei der zwei Arten von modifizierten Uridinnucleotiden verwendet wurden. Der TNF-$\alpha$-Pegel ist bei den erfindungsgemäß modifizierten RNAs um mindestens 50% geringer als bei unmodifizierter RNA.

Beispiel 5

Verfahren zur Herstellung von erfindungsgemäßer mehrfach modifizierter mRNA

a) Konstrukte für die in-vitro-Transkription

Für die in-vitro-Transkription von RFP-cDNA (678 bp) wurde ein einen SP6-Promotor enthaltendes Plasmid, pCS2+DsRedT4 verwendet. Für die in-vitro-Transkription von SP-B-cDNA (1146 bp) wurde ein einen T7-Promotor enthaltendes pVAX1-Plasmid (Invitrogen) verwendet. Um den Vektor für die in-vitro-Transkription von EGFPLuc (2,4 kb) zu erzeugen, wurde ein einen T7-Promotor enthaltendes pST1-2$\beta$-Globin-UTR-A-(120)-Konstrukt verwendet, das wie in (19) beschrieben erhalten wurde. Die Konstrukte wurden unter Verwendung von Standardtechniken der Molekularbiologie kloniert.

Erzeugung von modifizierter mRNA

Um Template für die in-vitro-Transkription zu erzeugen, wurden die pCS2+DsRed.T4 -, EGFPLuc- und SP-B-Plasmide mit *Xba*I linearisiert. Die linearisierten Vektor-DNAs wurden mit dem NucleoSpin-Extrakt-II-Kit (Macherey-Nagel) gereinigt und spektrophotometrisch ausgewertet. Die in-vitro-Transkription wurde mit dem mMESSAGE-mMACHINE SP6- bzw. T7-Ultrakit (Ambion) durchgeführt. Der SP-6-Kit verkappte die mRNA mit 7-MethylGpppG, während der T7-Kit die analoge antireverse Kappe (ARCA; 7-Methyl-(3'-O-methyl)GpppGm$^7$G(5)ppp(5')G in einer Transkriptionsreaktion mit ultrahoher Ausbeute erzeugte. Um RNA-Modifikationen zu erzeugen, wurden die folgenden modifizierten Ribonucleinsäuretriphosphate dem Reaktionsansatz in den angegebenen Verhältnissen zugefügt: 2'-Thiouridin-5'-triphosphat, 5'-Methylcytidin-5'-triphosphat, Pseudouridin-5'-triphosphat und N$^-$Methyladenosin-5'-triphosphat (alle von TriLink BioTechnologies und bezüglich der Reinheit mit HPLC und $^3$P-NMR kontrolliert). Nach der in-vitro-Transkription wurde die RNA aus dem pVAX1-SP-B-Plasmid enzymatisch polyadenyliert unter Verwendung des Poly(A)-Tail-Kits (Ambion). Die Poly(A)-Tails waren ungefähr 200 nt lang. Alle verkappten mRNAs (RFP, EGFPLuc und SP-B) wurden gereinigt unter Verwendung des MEGAclear-Kit (Ambion) und auf Größe und Reinheit analysiert mit dem Agilent RNA 6000 Nano Assay an einem Bioanalysegerät 2100 (Agilent Technologies).

Zelltransfektionen

Lungenzelltransfektion

Alveolar-Epithelzelllinien vom Typ II vom Menschen und von der Maus, A549 bzw. MLE12, wurden in Minimal-Essential-Medium (Invitrogen), das mit 10% fötalem Kälberserum (FCS), 1 % Penicillin-Streptomycin und 0,5% Gentamycin ergänzt war, gezüchtet. Einen Tag vor der Transfektion wurden 80.000 Zellen pro Napf in 24-Napf-Platten ausplattiert. Die Zellen (mehr als 90% Konfluenz) wurden mit 200 ng mRNA transfiziert unter Verwendung von Lipofectamin 2000 (Invitrogen) gemäß der Anleitung des Herstellers. Nach 4 Stunden wurden die Zellen mit PBS gewaschen und serumhaltiges Medium wurde zugefügt. Für Analysen zur langzeitigen Expression wurden die Zellen regelmäßig aufgeteilt (wenn die Konfluenz > 90% war).

Human-PBMC-Transfektion

In flüssigem Stickstoff kryokonservierte menschliche PBMCs (CTL-Europe GmbH) wurden bei 37°C vorsichtig aufgetaut unter Verwendung von CTL-Anti-Aggregate-Wash Supplement, wobei langsam sterilfiltriertes RPMI-1640 (Invitrogen) zugefügt wurde. Für alle beschriebenen Versuche wurde eine einzige charakterisierte Charge von PBMCs verwendet, um die Daten reproduzierbar zu machen.

Durchflusscytometrie

Eine durchflusscytometrische Analyse wurde durchgeführt an den A549- und MLE12-Zellen, die mit RFP-mRNA, wie oben beschrieben, transfiziert worden waren. Die Zellen wurden von der Plattenoberfläche mit 0,25% Trypsin/EDTA abgenommen, dreimal mit PBS gewaschen und wieder in PBS suspendiert, um die Fluoreszenz unter Verwendung eines FACSCalibur (BD Biosciences) zu messen. Die Transfektionseffizienz wurde berechnet aus dem Prozentanteil an Zellpopulation, der die Fluoreszenzintensität der Kontrollzellen, die nur mit PBS behandelt worden waren, überstieg. Mindestens 2500 Zellen pro Röhrchen wurden gezählt. Die Daten wurden mit Cellquest Pro analysiert.

Cytokinnachweis

Enzym-linked Immunosorbent Assays (ELISA) wurden durchgeführt unter Verwendung von Human-IL-8 und TNF-α-Kits (RayBio), Maus-IFN-γ- und IL-12-(P40/P70)-Kits (RayBio) und Maus-IFN-α-Kit (RnD Systems).

Echtzeit-in-vitro-Translation

500 ng RFP-mRNA wurde in vitro translatiert unter Verwendung von Retic Lysat IVT (Ambion). Methionin wurde auf eine Endkonzentration von 50 μM zugefügt. Die Mischung wurde bei 30°C in einem Wasserbad inkubiert und zu verschiedenen Zeitpunkten wurden Proben entnommen und die Fluoreszenzintensität bei 590 nm an einem Wallac Victor[2] 1420 Multilabel Counter (Perkin Elmer) gemessen.

Quantitative RT-PCR

Die Gesamt-RNA wurde aus A549-Zellen mit RNeasy Minikit (Qiagen) oder aus menschlichen PBMCs (siehe RIP-Protokoll unten) extrahiert und einer reversen Transkription (RT) in einem Ansatz von 20 μl unterzogen unter Verwendung von iScript cDNA Synthesekit (Bio-Rad) gemäß Produkthandbuch. cDNA wurde amplifiziert unter Verwendung von iQ SYBR Green Supermix und iCycler (Bio-Rad) in doppelten Ansätzen mit den folgenden Primern: RFP: 5'-GCACCCAGACCGCCAAGC (vorwärts), RFP: 5'-ATCTCGCCCTTCAGCACGC (rückwärts). $C_t$-Werte wurden erhalten unter Verwendung der iCycler IQ Software 3.1 (Bio-Rad), die automatisch die Basiszyklen und Schwellenwerte berechnete.

RNA-Immunpräzipitation (RIP)

1 x 10⁶ menschliche PBMCs (CTL-Europe GmbH) wurden mit 5 μg mRNA transfiziert unter Verwendung von 12,8 μl Lipofectamin 2000 in 1 ml OptiMEM 1. Nach 4 Stunden wurden die Medien mit 10% FCS ergänzt. Nach 24 Stunden wurde die Zellsuspension in Röhrchen überführt und die Zellen wurden durch 10-minütige Zentrifugation mit 350 Upm pelletisiert. Anschließend wurde eine modifizierte Version des ChIP-IT-Expressprotokolls (ActiveMotive) verwendet, um die RIP durchzuführen. Mit DEPC behandeltes Wasser (Serva Electrophoresis) wurde zur Herstellung aller notwendigen Reagenzien verwendet. Gemäß dem ChIP-IT Handbuch wurde den Zellen die Fixierungslösung und anschließend die Glycin-Stop-Fix-Lösung und eiskaltes 1 x PBS zugefügt und die Zellen wurden bei 4°C pelletisiert. Dann wurden die Zellen wieder in Lysepuffer suspendiert, dem die Proteaseinhibitoren PIC und PMSF zugefügt worden waren, und 30 min auf Eis inkubiert. Nach 10-minütiger Zentrifugation mit 2.400 Upm bei 4°C wurde der Überstand der Einfang-Reaktion unterzogen. Die TLR-mRNA/RIG-mRNA-Komplexe wurden über Nacht an magnetischen Kugeln in 8-Napf-PCR-Streifen eingefangen, wie in dem ChIP-IT-Express-Handbuch beschrieben. Außerdem wurde SUPERase-RNase-Inhibitor (Applied Biosystems/Ambion) auf eine Endkonzentration von 1 U/μl zugefügt. Anti-Human-TLR3-Maus-IgG1, TLR7-Kaninchen-IgG1, TLR8-Maus-IgG1 (alle von Imgenex) und RIG-1-Kaninchen-IgG1 (ProSci Incorporated) wurden als Antikörper verwendet. Nach dem Waschen der magnetischen Kugeln wurden die TLRmRNA/RIG-mRNA-Antikörperkomplexe eluiert, revers vernetzt und mit Proteinase K gemäß dem ChIP-IT-Expressprotokoll behandelt. Schließlich wurde die eluierte mRNA einer reversen Transkription und einer quantitativen RT-PCR unterzogen, wie oben beschrieben.

In-vivo-Biolumineszenz

D-Luciferinsubstrat wurde in Wasser gelöst, der pH-Wert auf 7 eingestellt und das Endvolumen so eingestellt, dass eine Konzentration von 30 mg/ml erreicht wurde. 50 μl dieser Lösung wurden auf die Nüstern der anästhesierten Mäuse aufgetragen und durch Schnüffeln absorbiert (1,5 mg Luciferin/Maus). Nach 10 min wurde die Biolumineszenz mit einem IVIS100-Bildsystem (Xenogen) wie in (21) beschrieben gemessen unter Verwendung der folgenden Kameraeinstellungen: Gesichtsfeld 10, f1 f-stop, Hochauflösungs- und Belichtungszeiten von 1 bis 10 min. Das Signal in der Lungenregion wurde quantitativ ausgewertet und analysiert, wobei der Hintergrund abgezogen wurde unter Verwendung der Living Image Software Version 2.50 (Xenogen).

Tieruntersuchungen

6 bis 8 Wochen alte weibliche BALB/C-Mäuse (Charles River Laboratories) wurden unter spezifischen pathogenfreien Bedingungen gehalten und mit einem 12-Stunden Hell:12-Stunden-Dunkel-Zyklus in einzeln belüfteten Käfigen gehalten, und mit Futter und Wasser ad libitum versorgt. Die Tiere wurden mindestens 7 Tage vor dem Start der Versuche akklimatisiert. Alle Tiermanipulationen waren zugelassen und wurden von der lokalen Ethikkommission kontrolliert und nach den Richtlinien des deutschen Tierschutzgesetzes durchgeführt. Für alle Versuche, außer der Injektion in die Schwanzvene wurden die Tiere i.p. mit einer Mischung von Medetomidine (0,5 mg/kg), Midazolam (5 mg/kg) und Fentanyl (50 $\mu$g/kg) anästhesiert. Nach dem jeweiligen Versuch wurden den Tieren ein Antidot s.c. verabreicht, das aus Atipamezol (50 $\mu$g/kg), Flumazenil (10 $\mu$g/kg) und Naloxon (24 $\mu$g/kg) bestand. Blut für die ELISA-Tests wurde zu verschiedenen Zeitpunkten durch Punktion der retrobulbären Vene erhalten unter Verwendung von heparinisierten 1,3-mm-Kapillaren (Marienfeld).

Injektion in die Schwanzvene:

25 $\mu$g RFP-mRNA wurden in vivo mit Megafectin (MP Biomedicals Europe) vermischt in einem Verhältnis von mRNA zu Lipid von 0,25 und Enhancer-3 wurde nach der Empfehlung des Herstellers zugefügt. Die Integrität und Teilchengröße der injizierten Komplexe wurde mit dynamischer Lichtbeugung (DLS) unter Verwendung eines Zeta-PALS/Zeta-Potenzial-Analysators (Brookhaven Instruments Corp.) bestimmt. Die Mäuse wurden in einen Restrainer gelegt und 100 $\mu$l der mRNA/Megafectin-Lösung (Äquivalent 5 $\mu$g mRNA) wurden in die Schwanzvene innerhalb von 30 Sekunden injiziert unter Verwendung einer 27-Gauge-Nadel und einer 1-ml-Spritze.

Intratracheale Verabreichung durch Hochdruckvernebelung:

BALB/c- und SP-B$^{-/-}$-Mäuse wurden wie in (14) beschrieben anästhesiert und so an einem Plattensystem (Halowell EMC) fixiert, dass die oberen Zähne in einem Winkel von 45° waren. Ein modifiziertes Kaltlichtothoskop Beta 200 (Heine Optotechnik) wurde verwendet, um den Rachen optimal auszuleuchten. Mit einem kleinen Spatel wurde der untere Kiefer der Maus geöffnet und eine stumpfe Pinzette wurde verwendet, um die Zunge zu verschieben, und den Oropharynx maximal freizulegen. Ein MikroSprayer Modell 1A-1C, der mit einer Hochdruckspritze Modell FMJ-250 (beide von PennCentury-Inc.) verbunden war, wurde endotracheal eingesetzt und aufeinander folgend wurden 25 $\mu$l Fluorinert FC-77 (Sigma) und 25 $\mu$l Luciferase-mRNA (10 $\mu$g) oder 50 $\mu$l SP-B-mRNA-Lösung (20 $\mu$g) angewendet. Die MikroSprayerspitze wurde nach 5 s abgezogen und die Maus wurde nach 5 min von dem Träger genommen.

Lungenfunktionsmessungen

Homozygote SP-B$^{-/-}$-Mäuse $\pm$ Doxycyclin $\pm$ modifizierte mRNA wurden wie oben beschrieben anästhesiert. Um spontanes Atmen zu verhindern, wurde Vecuroniumbromid (0,1 mg/kg) intraperitoneal injiziert. Die lungenmechanischen Messungen wurden durchgeführt, wie in (22) beschrieben. Kurz gesagt wurde eine stumpfe Stahlkanüle (äußerer Durchmesser 1 mm) in die Trachea mit Tracheostomie eingesetzt. Der Kolbenpumpenrespirator diente sowohl als Respirator als auch als Messvorrichtung (flexiVent, SAV). Während der Tidal-Ventilation wurde der Respirator eingestellt auf kontrollierte volumen- und druckbegrenzte Ventilation (Vt = 10 $\mu$l/g; Pmax = 30 cm H$_2$O, PEEP 2 - 3 cm H$_2$O) bei 2,5 Hz und 100% Sauerstoff. Die angewendete Vt war 8,4 $\pm$ 1,4 $\mu$l/g bei Tieren, die Doxycyclin erhielten und 8,9 $\pm$ 0,4 $\mu$l/g BW bei Tieren, die Doxycyclin und mRNA erhielten (N.S.). Die dynamischmechanischen Eigenschaften des Atmungssystems ebenso wie die Lungeneingangsimpedanz wurden in 5-minütigen Intervallen an Tieren gemessen, nachdem zweimal mit 15 $\mu$l/g für 1 s aufgeblasen worden war, um eine Standardvolumenhistorie zu erzeugen. Für die oszillatorische Messung wurde die Ventilation auf PEEP-Level gestoppt. Um die Impedanz des respiratorischen Systems (Z$_{rs}$) durch erzwungene Oszillationen (FOT) zu bestimmen, die aus einem pseudostatistischen oszillatorischen Signal von 8 s bestanden, wurde eine Amplitude von 3 ml/g angewendet. Das erzwungene Signal hatte Frequenzen zwischen 1,75 und 19,6 Hz (23,24). Die Daten wurden bei 256 Hz gesammelt und mit einem Fenster von 4 s bei 66% Überlappung analysiert. Die Lungenimpedanzdaten wurden als Widerstand (realer Teil) und Reaktionsfähigkeit (imaginärer Teil) des Atmungssystems innerhalb der Frequenzdomäne dargestellt. Die Lungenimpedanzdaten (Zrs) wurden aufgeteilt, wobei das konstante Phasenmodell der Lunge angewendet wurde, wie von Hantos et al. (25) vorgeschlagen. Bei diesem Modell besteht Zrs aus einem Atemwiderstand (Rn), einer Atemwegsinertia (Inertia), einer Gewebeelastizität (H$_L$) und einer Gewebedämpfung (G$_L$) nach der Gleichung:

$$Zrs = Raw + j\omega law + (G_L - jH_L)/\omega^\alpha,$$

wobei $\omega$ die Winkelfrequenz ist und $\omega$ die Frequenzabhängigkeit von Zrs ($\omega = (2/\omega \tan^{-1}(1/\omega))$. Die Lungenhysteresivität (eta = G$_L$/H$_L$) ist ein Maß für die Lungengewebezusammensetzung, wobei sowohl die Gewebedämpfung als auch die Gewebeelastizität eingebracht werden (26,27). Für jede Messung wird das konstante Phasenmodell automatisch auf die Anpassung getestet. Die Anpassungsqualität wird dargestellt als Kohärenz der Bestimmung (COD), die Daten werden zurückgewiesen, wenn der COD unter 0,85 ist.

Analyse des Surfactant Proteins

Der Gesamtproteingehalt der Lavage-Überstände wurde mit dem Bio-Rad Proteinassay-Kit (Bio-Rad) bestimmt. 10 µg Gesamtprotein wurden unter nicht reduzierenden Bedingungen auf NuPage 10% Bis-Tris-Gelen aufgetrennt unter Verwendung eines NOVEX Xcell II Mini-Cell System (Novex). Nach der Elektrophorese wurden die Proteine auf eine PVDF-Membran (ImmobilonP) mit einem NuPage Blot Modul (Novex) überführt. Surfactant Protein B (SP-B) wurde mit polyklonalem Kaninchen-Antiserum, das gegen SP-B gerichtet war, nachgewiesen (c329, Geschenk von Dr. W. Steinhilber, Altana AG) und anschließend wurde ein verbesserter Chemilumineszenztest (Amersham Biosciences) mit Meerrettichperoxidase konjugiertem polyklonalem Ziege-Anti-Kaninchen-Anti-IgG (1:10.000; Dianova) durchgeführt. Unter diesen Bedingungen konnte der Test etwa 2,5 ng SP-B pro Bahn nachweisen (28). Als Chemilumineszenz-Nachweissystem wurde DIANA III dev. 1.0.54 mit dem Aida-Bildanalysator (Ray test Isotopenmessgeräte GmbH) verwendet und die Daten wurden quantitativ ausgewertet mit Quantity One 4.6.7 (Bio-Rad).

Fluoreszenzmikroskopanalyse

Fixierte (3% Paraformaldehyd) und in Paraffin eingebettete Schnitte wurden einer Immunhistochemie unterzogen, wie vom Hersteller (Abcam, www.abcam.com/technica) empfohlen. Die Träger wurden mit Anti-Human-Anti-Maus-SP-B-Antikörper und mit Texasrot konjugiertem Anti-Kaninchen-IgG-Antikörper (beide von Abcam, 1:500) inkubiert und mit DAPI gegengefärbt. Fluoreszierende Bilder wurden von Zeiss Axiovert 135 erhalten.

Statistik

Unterschiede in der mRNA-Expression zwischen Gruppen wurden durch paarweise fixierte Reallokation-Randomisierungstests mit REST 2005 Software (29) analysiert. Die Halbwertszeiten für das Abklingen der Biolumineszenz wurden berechnet mit Prism 5.0. Alle anderen Analysen wurden durchgeführt unter Anwendung des Wilcoxon-Mann-Whitney-Tests mit SPSS 15 (SPSS Inc.). Die Daten sind angegeben als Mittelwert $\pm$ SEM (Standardfehler des Mittelwerts) oder als Median $\pm$ IQR (Interquartile Ranges) und $P < 0,05$ (zweiseitig) wurde als statistisch signifikant angesehen.

Beispiel 6

Erfindungsgemäß mehrfach modifizierte mRNA, die EPO codiert

Mit einem Verfahren im Wesentlichen, wie in Beispiel 3 beschrieben, wurde modifizierte mRNA hergestellt, die einen EPO codierenden Teil enthielt. Diese mRNA wurde auf ihre Expressionseffizienz getestet. Dazu wurden jeweils 5 µg erfindungsgemäß modifizierte mRNA bzw. nicht modifizierte mRNA in Mäuse i.m. injiziert. Jede Gruppe aus Mäusen hatte vier Mitglieder. Am Tag 14 und am Tag 28 nach Verabreichung der RNA wurde der Anteil an EPO im Serum mit einem ELISA-Test quantitativ ausgewertet. Der Hämatokritwert wurde in Vollblut von Mäusen bei dem gleichen Versuch ausgewertet. Die in der beigefügten Fig. 11 gezeigten Daten stellen jeweils den Mittelwert $\pm$ SEM dar. Der Scatter-Blot zeigt die einzelnen Hämatokritwerte, Balken zeigen Medianwerte. *$P < 0,05$ gegenüber der unbehandelten Gruppe zum jeweiligen Zeitpunkt; +$P < 0,05$ gegenüber der nicht modifizierten mEPO-Gruppe zum jeweiligen Zeitpunkt.

(c) Die Daten zeigen den Mittelwert $\pm$ SEM. Menschliche PBMCs wurden mit 5 µg unmodifiziertem bzw. modifiziertem RFP-mRNA transfiziert und die Wiedergewinnungsraten wurden mit RIP bestimmt unter Verwendung von für TLR-3, TLR-7 und TLR-8 spezifischen Antikörpern. Die Kästchen bedeuten Mittelwerte $\pm$ IQR. Die Striche zeigen die Minimal- bzw. Maximalwerte. *$P < 0,5$, **$P < 0,01$, ***$P < 0,001$ gegenüber unmodifizierter mEPO-Gruppe.

(d) 5 µg unmodifiziertes und modifiziertes mEPOmRNA wurden Mäusen intravenös injiziert (jeweils n = 4). Nach 24 Stunden wurden die Interferon-$\gamma$-, IL-12- und Interferon-$\alpha$-Pegel in Serum quantitativ mit ELISA ausgewertet.

Wie den Diagrammen zu entnehmen ist, sind für die erfindungsgemäß modifizierte RNA die Entzündungsmarker im unauffälligen Bereich, während für unmodifizierte RNA oder nur mit modifizierten Uridinnucleotiden modifizierte RNA die Entzündungsmarker stark erhöht sind.

Erfindungsgemäß wird somit eine mRNA, die EPO codiert, bereitgestellt, die sehr stabil ist und gleichzeitig keine bis wenig immunologische Reaktionen hervorruft. Eine derartige mRNA kann vorteilhaft zur Behandlung von Erythropoietinmangel eingesetzt werden. Aufgrund der hohen Stabilität ist eine Dosierung nur alle 2 bis 4 Wochen notwendig.

Beispiel 7

Es wurde untersucht, wie sich die wiederholte Applikation von EPO codierender erfindungsgemäß modifizierter mRNA auf die Hämatokrit-Werte auswirkt. Dadurch soll gezeigt werden, ob die erfindungsgemäß modifizierte mRNA auch über längere Zeit aktiv bleibt, wenn sie in den Körper appliziert wird. Eine immunologische Reaktion auf die erfindungsgemäße mRNA würde z.B. die Aktivität vermindern.

Es wurden daher 10 μg modifizierte mEpo-mRNA (wie in Beispiel 6 beschrieben) an den Tagen 0, 21 und 34 Mäusen intramuskulär appliziert (n = 10). Der Hämatokritwert wurde dann im Gesamtblut der Mäuse an den Tagen 0, 21, 34, 42 und 51 bestimmt. Die Ergebnisse sind in Fig. 14 gezeigt. Die Daten in dem Diagramm zeigen den Durchschnitt $\pm$ Standardfehler* P < 0,05 im Vergleich zum Hämatokritwert am Tag 0.

Die Ergebnisse belegen, dass wiederholte Applikation der erfindungsgemäß modifizierten mRNA zu einer lang anhaltenden Erhöhung des Hämatokritwerts führt. Dies zeigt, dass die mRNA, auch wenn sie mehrfach appliziert wird, aktiv bleibt.

Beispiel 8

Erfindungsgemäß modifizierte mRNA ist auch geeignet, um die Heilung oder das Einwachsen fördernde Proteine in die Umgebung von Implantaten zu bringen, um dadurch den Heilungsprozess oder das Einwachsen zu fördern. Um zu zeigen, dass die erfindungsgemäß modifizierte mRNA stabil und andauernd exprimiert wird, wenn sie in Form einer Beschichtung auf Titanoberflächen aufgebracht wird, wurde auf Titanplättchen ein Überzug aufgebracht, der mRNA enthielt, die Luciferase codierte. Es wurde dann überprüft, ob und wie lange Luciferase in der Umgebung, frei oder in Zellen, festgestellt werden konnte.

Zwei unterschiedliche Proteine codierende Sequenzen wurden für den Versuch verwendet, nämlich eine RNA für Luciferase, die von der sie exprimierenden Zelle sekretiert wird als Modell für solche Proteine, die in die Umgebung abgegeben werden sollen, wie z.B. Wachstumsfaktoren oder Angiogenesefaktoren. Weiterhin wurde RNA verwendet, die eine Luciferase codiert, die nicht sekretiert wird, sondern in der Zelle bleibt als Modell für solche Proteine, die in der Zelle etwas bewirken sollen. Für das Sekretionsmodell wurde RNA verwendet, die Metridia-Luciferase codierte, bei der im Vergleich zum Wildtyp 25% der Uridineinheiten durch s2U ersetzt waren und 25% der Cytidineinheiten durch m5C ersetzt waren. Für das Nichtsekretionsproteinmodell wurde eine Firefly-Luciferase codierende mRNA verwendet, bei der ebenfalls 25% der Uridineinheiten durch s2U ersetzt waren und 25% der Cytidineinheiten durch das modifizierte m5C.

Es wurde gefunden, dass die erfindungsgemäßen mRNA-Präparate, die geschützt war als Komplex mit Polymer, nach Abgabe aus dem Überzugsmaterial über längere Zeit aktiv blieben und exprimiert wurden. Es zeigte sich, dass das jeweils von der erfindungsgemäß modifizierten mRNA codierte Protein über längere Zeit nachgewiesen werden konnte.

Für die Tests wurde die erfindungsgemäß modifizierte mRNA, geschützt durch einen Polymerkomplex, in ein Trägermaterial, das als Schicht auf Titanplättchen aufgetragen wurde, eingebettet. Das Trägermaterial war Polylactid (PDLLA), ein für diesen Zweck wohlbekanntes Material, das die enthaltene mRNA gezielt nach und nach freisetzen kann. Vorteil eines derartigen Überzugs ist es, dass die Abgabe gezielt eingestellt werden kann. Die Ergebnisse zeigen, dass die beim Abbau frei werdenden Polylactidfragmente die Aktivität der mRNA nicht beeinträchtigen, so dass dieses System gut geeignet ist. Die mRNA selbst wurde durch ein Hüllpolymer stabilisiert.

Für die Versuche wurde Metridia-Luciferase codierende Plasmid-DNA (pDNA) bzw. modifizierte mRNA verwendet. jeweils 9 μg Metridia-Luciferase-pDNA oder doppelt modifizierte s2U(0,25)5C(0,25)mRNA in 200 μl H2O (+ gegebenenfalls 500 μg Lactose) wurden mit 9,4 μg L-PEI (L-Polyethylenimin) in 200 μl H2O komplexiert. Danach wurden die Komplexe in 100 μl einer Hüllpolymerlösung gegeben (2,4 μl 409,1 mM P6YE5C) und über Nacht lyophilisiert (das Hüllpolymer P6YE5C wurde hergestellt, wie in EP 11 98 489 beschrieben). Danach wurden die Komplexe in 72 μl einer PDLLA (Poly-DL-Lactid)/EtOAc (50 mg/ml PDLLA) Mischung auf Eis suspendiert und mithilfe eines Mikropotters dispergiert. Mit dieser Dispersion wurden autoklavierte Titanplättchen (r = 3 mm, je 18 μl) in einer 96-Napf-Platte beschichtet. Nach einer weiteren Lyophilisation über Nacht wurden A549-Zellen in 200 μl RPMI-1640-Medium zugegeben (5000 Zellen/200 μl). Ab dem zweiten Tag wurden jeweils 50 μl vom Überstand abgenommen, das Medium gewechselt und mithilfe von je 100 μl Coelenterazin-Lösung (0,003 mM Endkonzentration) an den folgenden Tagen die Metridia-Luciferase-Expression bestimmt.

In einem weiteren Versuch wurde die Aktivität der erfindungsgemäß modifizierten Metridia-Luciferase codierenden mRNA getestet, wenn diese auf Calciumphosphatpartikel abgeschieden wurde und in dieser Form in den Überzug eingebracht wurde. Hierzu wurden jeweils 4 μg Metridia-Luciferase s2U(0,25)m5C(0,25)mRNA in 600 μl 1 x HBS mit 33 μl 2,5 M CaCl2 gemischt. Nach 30 min wurden autoklavierte Titanplättchen (r = 3 mm, je 18 μl) in einer 96-Napf-Platte damit beschichtet. Nach Lyophilisation über Nacht wurden A549-Zellen in 200 μl RPMI-1640-Medium zugegeben

(5000 Zellen/200 μl). Ab dem zweiten Tag wurden jeweils 50 μl vom Überstand abgenommen, das Medium gewechselt und mithilfe von je 100 μl Coelenterazin-Lösung (0,003 mM Endkonzentration) an den folgenden Tagen die Metridia-Luciferase-Expression bestimmt.

Die Ergebnisse sind dem Diagramm in Fig. 15 zu entnehmen. Die Ergebnisse verdeutlichen, dass erfindungsgemäß modifizierte mRNA auch dann noch aktiv bleibt, wenn sie mit einer Polymerhülle geschützt wird, in eine verzögernd freisetzende Matrix eingebracht wird und auf Titanimplantate aufgebracht wird. Die erfindungsgemäß modifizierte mRNA bleibt weiterhin biologisch aktiv und wird kontinuierlich in das codierte Protein translatiert. Auch die Sekretionsfähigkeit bleibt erhalten, was sich daran zeigt, dass die Meridia-Luciferase im Zellkulturmedium nachgewiesen werden kann (als Modell für sekretierte Knochenwachstumsfaktoren wie z.B. BMP-2). Die Ergebnisse zeigen außerdem überraschenderweise, dass die Beschichtung mit modifizierter mRNA eine höhere Proteinexpression liefert als die Beschichtung von Titanimplantaten mit der analogen Plasmid-DNA. Wenn die mRNA/PEI-Komplexe mit einem Hüllpolymer versehen werden vor dem Einbau in die Titanimplantatbeschichtung, wird eine noch höhere Proteinexpression erhalten als bei Verwendung derselben Komplexe, aber ohne Hüllpolymer (in der Figur mod. mRNA/IPEI-P6YE5C). Weiterhin wurde gefunden, dass der Zusatz von Lactose als Hilfsstoff möglich ist, ohne dass die modifizierte mRNA ihre biologische Aktivität verliert.

Die Ergebnisse zeigen auch, dass auf Calciumphosphatpartikeln präzipitierte modifizierte mRNA ihre Aktivität behält und ihre vorteilhaften Eigenschaften in der Titanimplantatbeschichtung ausüben kann. Die biologische Aktivität bleibt erhalten. Dies ist von besonderer Bedeutung, da Calciumphosphat direkt in den Knochen eingebaut werden kann.

Wie oben angedeutet, wurde ein weiterer Versuch durchgeführt mit Firefly-Luciferase codierender DNA bzw. RNA. Hierzu wurden jeweils 9 μg Firefly-Luciferase pDNA bzw. modifizierte s2U0,25m5C0,25mRNA in 200 μl H2O mit 9,4 μg L-PEI in 200 μl H2O komplexiert. Danach wurden die Komplexe in 100 μl einer Hüllpolymerlösung gegeben (2,4 μl, 409,1 mM P6YE5C) und über Nacht lyophilisiert. Anschließend wurden die Komplexe in 72 μl einer Poly-DL-Milchsäure (PDLLA)/Ethylacetat (EtOAc) (50 mg/ml PDLLA)-Mischung auf Eis gelöst und mithilfe eines Mikropotters dispergiert. Mit dieser Dispersion wurden autoklavierte Titanplättchen (r = 3 mm, je 18 μl) in einer 96-Napf-Platte beschichtet. Nach einer weiteren Lyophilisation über Nacht wurden A549-Zellen in 200 μl RPMI-1640-Medium zugegeben (5000 Zellen/200 μl). Am zweiten Tag wurden je 1 μl 350 μM D-Luciferin zu den Näpfen gegeben, 20 min inkubiert und die Luciferaseexpression per Bio-Imaging bestimmt. Die Ergebnisse sind in Fig. 16 gezeigt. Wie dem Diagramm in Fig. 16 zu entnehmen ist, können Titanimplantate mit erfindungsgemäß modifizierter mRNA beschichtet werden, wobei die mRNA auch weiterhin biologisch aktiv bleibt und das codierte Protein translatiert. Das gebildete Protein bleibt in der Zelle und kann intrazellulär nachgewiesen werden. Außerdem zeigen die Ergebnisse, dass die Beschichtung mit modifizierter mRNA zu einer höheren Proteinexpression führt als die Beschichtung von Titanimplantaten mit der analogen Plasmid-DNA.

Beispiel 9

Um die Expression der erfindungsgemäß modifizierten mRNA so zu steuern, dass das codierte Protein nur in Zellen exprimiert wird, in denen es gewünscht ist, in anderen Zellen jedoch nicht, wurde eine Mikro-RNA-Bindungsstelle in die mRNA eingefügt, um eine zellspezifische Regulation der mRNA-Expression zu ermöglichen.

Hierzu wurden HEK293-Zellen in MEM mit 10% FCS und 1% Penicillin-Streptomycin kultiviert. 24 h vor der Transfektion wurden 100.000 Zellen/Napf in eine 24-Napf-Platte ausgesät. Unmittelbar vor der Transfektion wurde das Medium durch 400 μl Optimem (Invitrogen) ersetzt. U937-Zellen wurden in RPMI-1640-Medium mit 10% FCS und 1% Penicillin-Streptomycin kultiviert. Unmittelbar vor der Transfektion wurden 800.000 U937-Zellen in 400 μl Optimem-Medium (Invitrogen) pro Napf in eine 24-Napf-Platte ausgesät. Für jeden Napf wurden 100 ng EGFP-mRNA und 250 ng RFPmiRNA-BSmRNA (siehe unten) mit Optimem auf 50 μl verdünnt. 2 μl Lipofectamine 2000 wurden auf 50 μl mit Optimem aufgefüllt und 5 min lang bei Raumtemperatur inkubiert. Anschließend wurde die mRNA-Lösung zu der Lipofectamine-2000-Lösung pipettiert und weitere 20 min bei Raumtemperatur inkubiert. Die resultierende Lösung wurde in die Näpfe mit den Zellen pipettiert und nach 4 h wurde Penicillin-Streptomycin (5 μl) zugesetzt und die Inkubation im Brutschrank über Nacht fortgesetzt. Danach wurden die HEK293-Zellen mit PBS gewaschen und durch Zugabe von Trypsin vom Boden der Näpfe abgelöst, bevor sie 5 min lang bei 300 G zentrifugiert wurden. Die U937-Zellen wurden ebenfalls 5 min lang bei 300 G zentrifugiert. Der Überstand wurde entnommen und die Zellen anschließend jeweils zweimal mit PBS gewaschen. Im Anschluss wurden die Zellen in 500 μl PBS für die FACS-Analyse resuspendiert. In den beiden Diagrammen von Fig. 17 ist das Verhältnis der Expression von EGFP zur Expression von RFP dargestellt als Anzahl positiver Zellen (Figur 17a) und als mittlere RFP-Fluoreszenzintensität (Figur 17b).

Die Ergebnisse zeigen, dass durch die Inkorporation einer Mikro-RNA-Bindungsstelle in in vitro transkribierte mRNA die Expression zellspezifisch reguliert werden kann. In der RFPmiRNA-BSmRNA befindet sich 3' von der RFP-Sequenz und 5' von dem PolyA-Schwanz die untranslatierte Sequenz einer vierfachen Wiederholung einer Mikro-RNA-Bindungsstelle, die durch kurze Abstandssequenzen voneinander getrennt sind (SEQ ID Nr. 1). Es wurde eine Mikro-RNA-Bindungsstelle verwendet, die an die Mikro-RNA 142-3p bindet. Diese Mikro-RNA wird in hämatopoetischen Zellen, wie U937-Zellen, exprimiert, nicht aber in Zellen anderen Ursprungs, wie HEK-293-Zellen. Wenn Mikro-RNA 142-3p an die RFP-mi-RNA-BSmRNA bindet, z.B. in den U937-Zellen, wird der Abbau der mRNA über RNA-Interferenz eingeleitet. Dadurch wird die RFP-Bildung vermindert, d.h. weniger Zellen exprimieren RFP mit geringerer Intensität als in solchen Zellen, in denen Mikro-RNA 142-3p nicht vorhanden ist. Um zu zeigen, dass dieses Prinzip auch mit der erfindungsgemäß modifizierten mRNA gut funktioniert, wurden U937- und HEK-293-Zellen jeweils mit EGFP-mRNA (ohne Mikro-RNA-Bindungsstelle) und RFPmiRNA-BSmRNA (mit vierfach Tandem wiederholung der Mikro-RNA-Bindungsstelle für die Mikro-RNA 142-3p) cotransfiziert und anschließend die Expression von EGFP und RFP mittels FACS gemessen. Weil die RFPmiRNA-BSmRNA in U937-Zellen aufgrund der RNA-Interferenz schneller als in HEK-293-Zellen abgebaut wird, während die EGFP-mRNA in beiden Zellen gleich stabil ist, wird erwartet, dass das Verhältnis von EGFP zu RFP in HEK-293-Zellen höher ist als in U937-Zellen. Dies konnte in den durchgeführten Versuchen bestätigt werden. Die Abbildung zeigt deutlich, dass die Anzahl von RFPpositiven U937-Zellen nach Normalisierung auf die Anzahl EGFP-positiver Zellen deutlich niedriger ist als bei HEK-293-Zellen. Das gleiche trifft für die pro Zelle gebildete Menge an RFP zu. Die Ergebnisse zeigen damit auch deutlich, dass das Ausmaß der biologischen Aktivität von in vitro transkribierter mRNA durch den Einbau von Mikro-RNA-Bindungsstellen nach Transfektion in Zellen gesteuert werden kann. Hierdurch kann die mRNA-Translation in solchen Zellen, in denen die mRNA-Translation unerwünscht ist, unterdrückt werden. Dadurch können auch Nebenwirkungen reduziert werden.

Die für die Versuche in diesem Beispiel verwendete mRNA hat die folgende Sequenz (SEQ ID Nr. 1). Grau hinterlegt dargestellt ist die RFP-Sequenz. Die unterstrichene Sequenz zeigt die vierfache Tandemwiederholung der Mikro-RNA-Bindungsstelle für die Mikro-RNA 142-3p mit Abstandssequenzen. Die Sequenz wurde nach Synthese über BamHI-EcoRv in den Vektor pVAX1 kloniert.

GGATCCATGGCCTCCTCCGAGGACGTCATCAAGGAGTTCATGCGCTTCAAGGTGCGCAT
GGAGGGCTCCGTGAACGGCCACGAGTTCGAGATCGAGGGCGAGGGCGAGGGCCGCCCCTAC
GAGGGCACCCAGACCGCCAAGCTGAAGGTGACCAAGGGCGGCCCCCTGCCCTTCGCCTGG
GACATCCTGTCCCCTCAGTTCCAGTACGGCTCCAAGGTGTACGTGAAGCACCCCGCCGAC
ATCCCCGACTACAAGAACCTGTCCTTCGCCGAGGCCTTCAAGTGGGAGCGCGTGATGAAC
TTCGAGGACGGCGGCGTGGTGACCGTGACCCAGGACTCCTCCCTGCAGGACGGCGAGTTC
ATCTACAAGGTGAAGCTGCGCGGCACCAACTTCCCCTCCGACGGCCCCGTAATGCAGAAG

AAGACTATGGGCTGGGAGGCCCTCCACCGAGCGGCTGTACCCCGCCGACGGCCCTGGTCAAG
GGCGAGATCCACAAGGCCCTGAAGCTGAAGGACGGCGGCCACTACCTGGTGGAGTTCAAG
TCCATCTACATGGCCAAGAAGCCCGTGCAGCTGCCCGGCTACTACTACGTGGACTCCAAG
CTGGACATCACCTCCCACAACGAGGACTACACCATCGTGGAGCAGTACGAGCGCGCCGAG
GGCCGCCACCACCTGTTCCTGTAGCTAGAGTCGACTCCATAAAGTAGGAAACACTACACG
ATTCCATAAAGTAGGAAACACTACAACCGGTTCCATAAAGTAGGAAACACTACATCACTC
CATAAAGTAGGAAACACTACACAAAAAAAAAAAAAAAAAAAAAAAAAAAAAAAAAAAAAA
AAAAAAAAAAAAAAAAAAAAAGATATC

**Literaturstellen**

1. K. Kariko et al., Mol Ther (Sep 16, 2008)
2. L. Alexopoulou, A.C. Holt, R. Medzhitov, R.A. Flavell, Nature 413, 732 (Okt 18, 2001)
3. S.S. Diebold, T. Kaisho, H. Hemmi, S. Akira, C. Reis e Sousa, Science 303, 1529 (Mar 5, 2004)
4. F. Heil et al., Science 303, 1526 (Mar 5, 2004)
5. M. Yoneyama et al., Nat Immunol 5, 730 (Jul, 2004)

6. M. Bivas-Benita, R. Zwier, H.E. Junginger, G. Borchard, Eur J Pharm Biopharm 61, 214 (Okt, 2005)

7. D.J. Weiss et al., Mol Ther 8, 927 (Dez, 2003)

8. T.E. Weaver, J.A. Whitsett, Am J Physiol 257, L100 (Au, 1989)

9. S.W. Glasser et al., Proc Natl Acad Sci USA 84, 4007 (Jun 1987)

10. J.A. Whitsett, T.E. Weaver, N Engl J Med 347, 2141 (Dez 26, 2002)

11. L.M. Nogee, D.E. de Mello, L.P. Dehner, H.R. Colten, N Engl J Med 328, 406 (Feb 11, 1993)

12. A. Hamvas et al., J Pediatr 130, 231 (Feb, 1997)

13. J.C. Clark et al., Proc Natl Acad Sci USA 92, 7794 (Aug 15, 1995)

14. K.R. Melton et al., Am J Physiol Lung Cell Mol Physiol 285, L543 (Sept. 2003)

15. M. Ikegami, J.A. Whitsett, P.C. Martis, T.E. Weaver, Am J Physiol Lung Cell Mol Physiol 289, L962 (Dez, 2005)

16. B.D. Brown, M.A. Venneri, A. Zingale, L. Sergi Sergi, L. Naldini, Nat Med 12, 585 (Mai, 2006)

17. B.D. Brown et al., Nat Biotechnol 25, 1457 (Dez, 2007)

18. S.A. McKenna et al., Nat Protoc 2, 3270 (2007)

19. S. Holtkamp et al., Blood 108, 4009 (Dez 15, 2006)

20. M.L.Read et al., Nucleic Acids Res 33, e86 (2005)

21. M.K. Aneja, R. Imker, C. Rudolph, J Gene Med 9, 967 (Nov 2007)

22. P. Dames et al., Nat Nanotechnol 2, 495 (Aug 2007)

23. J.J. Pillow, T.R. Korfhagen, M. Ikegami, P.D. Sly, J Appl Physiol 91, 2730 (Dez 2001)

24. T.F. Schuessler, J.H. Bates, IEEE Trans Biomed Eng 42, 860 (Sept 1995)

25. Z. Hantos, A. Adamicza, E. Govaerts, B. Daroczy, J Appl Physiol 73, 427 (Aug 1992)

26. C.M. Alleyne, I.D. Frantz, 3rd, J.J. Fredberg, J Appl Physiol 66, 542 (Feb 1989)

27. P.D. Sly, R.A. Collins, C. Thamrin, D.J. Turner, Z. Hantos, J Appl Physiol 94, 1460 (Apr 2003)

28. M. Griese et al., Respir Res 6, 80 (2005)

29. M.W. Pfaffl, G.W. Horgan, L. Dempfle, Nucleic Acids Res 30, e36 (Mai 1, 2002)

**Patentansprüche**

1. Polyribonucleotid mit einer Sequenz, die ein Protein oder Proteinfragment codiert, wobei das Polyribonucleotid eine Kombination aus unmodifizierten und modifizierten Nucleotiden enthält, wobei 5 bis 50% der Uridinnucleotide und 5 bis 50% der Cytidinnucleotide modifizierte Uridinnucleotide bzw. modifizierte Cytidinnucleotide sind und wobei die modifizierten Uridinnucleotide 2-Thiouridin sind und die modifizierten Cytidinnucleotide 5-Methylcytidin sind.

2. Polyribonucleotid nach Anspruch 1, **dadurch gekennzeichnet, dass** das Polyribonucleotid mRNA ist.

3. Polyribonucleotid nach Anspruch 1 oder 2, **dadurch gekennzeichnet, dass** 5 bis 30% der Uridinnucleotide und 5 bis 30% der Cytidinnucleotide modifiziert sind.

4. Polyribonucleotid nach einem der Ansprüche 1 bis 3, **dadurch gekennzeichnet, dass** 7,5 bis 25% der Uridinnucleotide und 7,5 bis 25% der Cytidinnucleotide modifiziert sind.

5. Polyribonucleotid nach einem der Ansprüche 1 bis 4, **dadurch gekennzeichnet, dass** es eine RNA-Sequenz enthält, die ein Wachstumshormon wie Somatotropin, Cystic Fibrosis Transmembrane Conductance Regulator (CFTR), Wachstumsfaktoren wie GM-SCF, G-CSF, Protein C, Hepcidin, ABCA3 und Surfactant Protein B, einen Angiogenesefaktor, Stimulator, Induktor, ein Enzym, einen T-Zellrezeptor, Erythropoietin (EPO), BMP-2 oder ein Fragment davon codiert.

6. Polyribonucleotid nach einem der Ansprüche 1 bis 4, wobei das Polyribonucleotid eine SP-B oder ABCA3 codierende RNA enthält, zur Verwendung zur Behandlung von Lungenkrankheiten.

7. Polyribonucleotid nach einem der Ansprüche 1 bis 4, wobei das Polyribonucleotid eine CFTR codierende RNA enthält zur Verwendung zur Behandlung von zystischer Fibrose.

8. Polyribonucleotid nach einem der Ansprüche 1 bis 4, wobei das Polyribonucleotid maßgeschneiderte T-Zellrezeptoren exprimieren kann, zur Verwendung zur Behandlung von Krebserkrankungen.

9. Polyribonucleotid nach einem der Ansprüche 1 bis 4, wobei das Polyribonucleotid eine Sequenz enthält, die einen antigenen Teil eines Krankheitserregers codiert, zur Verwendung als Impfstoff.

**10.** Polyribonucleotid nach Anspruch 6, das eine SP-B codierende Sequenz enthält, zur Verwendung zur Behandlung von Respiratory Distress Syndrom bei Neugeborenen.

**11.** Polyribonucleotid nach einem der Ansprüche 1 bis 4, das eine EPO codierende Sequenz enthält, zur Verwendung zur Behandlung von EPO-Mangel.

**12.** Polyribonucleotid nach einem der Ansprüche 1 bis 4, das mindestens eine einen Wachstumsfaktor, Angiogenesefaktor, Stimulator, Induktor, oder ein Enzym codierende Sequenz enthält, zur Verwendung zur Förderung des Heilungsprozesses und des Einwachsens eines Implantats.

**13.** Pharmazeutische Zusammensetzung enthaltend mindestens eine RNA nach einem der Ansprüche 1 bis 5 zusammen mit pharmazeutisch annehmbaren Hilfsstoffen.

**14.** Pharmazeutische Zusammensetzung nach Anspruch 13, wobei als Hilfsstoff mindestens ein Stabilisierungsmittel ausgewählt aus Lipiden, Polymeren, Nanosystemen und Liposomen enthalten ist.

**15.** Pharmazeutische Zusammensetzung nach Anspruch 13 oder 14 in einer Form zur intratrachealen und/oder pulmonalen Verabreichung.

**16.** Vehikel bestehend aus einem Polyribonucleotid nach einem der Ansprüche 1 bis 5 und einem kationischen Agens.

**17.** Implantat mit einer Beschichtung aus modifizierter RNA gemäß einem der Ansprüche 1 bis 5 in einem verzögert freisetzenden Polymer als Träger.

**18.** Implantat nach Anspruch 17, das ein Zahnimplantat, eine Hüftendoprothese, Knieendoprothese oder ein Wirbelfusionskörper ist.

**19.** Implantat nach Anspruch 17 oder 18, wobei das Trägerpolymer mindestens eine Art von modifizierter RNA enthält, und/oder wobei das Trägerpolymer RNA enthält, die mindestens ein im Zusammenhang mit einer Implantation nützliches Protein codiert und/oder wobei das Trägerpolymer RNA enthält, die einen oder mehrere Wachstumsfaktoren und einen oder mehrere Angiogenesefaktoren codiert.

**20.** Verwendung eines Polyribonucleotids nach einem der Ansprüche 1 bis 5 zur Beschichtung eines Implantats.

**Claims**

**1.** A polyribonucleotide with a sequence which encodes a protein or protein fragment, wherein the polyribonucleotide contains a combination of unmodified and modified nucleotides, wherein 5 to 50% of the uridine nucleotides and 5 to 50% of the cytidine nucleotides are modified uridine nucleotides and modified cytidine nucleotides, respectively, and wherein the modified uridine nucleotides are 2-thiouridine and the modified cytidine nucleotides are 5-methylcytidine.

**2.** The polyribonucleotide according to claim 1, **characterized in that** the polyribonucleotide is mRNA.

**3.** The polyribonucleotide according to claim 1 or 2, **characterized in that** 5 to 30% of the uridine nucleotides and 5 to 30% of the cytidine nucleotides are modified.

**4.** The polyribonucleotide according to any one of claims 1 to 3, **characterized in that** 7.5 to 25% of the uridine nucleotides and 7.5 to 25% of the cytidine nucleotides are modified.

**5.** The polyribonucleotide according to any one of claims 1 to 4, **characterized in that** it contains an RNA sequence which encodes a growth hormone such as somatotropine, cystic fibrosis transmembrane conductance regulator (CFTR), growth factors such as GM-SCF, G-CSF, protein C, hepcidine, ABCA3 and surfactant protein B, an angiogenesis factor, a stimulator, an inducer, an enzyme, a T cell receptor, erythropoietine (EPO), BMP-2 or a fragment thereof.

**6.** The polyribonucleotide according to any one of claims 1 to 4, wherein the polyribonucleotide contains an RNA encoding SP-B or ABCA3, for use in the treatment of lung diseases.

7. The polyribonucleotide according to any one of claims 1 to 4, wherein the polyribonucleotide contains an RNA encoding CFTR, for use in the treatment of cystic fibrosis.

8. The polyribonucleotide according to any one of claims 1 to 4, wherein the polyribonucleotide can express tailored T cell receptors, for use in the treatment of cancer.

9. The polyribonucleotide according to any one of claims 1 to 4, wherein the polyribonucleotide contains a sequence which encodes an antigenic part of a pathogen for use as a vaccine.

10. The polyribonucleotide according to claim 6, which contains a sequence encoding SP-B, for use in the treatment of respiratory distress syndrome in the newborn.

11. The polyribonucleotide according to any one of claims 1 to 4, which contains a sequence encoding EPO, for use in the treatment of EPO deficiency.

12. The polyribonucleotide according to any one of claims 1 to 4, which contains at least one sequence encoding a growth factor, an angiogenesis factor, a stimulator, an inducer or an enzyme, for use in the enhancement of the healing process and integration of an implant.

13. A pharmaceutical composition containing at least one RNA according to any one of claims 1 to 5 together with pharmaceutically acceptable additives.

14. The pharmaceutical composition according to claim 13, wherein at least one stabilizing agent selected from lipids, polymers, nanosystems and liposomes is contained as an additive.

15. The pharmaceutical composition according to claim 13 or 14 in a form for intratracheal and/or pulmonary administration.

16. A vehicle consisting of a polyribonucleotide according to any one of claims 1 to 5 and a cationic agent.

17. An implant having a coating of a modified RNA according to any one of claims 1 to 5 in a delayed release polymer as carrier.

18. The implant according to claim 17, which is a dental implant, a hip endoprosthesis, knee endoprosthesis or a vertebral fusion body.

19. The implant according to claim 17 or 18, wherein the carrier polymer contains at least one type of modified RNA and/or wherein the carrier polymer contains RNA which encodes at least one protein beneficial in connection with an implantation and/or wherein the carrier polymer contains RNA which encodes one or more growth factors and one or more angiogenesis factors.

20. The use of a polyribonucleotide according to any one of claims 1 to 5 for the coating of an implant.

**Revendications**

1. Polyribonucléotide ayant une séquence codant une protéine ou un fragment de protéine, le polyribonucléotide contenant une combinaison de nucléotides modifiés et non modifiés, 5 à 50 % des nucléotides uridine et 5 à 50 % des nucléotides cytidine étant respectivement des nucléotides uridine modifiés et des nucléotides cytidine modifiés, et les nucléotides uridine modifiés étant la 2-thiouridine et les nucléotides cytidine modifiés étant la 5-méthylcytidine.

2. Polyribonucléotide selon la revendication 1, **caractérisé en ce que** le polyribonucléotide est un ARNm.

3. Polyribonucléotide selon la revendication 1 ou 2, **caractérisé en ce que** 5 à 30 % des nucléotides uridine et 5 à 30 % des nucléotides cytidine sont modifiés.

**4.** Polyribonucléotide selon l'une des revendications 1 à 3, **caractérisé en ce que** 7,5 à 25 % des nucléotides uridine et 7,5 à 25 % des nucléotides cytidine sont modifiés.

**5.** Polyribonucléotide selon l'une des revendications 1 à 4, **caractérisé en ce qu'**il contient une séquence d'ARN codant une hormone de croissance telle que la somatotropine, le régulateur de la conduction transmembranaire de la fibrose kystique (CFTR), les facteurs de croissance tels que GM-SCF, G-CSF, la protéine C, l'hepcidine, ABCA3 et la protéine B tensioactive, un facteur de l'angiogenèse, un stimulateur, un inducteur, une enzyme, un récepteur des lymphocytes T, l'érythropoïétine (EPO), BMP-2 ou un fragment de celle-ci.

**6.** Polyribonucléotide selon l'une des revendications 1 à 4, où le polyribonucléotide contient un ARN codant SP-B ou ABCA3, pour une utilisation dans le traitement des maladies du poumon.

**7.** Polyribonucléotide selon l'une des revendications 1 à 4, où le polyribonucléotide contient un ARN codant CFTR, pour une utilisation dans le traitement de la fibrose kystique.

**8.** Polyribonucléotide selon l'une des revendications 1 à 4, où le polyribonucléotide peut exprimer des récepteurs des lymphocytes T spécialement conçus, pour une utilisation dans le traitement des cancers.

**9.** Polyribonucléotide selon l'une des revendications 1 à 4, où le polyribonucléotide contient une séquence codant une partie antigénique d'un pathogène, pour une utilisation en tant que substance vaccinale.

**10.** Polyribonucléotide selon la revendication 6, qui contient une séquence codant SP-B, pour une utilisation dans le traitement du syndrome de détresse respiratoire chez les nouveaux-nés.

**11.** Polyribonucléotide selon l'une des revendications 1 à 4, qui contient une séquence codant l'EPO, pour une utilisation dans le traitement de la carence en EPO.

**12.** Polyribonucléotide selon l'une des revendications 1 à 4, qui contient au moins une séquence codant un facteur de croissance, un facteur de l'angiogenèse, un stimulateur, un inducteur, ou une enzyme, pour une utilisation dans le but de favoriser le processus de guérison et l'ancrage d'un implant.

**13.** Composition pharmaceutique, contenant au moins un ARN selon l'une des revendications 1 à 5 conjointement avec des excipients pharmaceutiquement acceptables.

**14.** Composition pharmaceutique selon la revendication 13, dans laquelle au moins un stabilisateur choisi parmi les lipides, les polymères, les nanosystèmes et les liposomes est présent en tant qu'excipient.

**15.** Composition pharmaceutique selon la revendication 13 ou 14 sous une forme destinée à l'administration intratrachéale et/ou pulmonaire.

**16.** Véhicule constitué d'un polyribonucléotide selon l'une des revendications 1 à 5 et d'un agent cationique.

**17.** Implant comprenant un revêtement constitué d'ARN modifié selon l'une des revendications 1 à 5 dans un polymère à libération retardée comme support.

**18.** Implant selon la revendication 17, qui est un implant dentaire, une endoprothèse de la hanche, une endoprothèse du genou ou un corps de fusion vertébrale.

**19.** Implant selon la revendication 17 ou 18, dans lequel le polymère support contient au moins un type d'ARN modifié, et/ou le polymère support contient un ARN codant au moins une protéine utile dans le contexte d'une implantation et/ou le polymère support contient un ARN codant un ou plusieurs facteurs de croissance et un ou plusieurs facteurs d'angiogenèse.

**20.** Utilisation d'un polyribonucléotide selon l'une des revendications 1 à 5 pour le revêtement d'un implant.

a

Figur 1a

Figuren 1b und 1c

EP 2 459 231 B1

a

b

Figuren 2a und 2b

35

c

Figur 2c

**d**

Figur 2d

**a**

**b**

Figuren 3a und 3b

c

SP-B⁻/⁻ (n = 5)

Histology, BAL

day 0 1           29

SP-B⁻/⁻ (n = 4)

Histology, BAL

day 0 1 4          29

SP-B⁻/⁻ (n = 4)

Histology, BAL

day 0 1           29

■ doxycycline

↓ 25 µl Perfluorocarbon
50 µl EGFPLuc mRNA $s2U_{(0.25)}m5C_{(0.25)}$

↓ 25 µl Perfluorocarbon
50 µl SP-B mRNA $s2U_{(0.25)}m5C_{(0.25)}$

d

$P = 0.009$

— + $s2U_{(0.25)}m5C_{(0.25)}$ control mRNA (n = 5)

— + $s2U_{(0.25)}m5C_{(0.25)}$ SP-B mRNA (n = 4)

Überlebensrate (%)

Tage nach Doxycyclin-Entzug

Figuren 3c und 3d

Figuren 3e, 3f, 3g, 3h und 3i

Figur 4

Figur 5

Figur 6

A549 - 200 ng RFP mRNA mod/mod (10%)

Figur 7

EP 2 459 231 B1

A549 - 200ng RFP mRNA s2U/m5C [x%]

MLE12 - 200ng RFP mRNA s2U/m5C [x%]

Figur 8

Figur 9

Figur 10a

Figur 10b

a

b

Figuren 11a und 11b

Figur 11c

**d**

Figur 11d

Figur 12

Figur 13

Figur 14

Figur 15

Figur 16

Figur 17a

Figur 17 b

GGATCCATGGCCTCCTCCGAGGACGTCATCAAGGAGTTCATGCGCTTCAAGGTG
CGCATGGAGGGCTCCGTGAACGGCCACGAGTTCGAGATCGAGGGCGAGGGCGA
GGGCCGCCCCTACGAGGGCACCCAGACCGCCAAGCTGAAGGTGACCAAGGGCG
GCCCCCTGCCCTTCGCCTGGGACATCCTGTCCCCCCAGTTCCAGTACGGCTCCAA
GGTGTACGTGAAGCACCCCGCCGACATCCCCGACTACAAGAAGCTGTCCTTCCC
CGAGGGCTTCAAGTGGGAGCGCGTGATGAACTTCGAGGACGGCGGCGTGGTGAC
CGTGACCCAGGACTCCTCCCTGCAGGACGGCTGCTTCATCTACAAGGTGAAGTTC
ATCGGCGTGAACTTCCCCTCCGACGGCCCCGTAATGCAGAAGAAGACTATGGGC
TGGGAGCCCTCCACCGAGCGCCTGTACCCCCGCGACGGCGTGCTGAAGGGCGAG
ATCCACAAGGCCCTGAAGCTGAAGGACGGCGGCCACTACCTGGTGGAGTTCAAG
TCCATCTACATGGCCAAGAAGCCCGTGCAGCTGCCCGGCTACTACTACGTGGACT
CCAAGCTGGACATCACCTCCCACAACGAGGACTACACCATCGTGGAGCAGTACG
AGCGCGCCGAGGGCCGCCACCACCTGTTCCTGTAGCTAGAGTCGACTCCATAAA
GTAGGAAACACTACACGATTCCATAAAGTAGGAAACACTACAACCGGTTCCATA
AAGTAGGAAACACTACATCACTCCATAAAGTAGGAAACACTACACAAAAAAAA
AAAAAAAAAAAAAAAAAAAAAAAAAAAAAAAAAAAAAAAAAAAAAAAAAAAAA
AGATATC

Figur 18

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007024708 A
- EP 1198489 A
- WO 2007000668 A

**In der Beschreibung aufgeführte Nicht-Patentliteratur**

- **GU et al.** *Nat Struct Mol Biol.,* Februar 2009, vol. 16 (2), 144-50
- **BROWN et al.** *Nat Biotechnol.,* Dezember 2007, vol. 25 (12), 1457-67
- **BROWN et al.** *Nat Med.,* Mai 2006, vol. 12 (5), 585-91
- **K. KARIKO et al.** *Mol Ther,* 16. September 2008
- **L. ALEXOPOULOU ; A.C. HOLT ; R. MEDZHITOV ; R.A. FLAVELL.** *Nature,* 18. Oktober 2001, vol. 413, 732
- **S.S. DIEBOLD ; T. KAISHO ; H. HEMMI ; S. AKIRA ; C. REIS ; E SOUSA.** *Science,* 05. Marz 2004, vol. 303, 1529
- **F. HEIL et al.** *Science,* 05. Marz 2004, vol. 303, 1526
- **M. YONEYAMA et al.** *Nat Immunol,* Juli 2004, vol. 5, 730
- **M. BIVAS-BENITA ; R. ZWIER ; H.E. JUNGINGER ; G. BORCHARD.** *Eur J Pharm Biopharm,* Oktober 2005, vol. 61, 214
- **D.J. WEISS et al.** *Mol Ther,* Dezember 2003, vol. 8, 927
- **T.E. WEAVER ; J.A. WHITSETT.** *Am J Physiol,* August 1989, vol. 257, L100
- **S.W. GLASSER et al.** *Proc Natl Acad Sci USA,* Juni 1987, vol. 84, 4007
- **J.A. WHITSETT ; T.E. WEAVER.** *N Engl J Med,* 26. Dezember 2002, vol. 347, 2141
- **L.M. NOGEE ; D.E. DE MELLO ; L.P. DEHNER ; H.R. COLTEN.** *N Engl J Med,* 11. Februar 1993, vol. 328, 406
- **A. HAMVAS et al.** *J Pediatr,* Februar 1997, vol. 130, 231
- **J.C. CLARK et al.** *Proc Natl Acad Sci USA,* 15. August 1995, vol. 92, 7794
- **K.R. MELTON et al.** *Am J Physiol Lung Cell Mol Physiol,* September 2003, vol. 285, L543
- **M. IKEGAMI ; J.A. WHITSETT ; P.C. MARTIS ; T.E. WEAVER.** *Am J Physiol Lung Cell Mol Physiol,* Dezember 2005, vol. 289, L962
- **B.D. BROWN ; M.A. VENNERI ; A. ZINGALE ; L. SERGI SERGI ; L. NALDINI.** *Nat Med,* Mai 2006, vol. 12, 585
- **B.D. BROWN et al.** *Nat Biotechnol,* Dezember 2007, vol. 25, 1457
- **S.A. MCKENNA et al.** *Nat Protoc,* 2007, vol. 2, 3270
- **S. HOLTKAMP et al.** *Blood,* 15. Dezember 2006, vol. 108, 4009
- **M.L.READ et al.** *Nucleic Acids Res,* 2005, vol. 33, e86
- **M.K. ANEJA ; R. IMKER ; C. RUDOLPH.** *J Gene Med,* November 2007, vol. 9, 967
- **P. DAMES et al.** *Nat Nanotechnol,* August 2007, vol. 2, 495
- **J.J. PILLOW ; T.R. KORFHAGEN ; M. IKEGAMI ; P.D. SLY.** *J Appl Physiol,* Dezember 2001, vol. 91, 2730
- **T.F. SCHUESSLER ; J.H. BATES.** *IEEE Trans Biomed Eng,* September 1995, vol. 42, 860
- **Z. HANTOS ; A. ADAMICZA ; E. GOVAERTS ; B. DAROCZY.** *J Appl Physiol,* August 1992, vol. 73, 427
- **C.M. ALLEYNE ; I.D. FRANTZ, 3RD ; J.J. FREDBERG.** *J Appl Physiol,* Februar 1989, vol. 66, 542
- **P.D. SLY ; R.A. COLLINS ; C. THAMRIN ; D.J. TURNER ; Z. HANTOS.** *J Appl Physiol,* April 2003, vol. 94, 1460
- **M. GRIESE et al.** *Respir Res,* 2005, vol. 6, 80
- **M.W. PFAFFL ; G.W. HORGAN ; L. DEMPFLE.** *Nucleic Acids Res,* 01. Mai 2002, vol. 30, e36